# FASCICULE DE BREVET EUROPEEN

(11) **EP 0 584 191 B1**
(45) Date de publication et mention de la délivrance du brevet: **13.08.1997**
(21) Numéro de dépôt: 92911015.3
(22) Date de dépôt: 04.05.1992
(51) Int. Cl.: C07C 65/26, C07C 65/17, C07C 63/49

(54) **NOUVEAUX COMPOSES POLYCYCLIQUES AROMATIQUES ET LEUR UTILISATION EN MEDECINE HUMAINE OU VETERINAIRE ET EN COSMETIQUE**
NEUE AROMATISCHE UND POLYCYCLISCHE VERBINDUNGEN UND IHRE VERWENDUNG IN DER HUMAN- ODER TIERMEDIZIN UND IN KOSMETIKA
NOVEL AROMATIC AND POLYCYCLIC COMPOUNDS AND THEIR USE IN HUMAN OR VETERINARY MEDICINE AND IN COSMETICS

(30) Priorité: 02.05.1991 FR 9105394
(43) Date de publication de la demande: 02.03.1994
(73) Titulaire: CENTRE INTERNATIONAL DE RECHERCHES DERMATOLOGIQUES GALDERMA, ( CIRD GALDERMA), 06560 Valbonne (FR)
(72) Inventeur: CHARPENTIER, Bruno, F-06410 Biot (FR)
(74) Mandataire: Stalla-Bourdillon, Bernard
(86) Numéro de dépôt international: FR9200404
(87) Numéro de publication internationale: WO9219583

(56) Documents cités:
- EP-A- 0 199 636
- EP-A- 0 315 537

## Description

La présente invention a pour objet de nouveaux dérivés polycycliques aromatiques, leur procédé de préparation et leur utilisation en médecine humaine et vétérinaire et en cosmétique.

Ces nouveaux composés trouvent une application dans le traitement topique et systémique des affections dermatologiques liées à un désordre de la kératinisation (différenciation-prolifération) et d'affections dermatologiques, ou autres, à composantes inflammatoires et/ou immunoallergiques et dans les maladies de dégénérescence du tissu conjonctif, et présentent une activité anti-tumorale. En outre, ces composés peuvent être utilisés dans le traitement de l'atopie, qu'elle soit cutanée ou respiratoire et du psoriasis rhumatoïde.

Ils trouvent également une application dans le domaine ophtalmologique, notamment dans le traitement des cornéopathies.

Divers composés polycycliques aromatiques, leur procédé de préparation ainsi que leur application dans les domaines pharmaceutique et cosmétique ont déjà été décrits dans EP-A-199636.

Les composés selon l'invention peuvent être représentés par la formule générale suivante : 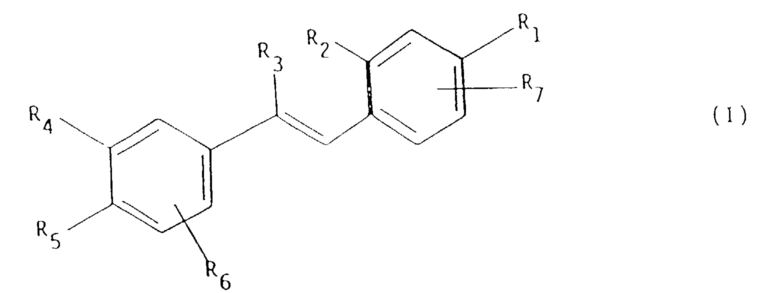 dans laquelle :
R₁ représente
   (i) un atome d'hydrogène,
   (ii) le radical -CH₃,
   (iii) le radical -CH₂-O-R₈,
      R₈ représentant un atome d'hydrogène ou un radical alkyle inférieur,
   (iv) un radical -OR₈,
   (v) un radical 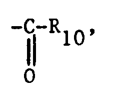 R₁₀ représentant :
      (a) un atome d'hydrogène,
      (b) un radical 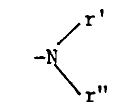 r' et r" représentant un atome d'hydrogène, un radical alkyle inférieur, un radical mono ou polyhydroxyalkyle, un radical aryle éventuellement substitué ou un reste d'amino acide ou de sucre ou encore pris ensemble forment un hétérocycle,
      (c) un radical -OR₁₁
         R₁₁ représentant un atome d'hydrogène, un radical alkyle, linéaire ou ramifié, ayant de 1 à 20 atomes de carbone, un radical mono ou polyhydroxyalkyle, un radical aryle ou aralkyle éventuellement substitué(s) ou un reste de sucre ou un reste d'amino acide, et
   (vi) un radical S(O)ₜ R₈,
      t étant 0, 1 ou 2 et R₈ étant tel que défini ci-dessus,
R₂ représente un atome d'hydrogène,
R₃ représente un atome d'hydrogène, un radical aryle, un radical aralkyle ou un radical alkyle inférieur éventuellement substitué par un hydroxyle, par un alcoxy inférieur ou par un radical 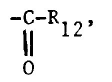
R₁₂ représentant un atome d'hydrogène, un radical alkyle inférieur, un radical hydroxyle, un radical alcoxy inférieur ou un radical 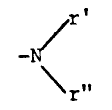
r' et r" ayant les mêmes significations que ci-dessus,
ou R₂ et R₃ pris ensemble forment, avec le noyau benzénique, un cycle naphtalénique,
R₄ représente un radical alkyle linéaire ou ramifié ayant de 1 à 15 atomes de carbone ou un radical cycloaliphatique,
R₅ représente le radical -(CH₂)ₙ-R₁₃, le radical 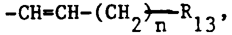 ou le radical 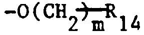
   n étant 0 ou 1 à 6,
   m étant 1 à 6
   R₁₃ représentant le radical 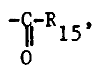
   un radical monohydroxyalkyle ou un radical polyhydroxyalkyle dont les hydroxyles sont éventuellement protégés sous forme de méthoxy ou d'acétoxy, un radical alkyle inférieur epoxydé ou le radical 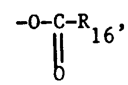
R₁₅ représentant le radical OR₁₆ ou le radical 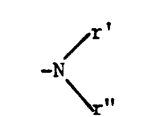
R₁₆ représentant un atome d'hydrogène, un radical alkyle inférieur, un radical aryle ou un radical aralkyle,
R₁₄ représentant un radical hydroxyle lorsque m > 2, un radical monohydroxyalkyle, un radical polyhydroxyalkyle, le radical 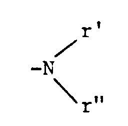
le radical 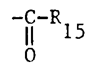 ou un radical alkényle mono ou polyhydroxylé ayant de 2 à 10 atomes de carbone, ou lorsque R₂ et R₃ ne sont pas pris ensemble, m peut être 0 et/ou R₁₄ peut représenter un atome d'hydrogène ou un radical alkyle inférieur,
R₆ et R₇ représentent un atome d'hydrogène, un atome d'halogène, un radical alkyle inférieur ou le radical -OR₁₆,
R₅ et R₆ peuvent en outre former un cycle méthylène dioxy lorsque R₆ est en position 3 du noyau benzénique, et les sels des composés de formule (I) lorsque R₁ ou R₁₃ représente une fonction acide carboxylique ou lorsque R₁₄ représente une fonction amine et les analogues chiraux desdits composés de formule (I) et les isomères géométriques desdits composés lorsque R₂ et R₃ ne sont pas pris ensemble.

Lorsque les composés selon l'invention se présentent sous forme de sels, lorsque R₁ ou R₁₃ représente une fonction carboxylique, il s'agit alors de sels d'un métal alcalin ou alcalino-terreux ou encore de zinc ou d'une amine organique.

Lorsque R₁₄ représente une fonction amine, il s'agit alors de sels pharmaceutiquement ou cosmétiquement acceptables, formés par addition d'acide organique ou minéral, en particulier l'acide chlorhydrique, sulfurique, acétique, citrique, fumarique, hémisuccinique, maléique et mandélique.

Par radical alkyle inférieur, on entend un radical ayant de 1 à 6 atomes de carbone et de préférence les radicaux méthyle, éthyle, isopropyle, butyle et tertiobutyle.

Par radical alcoxy inférieur, on doit entendre les radicaux ayant de 1 à 4 atomes de carbone notamment les radicaux méthoxy, éthoxy, isopropoxy ou butoxy.

Par radical cycloaliphatique on doit entendre un radical mono ou polycyclique tel que par exemple le radical 1-méthyl cyclohexyle ou 1-adamantyle.

Par radical monohydroxyalkyle, on doit entendre un radical ayant de 1 à 6 atomes de carbone, notamment un radical hydroxyméthyle, 2-hydroxyéthyle, 2-hydroxypropyle, 3-hydroxypropyle, 4-hydroxybutyle, 5-hydroxypentyle ou 6-hydroxyhexyle.

Par radical polyhydroxyalkyle, on doit entendre un radical contenant de 3 à 6 atomes de carbone et de 2 à 5 groupes hydroxyles tels que les radicaux 2,3-dihydroxypropyle, 2,3,4-trihydroxybutyle, 2,3,4,5-tétrahydroxypentyle ou le reste du pentaérythritol.

Par radical aryle, on doit entendre un radical phényle éventuellement substitué par au moins un atome d'halogène, un hydroxyle ou une fonction nitro.

Par radical aralkyle, on doit entendre le radical benzyle ou phénéthyle éventuellement substitué par au moins un atome d'halogène, un hydroxyle ou une fonction nitro.

Lorsque les radicaux R₆ et R₇ représentent un atome d'halogène, celui-ci est de préférence un atome de chlore, de brome ou de fluor.

Par reste d'aminoacide, on doit entendre un reste dérivant de la lysine, de la glycine ou de l'acide aspartique.

Par reste d'un sucre on doit entendre un reste dérivant par exemple de glucose, de galactose ou de mannose.

Par hétérocycle, on entend de préférence un radical pipéridino, morpholino, pyrrolidino ou pipérazino, éventuellement substitué en position 4 par un radical alkyle en C₁-C₆ ou mono ou polyhydroxyalkyle tels que définis ci-dessus.

Parmi les composés de formule (I) ci-dessus, on peut notamment citer les suivants :
1) Chlorhydrate de l'acide 6-[3-(1-adamantyl)-4-(3-aminopropyloxy) phényl]-2-naphtoïque ;
2) 6-[3-(1-adamantyl)-4-(2,3-dihydroxypropyloxy)phényl]-2-naphtoate de méthyle ;
3) Acide 6-[3-(1-adamantyl)-4-(2,3-dihydroxypropyloxy)phényl] -2-naphtoïque ;
4) 6-[3-(1-adamantyl)-6-méthoxycarbonylméthyloxyphényl] -2-naphtoate de benzyle ;
5) Acide 6-[3-(1-adamantyl)-4-méthoxycarbonylméthyloxyphényl] -2-naphtoïque ;
6) Acide 6-[3-(1-adamantyl)-4-carboxyméthyloxyphényl] -2-naphtoïque ;
7) 6- [3-(1-adamantyl)-4-(3-hydroxypropyloxy)phényl]-2-naphtoate de méthyle ;
8) Acide 6-[3-(1-adamantyl)-4-(3-hydroxypropyloxy)phényl] -2-naphtoïque ;
9) 6-[3-(1-adamantyl)-4-benzyloxycarbonylphényl]-2-naphtoate de méthyle ;
10) 6-[3-(1-adamantyl)-4-carboxyphényl]-2-naphtoate de méthyle ;
11) 6-[3-(1-adamantyl)-4-hydroxymethylphényl]-2-naphtoate de méthyle ;
12) Acide 6-[3-(1-adamantyl)-4-hydroxyméthylphényl]-2-naphtoïque;
13) Acide 6-[3-(1-adamantyl)-4-acétoxyméthylphényl]-2-naphtoïque;
14) 6- [3-(1-adamantyl)-4-methoxycarbonylphényl]-2-naphtoate de méthyle ;
15) Acide 6-[3-(1-adamantyl)-4-méthoxycarbonylphényl]-2-naphtoïque;
16) Acide 6-[3-(1-adamantyl)-4-carboxyphényl]-2-naphtoïque ;
17) Acide 6-[3-(1-adamantyl)-4-carboxamidophényl]-2-naphtoïque ;
18) 6-[3-(1-adamantyl)-4-méthoxycarbonyléthénylphényl]-2-naphtoate de benzyle ;
19) Acide 6-[3-(1-adamantyl)-4-(méthoxycarbonyléthyl)phényl] -2-naphtoïque ;
20) 6-[3-(1-adamantyl)-4-(2,3-époxypropyl)phényl]-2-naphtoate de benzyle ;
21) Acide 6-[3-(1-adamantyl)-4-(2-hydroxypropyl)phényl] -2-naphtoïque ;
22) Acide 6-[3-(1-adamantyl)-4-(3-méthoxy-2-hydroxypropyl)phényl] -2-naphtoïque.
23) Acide 4[(E)-2-(3-(1-adamantyl)-4-méthoxy phényl)propenyl] benzoïque ;
24) 4-[(E)-2-(3-(1-adamantyl)-4-méthoxyphényl)propényl]benzoate de benzyle ;
25) Acide 4-[(E)-2-(3-(1-adamantyl)-4-hydroxyphényl)-1-propényl] benzoïque ;
26) 6-[3-(1-adamantyl)-4-méthoxycarbonylphényl]-2- naphtylcarboxamide ;
27) 4-[(E)-2-(3-(1-méthylcyclohexyl)-4-hydroxyphényl)propényl]benzoate d'éthyle ;
28) 4-[(E)-2-(3-(1-méthylcyclohexyl)-4-méthoxyphényl)propényl]benzoate d'éthyle ;
29) Acide 4-[(E)-2-(3-(1-méthylcyclohexyl)-4-méthoxyphényl)propényl]benzoïque ;
30) 4-[(Z)-2-(3-(1-méthylcyclohexyl)-4-hydroxyphényl)propényl]benzoate d'éthyle ;
31) 4-[(Z)-2-(3-(1-méthylcyclohexyl)-4-méthoxyphényl)propényl]benzoate d'éthyle ;
32) Acide 4-[(Z)-2-(3-(1-méthylcyclohexyl)-4-méthoxyphényl)propényl]benzoïque;
33) 4-[(Z)-2-(3-tert-butyl-4-méthoxyphényl)propényl]benzoate d'éthyle ;
34) Acide 4-[(Z)-2-(3-tert-butyl-4-méthoxyphényl)propényl]benzoïque;
35) Acide 4-[(E)-2-(3-tert-butyl-4-hydroxyphényl)propényl]benzoïque;
36) Acide 4-[(E)-2-(3-(1-méthylcyclohexyl)-4- hydroxyphényl)éthényl]benzoïque ;
37) 4-[(E)-2-(3-(1-méthylcyclohexyl)-4-(6-tert- butoxycarbonylpentyloxy)phényl) éthényl]benzoate d'éthyle ;
38) 4-[(E)-2-(3-(1-méthylcyclohexyl)-4-(6-carboxypentyloxy)phényl)éthényl]benzoate d'éthyle ;
39) Acide 4-[(E)-2-(3-(1-adamantyl)-4-hydroxyphényl)éthényl] benzoïque ;
40) 6-[3-(1-adamantyl)4-(1,2-dihydroxyéthyl)phényl]-2-naphtoate de benzyle ;
41) Acide 6-[3-(1-adamantyl)-4-(1,2-dihydroxyéthyl)phényl]-2-naphtoïque ;
42) 6-[3-(1-adamantyl)-4-(3-hydroxypropyl)phényl]-2-naphtoate de benzyl ;
43) Acide 6-[3-(1-admantyl)-4-(3-hydroxypropyl)phényl]-2-naphtoïque ;
44) 6-[3-(1-adamantyl)-4-(3-acétoxypropyl)phényl]-2-naphtoate de benzyle ;
45) Acide 6-[3-(1-adamantyl)-4-(3-acétoxypropyl)phényl]-2-naphtoïque ;
46) 6-[3-(1-adamantyl)-4-(2,3-dihydroxypropyl)phényl]-2-naphtoate de benzyle ;
47) Acide 6-[3-(1-adamantyl)-4-(2,3-dihydroxypropyl)phényl]-2- naphtoïque ;
48) N-méthoxycarbonylméthyl-4-(6-benzyloxycarbonylnaphtyl)-2-(1-adamantyl)phényl carboxamide ;
49) N-méthoxycarbonylméthyl4-(6-Carboxynaphtyl)-2-(1-adamantyl)phénylcarboxamide ;
50) Acide 6-[3-(1-admnantyl)-4-(N,N-diméthylcarbamoyl)phényl]-2-naphtoïque;
51) 6-[3-(1-adamantyl)-4-(2(R),3-dihydroxypropyloxy)phényl]-2-naphtoate de benzyle ;
52) Acide 6-[3-(1-adamantyl)-4-(2(R),3-dihydroxypropyloxy)phényl]-2-naphtoïque;
53) Acide 6-[3-(1-adamantyl)-4,5-méthylène dioxyphényl]-2-naphtoïque;
54) N-éthyl 6-[3-(1-adamantyl)-4-méthoxycarbonylphényl]-2- naphtylcarboxamide ;
55) N,N-morpholyl 6-[3-(1-adamantyl)-4-méthoxycarbonylphényl]-2- naphtylcarboxamide ;
56) 4-[(E)-2-(3-tert-butyl-4-méthoxyphényl)propényl]phénylcarbinol ; et
57) 4-[(E)-2-(3-tert-butyl-4-méthoxyphényl)propényl]benzylacétate.

La présente invention a également pour objet le procédé de préparation des composés de formule (I).

Les composés de formule (I) pour lesquels R₂ et R₃ pris ensemble forment avec le noyau benzénique un cycle naphtalénique sont obtenus par une réaction de couplage entre un dérivé halogéné (1) et un dérivé halogéné de formule (2) :

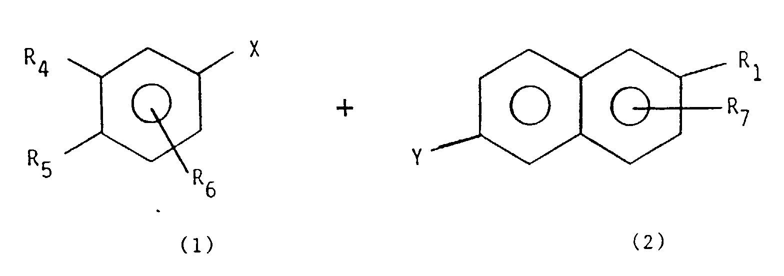

X et Y représentant un atome de chlore, de brome ou d'iode.

Dans un premier temps, l'halogènure (1) est converti en un lithien, magnesien ou zincique puis est couplé au dérivé (2) en présence d'un catalyseur au nickel ou au palladium, selon les conditions de couplage biaryl décrites par E. Negishi et al., J. Org. Chem. (1977) 42, 1821.

Les composés de formule (I) pour lesquels R₂ et R₃ ne sont pas pris ensemble peuvent ête obtenus par le schéma réactionnel suivant en mettant en jeu une réaction de Wittig ou Horner-Emmons (Voir tableau I).

Dans ces réactions d'oléfination, l'isomère géométrique de configuration E peut aussi être obtenu par conversion sous irradiation sous lumière UV de l'isomère de configuration géométrique Z.

Dans ces formules R₁, R₄, R₆ et R₇ ont les mêmes significations que celles données ci-dessus pour la formule générale ou en sont des dérivés convenablement protégés pour être compatibles avec les conditions de couplage. En particulier, le substituant R₅ est un phénol protégé sous forme de tert-butyldiméthylsililoxy ou un radical alcoxy.

Le dérivé obtenu est ensuite converti en phénol par déprotection au niveau du substituant R₅ du groupe TBDMS ou alcoxy puis est traité selon l'une des deux voies mentionnées ci-dessous :
- traitement du phénol ainsi obtenu par un hydrure métallique que l'on fait réagir sur un halogénure,
- conversion du phénol ainsi obtenu en triflate puis substitution nucléophile en présence d'un catalyseur au palladium (voir tableau II) selon les conditions générales décrites par :
   - S. Cacchi et al., Tetrahedron Letter, 1986, 27, 3931-3934
   - W.J Scott et al., J. Org. Chem., 1985, 50, 2302-2308
   Dans le cas où R₂ et R₃ ne sont pas pris ensemble, il est préférable d'effectuer cette réaction de substitution nucléophile avant la réaction d'oléfination (Wittig ou Horner-Emmons) décrite dans le tableau I.

La présente invention a également pour objet, à titre de produit intermédiaire dans la synthèse des composés selon l'invention, les composés suivants :
- l'acide 6-[3-(1-adamantyl)-6-(3-aminopropyloxy)phényl]-2 naphtoïque,
- le 3-(1-adamantyl)-4-(2,2-diméthyl-1,3-dioxolanne-4-méthyloxy)bromobenzène, et
- le 6-[3-(1-adamantyl)-4-(2,2-diméthyl-1,3-dioxolanne-4-méthyloxy)phényl]-2-naphtoate de méthyle.

La présente invention a également pour objet à titre de médicament les composés de formule (I) telle que définie ci-dessus.

Les composés selon l'invention présentent une bonne stabilité à la lumière et à l'oxygène.

Ces composés présentent une activité dans le test de différenciation des cellules (F9) de tératocarcinome embryonnaire de souris (Cancer Research 43, p. 5268, 1983) et/ou dans le test d'inhibition de l'ornithine décarboxylase après induction par le TPA chez la souris (Cancer Research 38, p.793-801, 1978). Ces tests montrent les activités des composés respectivement dans les domaines de la différenciation et de la prolifération cellulaire.

Les composés selon l'invention conviennent particulièrement bien dans les domaines de traitement suivants :
1) Pour traiter les affections dermatologiques liées à un désordre de la kératinisation portant sur la différenciation et sur la prolifération notamment pour traiter les acnés vulgaires, comédoniennes, polymorphes, les acnés nodulokystiques, conglobata, les acnés séniles, les acnés secondaires telles que l'acné solaire, médicamenteuse, professionnelle.
2) Pour traiter d'autres types de trouble de la kératinisation, notamment les ichtyoses, les états ichtyosiformes, la maladie de Darier, les kératodermies palmoplantaires, les leucoplasies et les états leucoplasiformes, le lichen cutané ou muqueux,
3) Pour traiter d'autres affections dermatologiques liées à un trouble de la kératinisation avec une composante inflammatoire et/ou immuno-allergique et, notamment, toutes les formes de psoriasis qu'il soit cutané, muqueux ou unguéal, et même le rhumatisme psoriatique, ou encore l'atopie cutanée, telle que l'eczéma, ou l'atopie respiratoire ou l'hypertrophie gingivale ; les composés peuvent également être utilisés dans certaines affections inflammatoires ne présentant pas de trouble de la kératinisation.
4) Pour traiter toutes les proliférations dermiques ou épidermiques qu'elles soient bénignes ou malignes, qu'elles soient d'origine virale telles que verrues vulgaires, les verrues planes et l'épidermodysplasie verruciforme, les papillomatoses orales florides et les proliférations pouvant également être induites par les ultra-violets notamment dans le cas des épithélioma baso et spino cellulaires.
5) Pour traiter d'autres désordres dermatologiques tels que les dermatoses bulleuses et les maladies du collagène.
6) Pour traiter certains troubles ophtalmologiques, notamment les cornéopathies.
7) Pour réparer et lutter contre le vieillissement de la peau, qu'il soit photoinduit ou chronologique ou à réduire les pigmentations et les kératoses actiniques.
8) Pour prévenir ou guérir les stigmates de l'atrophie épidermique et/ou dermique induite par les corticostéroïdes locaux ou systémiques, ou tout autre forme d'atrophie cutanée.
9) Pour prévenir ou traiter les troubles de la cicatrisation, pour prévenir et réparer les vergetures.
10) Pour lutter contre les troubles de la fonction sébacée tels que l'hyperséborrhée de l'acné ou la séborrhée simple.
11) Dans le traitement de situations cancéreuses ou précancéreuses en particuier au niveau cutané.
12) Dans le traitement d'affections inflammatoires telles de l'arthrite.

La présente invention a également pour objet des compositions médicamenteuses contenant au moins un composé de formule (I) telle que définie ci-dessus, ou un de ses sels.

La présente invention a donc aussi pour objet une nouvelle composition médicamenteuse destinée notamment au traitement des affections susmentionnées, caractérisée par le fait qu'elle comporte, dans un support pharmaceutiquement acceptable, au moins un composé de formule (I) et/ou un de ses sels.

Les composés selon l'invention sont généralement administrés à une dose journalière d'environ 0,01 mg/kg à 100 mg/kg en poids corporel en 1 à 3 prises.

L'administration peut être effectuée par voie entérale, parentérale, topique ou oculaire. Par voie entérale, les médicaments peuvent se présenter sous forme de comprimés, de gelules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, les compositions peuvent se présenter sous forme de solutions ou suspensions pour perfusion ou pour injection.

Par voie topique, les compositions pharmaceutiques à base de composés selon l'invention sont destinées au traitement de la peau et des muqueuses et se présentent sous forme d'onguents, de crèmes, de laits, de pommades, de poudres, de tampons imbibés, de solutions, de gels, de sprays, de lotions ou de suspensions. Elles peuvent également se présenter sous forme de microsphères ou nanosphères ou vésicules lipidiques ou polymériques ou de patches polymériques ou d'hydrogels permettant une libération contrôlée.

Ces compositions par voie topique peuvent se présenter soit sous forme anhydre, soit sous forme aqueuse selon l'indication clinique.

Par voie oculaire, ce sont principalement des collyres.

Ces compositions contiennent au moins un composé de formule (I) telle que définie ci-dessus ou un des ses sels, à une concentration de préférence comprise entre 0,001 et 5 % par rapport au poids total de la composition.

Les composés de formule (I), selon l'invention, trouvent également une application dans le domaine cosmétique, en particulier dans l'hygiène corporelle et capillaire et notamment pour le traitement des peaux à tendance acnéique, pour la repousse des cheveux, l'anti-chute, pour lutter contre l'aspect gras de la peau ou des cheveux, dans la protection contre les effets néfastes du soleil ou dans le traitement des peaux physiologiquement sèches.

La présente invention vise donc également une composition cosmétique contenant, dans un support cosmétiquement acceptable, au moins un composé de formule (I) ou un de ses sels, cette composition se présentant notamment sous forme d'une crème, d'un lait, d'une lotion, d'un gel, de microsphères ou nanosphères ou vésicules lipidiques ou polymériques, d'un savon ou d'un shampooing.

La concentration en composé de formule (I), dans les compositions cosmétiques est comprise de préférence entre 0,001 et 3 % en poids.

Les compositions médicamenteuses et cosmétiques selon l'invention peuvent contenir des additifs inertes ou même pharmacodynamiquement ou cosmétiquement actifs ou des combinaisons de ces additifs et notamment : des agents mouillants, des agents dépigmentants tels que l'hydroquinone, l'acide azélaïque et l'acide caféïque, l'acide kojique, des agents émollients, des agents hydratants comme le glycérol, le PEG 400, la thiamorpholinone et ses dérivés ou l'urée ; des agents antiséborrhéiques ou antiacnéiques, tels que la S-carboxyméthylcystéine, la S-benzyl-cystéamine, leurs sels et leurs dérivés, la tioxolone ou le peroxyde de benzoyle ; des antibiotiques comme l'érythromycine et ses esters, la néomycine, la clindamycine et ses esters, les tétracyclines ; des agents anti-fongiques tels que le kétoconazole ou les polyméthylène-4,5 isothiazolinones-3 ; des agents favorisant la repousse des cheveux, comme le "Minoxidil" (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoin (5,5-diphényl-imidazolidine 2,6-dione) ; des agents anti-inflammatoires stéroïdiens et non stéroïdiens ; des caroténoïdes et, notamment le β-carotène ; des agents anti-psoriatiques tels que l'anthraline et ses dérivés et les acides eicosa-5,8,11,14-tétraynoique et eicosa-5,8,11-triynoïque, leurs esters et leurs amides ou des agents anti-irritants tels que des dérivés d'α-hydroxyacides et plus particulièrement les dérivés de l'acide mandélique.

Les compositions selon l'invention peuvent également contenir des agents d'amélioration de la saveur, des agents conservateurs tels que les esters de l'acide parahydroxybenzoïque, des agents stabilisants, des agents régulateurs d'humidité, des agents régulateurs de pH, des agents modificateurs de pression osmotique, des agents émulsionnants, des filtres UV-A et UV-B, des anti-oxydants tels que l'α-tocophérol, le butylhydroxyanisole ou le butylhydroxytoluène.

On va maintenant donner, à titre d'illustration et sans aucun caractère limitatif, plusieurs exemples de préparation des composés actifs de formule (I) selon l'invention ainsi que des exemples de compositions les contenant.

### EXEMPLE 1 : Chlorhydrate de l'acide 6-[3-(1-adamantyl) -4-(3-aminopropyloxy) phényl]-2-naphtoïque.

### a) 6-[3-(1-adamantyl)-4-(N-triphénylméthyl-3-aminopropyloxy)phényl] -2-naphtoate de méthyle

Dans un tricol, on introduit 360 mg (12 mmoles) d'hydrure de sodium (80% dans l'huile) et 50 ml de DMF. Sous courant d'azote, on ajoute goutte à goutte une solution de 4,1g (10 mmoles) de 6-[3-(1-adamantyl)-4-hydroxyphényl]-2-naphtoate de méthyle dans 75 ml de DMF et agite jusqu'à cessation du dégagement gazeux. On introduit ensuite goutte à goutte une solution de 42 g (11 mmoles) de N-triphénylméthyl-3-bromopropylamine dans 50 ml de DMF et agite à température ambiante 8 h. On verse le milieu réactionnel dans l'eau, extrait avec de l'éther éthylique, décante la phase organique, lave à l'eau, sèche sur sulfate de magnésium et évapore. Le résidu obtenu est purifié par chromatographie sur silice, élué avec un mélange de dichlorométhane et d'hexane (40-60). Après évaporation des solvants, on recueille 4,7 g (66%) du produit attendu, qui fond à 168-9°C.

### b) Acide 6-[3-(1-adamantyl-4-(N-triphénylméthyl -3-aminopropyloxy)phényl]-2-napthoïque

Dans un ballon, on introduit 4,5 g (6,3 mmoles) du produit précédent et 100 ml de soude méthanolique 2N. On chauffe à reflux durant 4 heures, évapore à sec, reprend le résidu par l'eau et acidifie à pH=1 avec de l'acide chlorhydrique concentré. On filtre le solide qui a précipité, le lave à l'eau, puis avec 20 ml d'acétone. Après séchage en présence de pentoxyde de phosphore, on recueille 4,1 g (93%) de l'acide attendu de point de fusion : 243-4°C.

### c) Acide 6-[3-(1-adamantyl)-4-(3-aminopropyloxy)phényl] -2-napthoïque

Dans un ballon, on introduit 2,8 g (4 mmoles) de l'acide précédent, 200 ml d'acide acétique et 100 ml d'acide chlorhydrique 6N. On chauffe à reflux durant 12 h, refroidit le mélange réactionnel et filtre le solide. Le solide obtenu est introduit dans 30 ml d'eau et on ajuste le pH à 5, filtre le solide, le lave à l'eau puis avec 100 ml d'acétone, sèche en présence de pentoxyde de phosphore. On recueille 1,6 g (89%) d'acide attendu qui fond en se décomposant à 296-7°C.

### d) Chlorhydrate de l'acide 6-[3-(1-adamantyl)-4-(3-aminopropyloxy) phényl]-2-naphtoïque.

Dans un ballon, on introduit 455 mg (1 mmole) du produit précédent et 100 ml de méthanol. On ajoute goutte à goutte une solution d'acide chlorhydrique dans l'alcool isopropylique (2N) jusqu'à pH=1 et évapore à sec le milieu réactionnel. Le résidu est trituré dans 20 ml d'acétone, le solide obtenu est filtré, lavé avec 50 ml d'éther éthylique et séché sous vide à 80°C. On recueille 430 mg (88%) du chlorhydrate attendu qui fond à 303-6°C en se décomposant.

### EXEMPLE 2 : 6-[3-(1-adamantyl)-4-(2,3-dihydroxypropyloxy)phényl] -2-naphtoate de méthyle.

### a) 3-(1-adamantyl)-4-(2,2-diméthyl-1,3-dioxolanne-4-méthyloxy) bromobenzène

A une solution de 10g (32,5 mmoles) de 3-(1-adamantyl)-4-bromophénol dans 140 ml de diméthylformamide contenant 4,95 g (35,8 mmoles) de carbonate de potassium, on additionne goutte à goutte sous agitation 11,2 g (39 mmoles) de 3-tosyloxy-1,2-propanediolacétonide. La réaction est laissée à 100°C durant la nuit puis est versée dans de l'eau glacée et extraite à l'éther. Après lavage de la phase organique, séchage et évaporation, le résidu est chromatographié sur silice dans le mélange dichlorométhane/hexane (50/50) pour conduire à 7,5 g (55%) de produit attendu fondant à 90,6°C.

### b) 6- [3-(1-adamantyl)-4-(2,2-diméthyl-1,3-dioxolanne-4-méthyloxy)phényl]-2-naphtoate de méthyle.

Dans un tricol sous azote contenant 962 mg de magnésium dans 100 ml de THF, on ajoute 7,4 g(17,6 mmoles) de 3-(1-adamantyl)-4-(2,2-diméthyl-1,3-dioxolanne-4-méthyloxy) bromobenzène dans 50 ml de tétrahydrofuranne et 300 ml de dibromoéthane. On chauffe à reflux pendant lh30, laisse refroidir et ajoute 5,09g (37,6 mmoles) de chlorure de zinc, agite pendant 1 h et ajoute successivement 9,97g (37,6 mmoles) de 6-bromo-2-naphtoate de méthyle dans 50 ml de THF, puis 300 mg de complexe NiCl₂/DPPE, On agite 8h à température ambiante, verse dans une solution aqueuse saturée de chlorure d'ammonium, extrait à l'éther, lave la phase organique et évapore. Le résidu est chromatographié sur silice dans le mélange hexane/dichlorométhane (70/30) pour conduire à 8,3g (89%) de dérivé attendu fondant à 116-118°C.

### c) 6-[3-(1-adamantyl)-4-(2,3-dihydroxypropyloxy)phényl]-2-naphtoate de méthyle.

4,8g (9,15 mmoles) de l'ester (2b) sont mis en suspension dans 90 ml d'une solution aqueuse d'acide formique à 40%. Le mélange est chauffé à 100°C pendant 48h. Le milieu réactionnel est versé dans l'eau et est extrait par de l'acétate d'éthyle. Après lavage à l'eau de la phase organique, séchage et évaporation, le résidu est chromatographié sur silice dans l'éluant CH₂Cl₂/THF, (90/10) pour conduire à 3,2 g (72%) du dérivé attendu fondant à 209,6°C.

### EXEMPLE 3 : Acide 6-[3-(1-adamantyl)-4-(2,3-dihydroxypropyloxy) phényl]-2-naphtoïque.

3g de l'ester obtenu dans l'exemple 2(c) sont mis en suspension en présence de 1,5g de soude dans 60 ml de méthanol. Le milieu réactionnel est chauffé à reflux pendant 24 h. Après évaporation du méthanol, le résidu est repris dans l'eau, acidifié à pH=1 (HCl concentré) et extrait par de l'acétate d'éthyle. La phase organique est lavée, séchée et évaporée pour conduire à 2,67g (92%) après recristallisation dans l'acétate d'éthyle du dérivé attendu fondant à 277,8°C.

### EXEMPLE 4 : 6-[3-(1-adamantyl)-4-méthoxycarbonylméthyloxyphényl] -2-naphtoate de benzyle.

### a) 6-[3-(1-adamantyl)-6-hydroxyphényl]-2-naphtoate de benzyle.

Dans un tricol sous azote, on place 39,85g (0,1 mole) d'acide 6-[3-(1-adamantyl)-4-hydroxyphényl]-2-naphtoïque dans 400 ml de DMF et ajoute par fractions 3,2 g de NaH à 80% dans l'huile. On laisse à température ambiante sous agitation 1 h puis ajoute goutte à goutte 13,2 ml (0,11 mole) de bromure de benzyle et laisse agiter à température ambiante durant la nuit. Le milieu réactionnel est versé dans de l'eau glacée, est extrait par 1,8 1 d'éther. La phase organique est lavée à l'eau, séchée et évaporée. Le résidu est chromatographié sur silice dans le mélange dichlorométhane hexane (70/30) pour conduire à 42,2g (79%) du dérivé attendu fondant à 185-186°C.

### b) 6-[3-(1-adamantyl)-4-méthoxycarbonylméthyloxyphényl] -2-naphtoate de benzyle.

A une solution de 8 g (16 mmoles) de 6-[3-(1-adamantyl)-4-hydroxyphényl]-2-naphtoate de benzyle dans 100 ml de DMF, on ajoute 136 mg d'hydrure de sodium à 80% dans l'huile. On laisse sous agitation durant 1h et ajoute 1,6 ml (16,4 mmole) de bromoacétate de méthyle. Le mélange réactionnel est laissé sous agitation durant la nuit puis est versé dans l'eau glacée et acidifié à pH 2 par de l'HCl concentré. Le solide est filtré et recristallisé dans l'éther. On recueille 8,52g (95%) du dérivé attendu fondant à 183°C.

### EXEMPLE 5 : Acide 6-[3-(1-adamantyl)-4-méthoxycarbonylméthyloxyphényl]-2-naphtoïque.

4,24 g (7,58 mmoles) du diester obtenu dans l'exemple 4 en solution dans le mélange dioxanne/acide acétique (99/1) sont traités par 800mg de Pd-C (10%) sous une pression de 7 bars d'hydrogène à 40°C pendant 6h. Le milieu réactionnel est alors filtré sur célite, évaporé, lavé à l'eau et recristallisé dans le mélange acétate/tétrahydrofuranne. On isole 2,94g (82%) d'un solide fondant à 283-285°C.

### EXEMPLE 6 : Acide 6-[3-(1-adamantyl)-4-carboxyméthyloxyphényl] -2-napthoïque.

1 g (2,1 mmoles) du monoester obtenu dans l'exemple 5 sont traités par 0,58g de KOH dans 50 ml de n-butanol. On chauffe à 100°C durant lh30, puis évapore le butanol, reprend le solide par 50 ml d'eau, acidifie à pH = 1 par HCl concentré et filtre. On isole après séchage, lavage à l'éther et recristallisation dans le THF, 720 mg du produit attendu fondant à 332-335°C.

### EXEMPLE 7 : 6-[3-(1-adamantyl)-4-(3-hydroxypropyloxy)phényl] -2-naphtoate de méthyle.

A 1 g (2,42 mmoles) de 6-[3-(1-adamantyl)-6-hydroxyphényl] -2-naphtoate de méthyle en solution dans 10 ml de DMF, on ajoute 77 mg (2,55 mmoles) d'hydrure de sodium à 80% dans l'huile. Après lh30 d'agitation, on ajoute à ce milieu réactionnel 230 ml (2,42 mmoles) de bromopropanol et laisse agiter 12 heures à température ambiante sous azote. Le milieu réactionnel est versé dans l'eau, acidifié à pH=1 par HCl concentré et extrait par de l'acétate d'éthyle. La phase organique est lavée à l'eau, séchée sur sulfate de magnésium, filtrée et évaporée. Le résidu est chromatographié sur silice dans le dichlorométhane, cristallisé dans le mélange dichlorométhane/hexane pour conduire à 600 mg (53%) du dérivé attendu fondant à 201,9°C.

### EXEMPLE 8 : Acide 6-[3-(1-adamantyl)-4-(3-hydroxypropyloxy)phényl] -2-naphtoïque.

0,57 g (1,21 mmole) de l'ester obtenu à l'exemple 7 en suspension dans 30 ml de n-butanol sont traités par 400 mg d'hydroxyde de potassium. Le mélange réactionnel est chauffé 3 h à 100°C, puis est évaporé à sec, est repris par 50 ml d'eau et lavé à l'éther. La phase aqueuse est acidifiée à pH=1 avec de l'HCl concentré et extraite par 600 ml d'éther. La phase organique est lavée à l'eau, séchée sur sulfate de magnésium, filtrée et évaporée pour conduire à 0,49g (90%) de l'acide attendu fondant à 277-279°C.

### EXEMPLE 9 : 6-[3-(1-adamantyl)-4-benzyloxycarbonylphényl] -2-naphtoate de méthyle.

### a) 6- 3-(1-adamantyl)-6-trifluorométhylsulfonyloxyphényl -2-naphtoate de méthyle.

A 19,8 g (48 mmoles) de 6-[3-(1-adamantyl)-6-hydroxyphényl] -2-naphtoate de méthyle dans 200 ml de CH₂Cl₂, on ajoute 59 mg de diméthylaminopyridine et 12 ml de pyridine. A cette solution sous azote refroidie à -70°C, on ajoute goutte à goutte 9,7 ml (0,057 mmole) d'anhydride trifluorométhanesulfonique dans 10 ml de CH₂Cl₂. On laisse remonter sous agitation à température ambiante pendant la nuit. Le milieu réactionnel est versé dans de l'eau glacée et est extrait par 1 1 d'éther. La phase organique est lavée par une solution d'acide (HCl 1N) puis est rincée à l'eau, séchée sur sulfate de magnésium, filtrée et évaporée. On isole après chromatographie sur silice dans le mélange dichlorométhane/ hexane (40/60) et recristallisation dans le même solvant 23,5g (90%) du produit attendu fondant à 185,5°C.

### b) 6 [3-(1-adamantyl)-4-benzyloxycarbonylphényl]-2-naphtoate de méthyle.

Dans un réacteur autoclave, on place 13,11 g (0,024 mmole) du triflate obtenu ci-dessus dans 100 ml de DMF, 6,70 ml (0,048 mole) de triéthylamine, 270 mg (5% molaire) d'acétate de palladium, 1,33g (2,4 mmoles) de diphénylphosphinoferrocène (DPPF) et 25 ml d'alcool benzylique. Le mélange réactionnel est chauffé à 70°C sous 3 bars de monoxyde de carbone pendant 6 h. On dilue alors la réaction par du chlorure de sodium saturé, extrait par 1 1 d'éther, lave la phase organique HCl 1N, puis à l'eau, sèche sur sulfate de magnésium et évapore. On isole après chromatographie sur silice dans le mélange CH₂Cl₂/hexane (40/60). On obtient 9,15 g (72%) du dérivé attendu fondant à 170°C.

### EXEMPLE 10 : 6-[3-(1-adamantyl)-4-carboxyphényl]-2-naphtoate de méthyle.

Dans un réacteur autoclave, on place 9,02 g (17 mmoles) du diester obtenu à l'exemple 9(b) dans 90 ml de dioxanne et 5 ml d'acide acétique. On ajoute alors 900 mg de Pd-C (10%) et traite le mélange à 70°C sous agitation par une pression de 7 bars d'hydrogène pendant 4 h. Après élimination du catalyseur, le filtrat est évaporé et le résidu est lavé à l'eau et à l'hexane pour conduire à 6,81g (91%) du dérivé attendu fondant à 239-240°C.

### EXEMPLE 11 : 6-[3-(1-adamantyl)-4-hydroxyméthylphényl] -2-naphtoate de méthyle.

6,2 g (0,014 mole) de l'acide obtenu à l'exemple 10 sont dissous dans 30 ml de THF et traités par 49 ml de BH3 (1M) dans le THF. Le milieu réactionnel est chauffé à reflux pendant 12 h puis est évaporé, repris par 900 ml d'eau et traité avec 35 ml d'HCl 1N. On extrait par 800 ml d'acétate d'éthyle, lave la phase organique à l'eau et évapore. Le résidu est chromatographié sur silice dans le dichlorométhane pour conduire à 4,45g (75%) du dérivé attendu fondant à 212°C.

### EXEMPLE 12 : Acide 6-[3-(1-adamantyl)-4-hydroxyméthylphényl] -2-naphtoïque.

4,40 g (103 mmoles) de l'ester obtenu à l'exemple 11 dans 50 ml de méthanol sont traités par 4g d'hydroxyde de sodium sous agitation durant lh30. Après le même traitement qu'à l'exemple 8 et recristallisation dans le mélange éthanol/eau, on isole 3g (96%) du produit attendu fondant à 267-270°C.

### EXEMPLE 13 : Acide 6-[3-(1-adamantyl)-4-acétoxyméthylphény] -2-naphtoïque.

2,42g (5,87 mmoles) de l'alcool obtenu à l'exemple 12 dans 12 ml de pyridine sont traités par 0,63 ml de chlorure d'acétyle durant 30 mn. Le milieu réactionnel est versé dans de l'eau glacée, extrait par de l'acétate d'éthyle. La phase organique est lavée à l'eau, séchée sur sulfate de magnésium et évaporée pour conduire après recristallisation dans le mélange acétate d'éthyle/hexane à 1,67g (63%) d'un produit fondant à 251°C.

### EXEMPLE 14 : 6-[3-(1-adamantyl)-4-méthoxycarbonylphényl] -2-naphtoate de méthyle.

3g (5,5 mmoles) du triflate (9a) obtenu à l'exemple 9 dans 30 ml de DMF et 1,54 ml de triéthylamine et 8 ml de méthanol sont traités par 63,8 mg (5% molaire) d'acétate de palladium et 304,7 mg (10% molaire) de diphénylphosphinoferrocène puis sont soumis à une pression de 1 bar de monoxyde de carbone pendant 30 mn à 70°C. Le milieu réactionnel est alors versé dans une solution de chlorure de sodium saturé et extrait par de l'éther. La phase organique est lavée par HCl 1N, puis à l'eau, séchée et évaporée. Le résidu est chromatographié sur silice pour conduire à 1,9g (75%) du dérivé attendu fondant à 181-182°C.

### EXEMPLE 15 : Acide 6-[3-(1-adamantyl)-6-méthoxycarbonylphényl] -2-naphtoïque.

A 1,92g (4,22 mmoles) du diester obtenu à l'exemple 14 dans 25 ml de méthanol on ajoute 0,34g d'hydroxyde de sodium et laisse à reflux durant 24 heures. Après le même traitement qu'à l'exemple 8 et recristallisation dans le mélange éther isopropylique-acétate d'éthyle, on isole 1,7g (91%) du dérivé attendu fondant à 272-273°C.

### EXEMPLE 16 : Acide 6-[3-(1-adamantyl)-6-carboxyphényl] -2-naphtoïque.

A une solution de 1,20 g (2,72 mmoles) du diester obtenu à l'exemple 15 dans 50 ml de n-butanol, on ajoute 2g d'hydroxyde de potassium et chauffe à 110°C pendant 24 heures. Après le même traitement qu'à l'exemple 8, et recristallisation dans le mélange acétate d'éthyle THF, on isole 605 mg (52%) du dérivé attendu fondant à 333-335°C.

### EXEMPLE 17 : Acide 6-[3-(1-adamantyl)-4-carboxamidophényl] -2-napthoïque.

### a) 6-[3-(1-adamantyl)-4-carboxamidophényl]-2-naphtoate de méthyle.

A 1,91 g (4,34 mmoles) de l'acide obtenu à l'exemple 10, on ajoute 15 ml de chlorure de thionyle et chauffe à reflux 40 mn. On évapore à sec et reprend le solide par 20 ml de dichlorométhane et fait barbotter de l'ammoniac gazeux à 0°C. On laisse revenir à température ambiante le milieu réactionnel durant la nuit, puis ajoute 800 ml de dichlorométhane, lave à l'eau, sèche sur sulfate de magnésium et évapore. On isole après chromatographie sur silice dans gradient d'éluant hexane/éther (50/50) jusqu'à éther (100%) et obtient 0,94g (49%) du produit attendu fondant à 285°C.

### b) Acide 6-[3-(1-adamantyl)-4-carboxamidophényl]-2-napthoïque.

Suivant le procédé décrit à l'exemple 15, 935 mg (2,1 mmoles) de l'ester précédent sont saponifiés. Le solide obtenu est recristallisé dans l'éthanol absolu. On obtient 450 mg (50%) du dérivé attendu fondant à 322-325°C.

### EXEMPLE 18 : 6-[3-(1-adamantyl)-4-méthoxycarbonyléthénylphényl] -2-naphtoate de benzyle.

### a) 6- [3-(1-adamantyl)-4-trifluorométhylsulfonyloxyphényl] -2-naphtoate de benzyle.

A une solution de 23,5 g (48 mmoles) du phénol obtenu à l'exemple 4(a) dans 150 ml de CH₂Cl₂, on ajoute 59 mg de diméthylaminopyridine, 12 ml de pyridine, puis place cette solution à -70°C et ajoute 9,70ml (57,7 mmoles) d'anhydride trifluorométhanesulfonique dans 10 ml de CH₂Cl₂. On laisse le milieu réactionnel sous agitation remonter à température ambiante durant la nuit, puis le verse dans de l'eau glacée et extrait par 1 1 d'éther. La phase organique est lavée à l'eau, séchée et évaporée. Le résidu est chromatographié sur silice dans le mélange CH₂Cl₂/hexane (40/60). On isole après recristallisation dans le mélange dichlorométhane/hexane 25,86g (87%) du dérivé attendu fondant à 132°C.

### b) 6-[3-(1-adamantyl)-4-(méthoxycarbonyléthényl)phényl] -2-naphtoate de benzyle.

A 11,53g (18,58 mmoles) du composé obtenu ci-dessus dans 100 ml de DMF, on ajoute 6,7 ml (74,3 mmoles) d'acrylate de méthyle, 15,5 ml de triéthylamine (0,11 mmole) et 521 mg de Pd(PPh₃)₂Cl₂. On chauffe à 90°C pendant 5 jours. Le milieu réactionnel est versé dans de l'eau glacée et est extrait par 1,2 1 d'acétate d'éthyle. La phase organique est lavée, séchée et évaporée pour conduire après chromatographie sur silice dans le mélange CH₂Cl₂/hexane (40/60) à 6,2 g (62%) du dérivé attendu fondant à 159°C.

### EXEMPLE 19 : Acide 6-[3-(1-adamantyl)-4-(méthoxycarbonyléthylphényl]-2-naphtoïque.

A une solution de 2,23g (4 mmoles) du diester obtenu à l'exemple 18(b) dans 50 ml de dioxanne et 1 ml d'acide acétique, on ajoute 334 mg de Pd-C(10%) et agite le mélange réactionnel sous une pression de 7 bars d'hydrogène à 70°C pendant 5 heures. On filtre le milieu réactionnel sur célite et évapore. Le filtrat est lavé à l'eau et séché pour conduire après recristallisation dans l'acétate d'éthyle à 1,65g (82%) du dérivé attendu fondant à 259°C.

### EXEMPLE 20 : 6-[3-(1-adamantyl)-4-(2,3-époxypropyl)phényl] -2-naphtoate de benzyle.

### a) 6-[3-(1-adamantyl)-4-(2-propényl)phényl]-2-naphtoate de benzyle.

A une solution de 1,24g (0,02 mole) du composé obtenu à l'exemple 18(a) dans 60 ml de DMF on ajoute 6,4 ml (0,02 mole) d'allyltributylétain, 1,70g de chlorure de lithium et laisse agiter sous atmosphère inerte à température ambiante 30mn. On ajoute alors 280 mg (0,4 mmole) de PdCl₂(P(C6H5)₃)₂ et chauffe progressivement jusqu'à 100°C pendant 40 mn. Le milieu réactionnel est versé dans de l'eau glacée, et extrait par 1 1 d'éther. La phase organique est lavée à l'eau, séchée et évaporée. Le résidu est chromatographié sur silice dans le mélange hexane/éther (95/5) pour conduire à 11,6g (95%) du dérivé attendu fondant à 115°C.

### b) 6-[3-(1-adamantyl)-4-(2,3-époxypropyl)phényl]-2-naphtoate de benzyle.

2,56g (5 mmoles) du dérivé obtenu ci-dessus dissous dans 25 ml de CH₂Cl₂ sont traités par 1,52g d'acide métachloroperbenzoïque à 0°C. On laisse remonter à température ambiante en 12h sous agitation. Le milieu réactionnel est dilué par du dichlorométhane et lavé par de l'hydrogénosulfite de sodium puis par du bicarbonate de sodium. La phase organique est séchée et évaporée. Le résidu obtenu est chromatographié sur silice dans le mélange CH₂Cl₂/hexane (60/40) pour conduire à 1,91g (72%) du dérivé attendu fondant à 118-120°C.

### EXEMPLE 21 : Acide 6-[3-(adamantyl)-4-(2-hydroxypropyl)phényl] -2-naphtoïque.

1,40g (2,65 mmoles) du dérivé obtenu à l'exemple 20(b) sont dissous dans 50 ml de dioxanne et 1 ml d'acide acétique et sont hydrogénés en présence de 280 mg de Pd-C (10%) à 70°C sous une pression de 7 bars pendant 6h. Après filtration du palladium et évaporation, le résidu est chromatographié dans le mélange éther/hexane (80/20) pour conduire après recristallisation dans l'hexane à 523 mg (45%) du produit attendu fondant à 282°C.

### EXEMPLE 22 : Acide 6-[3-(1-adamantyl)-4-(3-méthoxy -2-hydroxypropyl)phény]-2-naphtoïque.

1g (1,89 mmoles) de l'ester obtenu à l'exemple 20(b), dans 100 ml du mélange méthanol/THF (1/1) sont traités par 230 mg d'hydroxyde de sodium et sont laissés sous agitation une nuit à température ambiante puis 3h à reflux. Après un traitement identique à celui effectué à l'exemple 12, et recristallisation dans le mélange acétate d'éthyle/hexane, on isole 0,65g (78%) du dérivé attendu fondant à 240-242°C.

### EXEMPLE 23 : Acide 4-[(E)-2-(3-(1-adamantyl) -4-méthoxyphényl)propényl]benzoïque.

### a) 3-(1-adamantyl)-4-méthoxyphényléthanone

15,75 g (0,055 moles) d'acide 3-(1-adamantyl)-4-méthoxyphényl benzoïque en solution dans 150 ml de toluène sont traités par 7,3 ml de chlorure de thionyle et sont chauffés à 100°C durant 3h30. Le milieu réactionnel est évaporé à sec puis on ajoute sous azote à ce résidu d'évaporation 30 ml d'hexaméthylphosphoramide, 8 ml (0,0575 moles) de tétraméthylétain et 22 mg de PhCH₂Pd(PPh₃)₂Cl. Le milieu réactionnel est chauffé à 65°C pendant 30 mn puis est laissé sous agitation à température ambiante pendant une nuit. Le milieu réactionnel est versé dans l'eau et extrait à l'éther. On isole après chromatographie sur silice dans l'éluant dichlorométhane/hexane (60/40), 10,47 g (71 %) du dérivé attendu fondant à 138-140°C.

### b) Acide 4-[(E)-2-(3-(1-adamantyl)-4-méthoxyphényl)propényl] benzoïque.

Dans un tricol de 250 ml sous azote, on place 0,90 g (0,03 moles) d'hydrure de sodium à 80 % dans l'huile et ajoute goutte à goutte 8,58 g (0,03 moles) de 4-méthoxycarbonylbenzylphosphonate de diéthyle dans 15 ml de diméthoxyéthane. On ajoute ensuite 8,53 g (0,03 moles) du dérivé obtenu en (a) ci-dessus et chauffe 10h à 80°C puis à température ambiante durant 65 heures. On verse ensuite le milieu réactionnel dans l'eau, extrait par 1 1 d'acétate d'éthyle, rince la phase aqueuse jusqu'à pH neutre et évapore. On isole après chromatographie sur silice dans le mélange hexane/acétate d'éthyle (95/5) 3g de 4-[(Z)-2-(3-(1-adamantyl) -4-méthoxyphényl)propényl]benzoate de méthyle et 0,45 g de 4-[(E)-2-(3-(1-adamantyl)-4-méthoxyphényl)-propényl]benzoate de méthyle.

0,45 g (1,1 moles) de l'ester méthylique de configuration (E) obtenu ci-dessus sont traités par 0,2 g d'hydroxyde de sodium dans 5 ml de méthanol et chauffés à reflux 3h30. Après le même traitement qu'à l'exemple 1(b) et recristallisation dans un mélange THF/éthanol, on isole 0,2 g (52 %) d'acide 4-[(E)-2-(3-(1-adamantyl) -4-méthoxyphényl)propény]-benzoïque fondant à 307-308°C.

### c) Acide 4-[(Z)-2-(3-(1-adamantyl)-6-méthoxyphényl-propényl] benzoïque.

3 g (7,2 moles) de 4-[(Z)-2-(3-(1-adamantyl)-4-méthoxyphényl) propényl]-benzoate de méthyle sont traités par 4 g d'hydroxyde de sodium dans 50 ml de méthanol et chauffés à reflux 2h30. Après évaporation, le milieu réactionnel est traité dans les conditions décrites à l'exemple 1(b). On isole après recristallisation dans l'éthanol absolu 2,4 g (84%) d'acide 4-[(Z)-2-(3-(1-adamantyl) -4-méthoxyphényl)-propényl]benzoïque fondant à 267°C.

### EXEMPLE 24

### 4-[(E)-2-(3-(1-adamantyl)-4-méthoxyphényl)propényl]benzoate de benzyle

2 g (5 mmoles) d'acide 4-[Z)-2-(3-(1-adamantyl)-4- methoxyphenyl)propényl] benzoïque obtenu à l'exemple 23(c) dans 900 ml de THF sont irradiés sous UV à l'aide d'une lampe à mercure HANOVIA (550W) pendant 10 h à température ambiante, pour conduire à un mélange 1/1 d'isomères E et Z. Après évaporation du THF, ce mélange est traité par 190 mg (6 mmoles) de NaH à 80% dans l'huile et 0,83 ml (7 mmoles) de bromure de benzyle. Après le même traitement qu'à l'exemple 1(a) suivi d'une recristallisation dans l'hexane, on isole 0,54 g de 4-[(E)-2-(3-(1-adamantyl)-4-méthoxyphényl)propényl]benzoate de benzyle fondant à 230-240°C.

### EXEMPLE 25

### Acide 4-[(E)-2-(3-(1-adamantyl)-4-hydroxyphényl)-1-propényl]benzoïque

### a) 3-(1-adamantyl)-4-tert-butyldiméthylsilyloxyphényléthanone

14,5 g (37,5 mmoles) d'acide 3-(1-adamantyl)-4-tert-butyldiméthylsilyloxy benzoïque dans 150 ml d'éther sont traités à -20°C par 75 mmoles de méthyl lithium (1,6 M dans Et2O). Le mélange réactionnel est laissé sous agitation à température ambiante pendant une nuit sous azote, puis est versé dans l'eau glacée et extrait par 500 ml d'éther. La phase organique est lavée à l'eau, séchée sur sulfate de magnésium et évaporée pour conduire après chromatographie sur silice, dans l'éluant éther/hexane (50:50) à 12 g (83%) du dérivé attendu fondant à 114,5 °C.

### b) 3-(1-adamantyl)4-tert-butyldiméthylsilyloxyphényléthanol

11,5 g (30 mmoles) du dérivé obtenu à l'exemple 25(a), en solution dans 100 ml de THF sont traités par 1,1 g (30 mmoles) de NaBH4 pendant 2 h à température ambiante. Le milieu réactionnel est évaporé, repris dans 100 ml d'éther, lavé à l'eau, séché et évaporé pour conduire à 9 g (78%) du produit attendu fondant à 71-72 °C.

### c) Bromure de 3-(1-adamantyl)-4-tert-butyldiméthylsilyloxyéthylphosphonium

9 g (23,3 mmoles) du dérivé obtenu à l'exemple 25(b), en solution dans 50 ml de méthanol, sont traités par 8,2 g (23,9 mmoles) de bromhydrate de triphénylphosphine et agités sous azote à température ambiante, pendant une nuit. Le milieu réactionnel est évaporé, puis le résidu est trituré dans l'éther pour conduire à 15,5 g (93%) du dérivé attendu fondant à 221-222°C.

### d) 4-[(E)-2-(3-(1-adamantyl)-4-tert-butyldiméthylsilyloxyphényl)-1-propényl]benzoate de méthyle

3 g (4,2 mmoles) du dérivé obtenu à l'exemple 25(c), en solution dans 50 ml de THF sont traités à -40°C par 2,9 ml de n-butyllithium (1,6 M dans l'hexane). On laisse remonter le milieu réactionnel à 20°C pendant 6 h et ajoute 690 mg (4,2 mmoles) de p-carboxaldéhydebenzoate de méthyle dans 10 ml de THF et laisse réagir sous azote, à température ambiante, durant une nuit. On isole, après le même traitement que pour l'exemple 1(a), suivi d'une recristallisation dans l'heptane, 0,6 g du dérivé attendu, fondant à 144°C.

### e) Acide 4-[(E)-2-(3-(1-adamantyl)-4-hydroxyphényl)-1-propényl]benzoïque

0,6 g (1,16 mmole) du dérivé obtenu à l'exemple 25(d), en solution dans 5 ml de THF et 5 ml de méthanol, sont traités par 12 ml de soude 2N. La réaction est laissée sous agitation à température ambiante pendant 24 h. On isole après le même traitement qu'à l'exemple 8, suivi d'une recristallisation dans le mélange cyclohexane/diisopropyléther, 150 mg (32%) du dérivé attendu, fondant à 245-246°C.

### EXEMPLE 26

### 6-[3-(1-adamantyl)-4-méthoxycarbonylphényl]-2-naphtylcarboxamide

### a) Chlorure d'acide 6-[3-(1-adamantyl)-4-méthoxycarbonylphényl]-2-naphtoïque

2,1 g (4,77 mmoles) de l'acide obtenu à l'exemple 15, dans 60 ml de toluène à 50°C, sont traités par 0,70 ml (9,65 mmoles) de chlorure de thionyle. Le mélange réactionnel est chauffé à 110°C pendant 6 h, puis évaporé à sec pour conduire à 2,22 g du chlorure d'acide attendu.

### b) 6-[3-(1-adamantyl)-4-méthoxycarbonylphényl]-2-naphtylcarboxamide

A 10 ml d'une solution aqueuse d'ammoniaque à 33%, on ajoute goutte-à-goutte 0,74 g (1,61 mmoles) du chlorure d'acide obtenu à l'exemple 26(a). Le mélange réactionnel est agité à température ambiante pendant une nuit puis versé dans l'eau et extrait par de l'acétate d'éthyle. La phase organique est rincée à l'eau jusqu'à pH neutre, séchée sur sulftate de magnésium et évaporée. On isole, après chromatographie sur silice dans l'éluant dichlorométhane/méthanol (95:5) 347 mg (50%) du dérivé attendu, fondant à 270-272°C.

### EXEMPLE 27

### 4-[(E)-2-(3-(1-méthylcyclohéxyl)-4-hydroxyphényl)propényl]benzoate d'éthyle

### a) 3-(1-méthylcyclohéxyl)-4-tert-butyldiméthylsilyloxyphényléthanone

28,82 g (0,083 mole) d'acide 3-(1-méthylcyclohéxyl)-4- tert-butyldiméthylsilyloxy benzoïque, dans 300 ml d'éther sont traités à -20°C, sous azote, par 0,166 mole de méthyllithium (1,6 M dans l'éther). Le mélange réactionnel est agité sous azote durant la nuit, puis est traité comme pour l'exemple 25(a). Après filtration du résidu sur silice, on isole 22 g (76%) du produit attendu, sous forme d'huile jaune.

RMN (1H) : dppm (CDCl3): 0,36(6H,s);1,04(9H,s);1,31(3H,s);1,47-1,75(8H,m);2,19(2H,m); 2,56(3H,s);6,84(1H,d);7,71(1H,d);7,99(1H,s).

### b) Isomères Z et E du 4-[2-(3-(1-méthylcyclohéxyl)-4-tert-butyldiméthylsilyloxy phényl)propényl]benzoate d'éthyle

A une suspension de 2,16 g (72 mmoles) de NaH (80% dans l'huile), dans 50 ml de THF sous argon, on ajoute goutte à goutte le mélange constitué par 21 g (60 mmoles) de la cétone obtenue à l'exemple 27(a), 21,6 g (60 mmoles) de 4-éthoxycarbonylbenzylphosphonate de diéthyle et 2,64 g d'éther couronne (15-crown 5) dans 400 ml de THF. Le milieu réactionnel est agité sous azote, à température ambiante, pendant 36 h. Après le même traitement qu'à l'exemple 23(b) (réaction de Wittig-Homer), on isole 10,5 g (35,5%) de l'isomère Z et 2,52 g (8,5%) de l'isomère E, chacun sous forme d'huile jaunâtre.

7,92 g (16 mmoles) de l'isomère Z sont irradiés sous UV, dans les conditions décrites à l'exemple 24, pendant 24 h pour conduire à un mélange 1/1 d'isomères Z et E. On isole, après chromatographie sur silice dans l'éluant hexane/éther (97:3) 3,09 g du 4-[(Z)-2-(3-(1-méthylcyclohéxyl)-4-tert- butyldiméthylsilyloxy phényl)propényl]benzoate d'éthyle sous forme d'huile, et 2,7 g 4-[(E)-2-(3-(1-méthylcyclohéxyl)-4-tert-butyldiméthylsilyloxyphényl)propényl]benzoate d'éthyle sous forme d'huile.

RMN (1H) : Isomère Z d ppm (CDCl3):
0,30(6H,s);0,88(6H,m);1(9H,s);1,18(2H,s);1,23(2H,m);1,33(3H,t); 1,99(2H,m);2,2(3H,s);4,30(2H,q);6,41(1H,s);6,88(1H,d);6,90(1H,d);6,92(1H,s); 6,96(2H,d);7,74(2H,d).

Isomère E d ppm (CDCl3):
0,31(6H,s);0,85(4H,m);1(9H,s);1,24(3H,s);1,31(2H,s);1,38(3H,t);

1,73(2H,m);2,15(2H,m);2,26(3H,s);4,36(2H,q);6,76(1H,s);6,7 9(1H,d);7,23(1H,d);7,39(2H,d);7,46(1H,s);8(2H,d).

### c) 4-[(E)-2-(3-(1-méthylcyclohéxyl)-4-hydroxyphényl)propényl]benzoate d'éthyle

7,22 g (14,6 mmoles) de l'isomère E obtenu à l'exemple 27(b) dans 40 ml de THF, sont traités par 16,5 mmoles de fluorure de tétrabutylammonium. On laisse sous agitation, à température ambiante, pendant 1 h. 30. On isole, après le même traitement qu'à l'exemple 4(a), suivi d'une cristallisation dans l'hexane 4,23 g (76%) du dérivé attendu, fondant à 131,2°C.

### EXEMPLE 28

### 4-[(E)-2-(3-(1-méthylcyclohéxyl)-4-méthoxyphényl)propényl]benzoate d'éthyle

2,20 g (5,84 mmoles) du phénol obtenu à l'exemple 27(c), en solution dans 20 ml de diméthylformamide (DMF), sont traités par 194 mg (6,42 mmoles) d'hydrure de sodium (80% dans l'huile), puis par 0,365 ml d'iodure de méthyle. La réaction est agitée sous azote, à température ambiante, pendant 1 h. 30 puis est traitée comme à l'exemple 4(a). On obtient, après chromatographie sur silice, dans l'éluant dichlorométhane/hexane (60:40) et recristallisation dans l'hexane, 1,56 g (81%) du dérivé attendu, fondant à 60-62°C.

### EXEMPLE 29

### Acide 4-[(E)-2-(3-(1-méthylcyclohéxyl)-4-méthoxyphényl)propényl]benzoïque

1,71 g (4,36 mmoles) du dérivé obtenu à l'exemple 28, sont traités par 2,06 g d'hydroxyde de sodium dans 25 ml de méthanol. Le milieu réactionnel est agité 24 h à température ambiante, puis est chauffé à reflux, pendant 1 h. Après le même traitement qu'à l'exemple 8 on isole 1,20 g (75%) du dérivé attendu fondant à 242-244°C.

### EXEMPLE 30

### 4-[(Z)-2-(3-(1-méthylcyclohéxyl)-4-hydroxyphényl)propényl]benzoate d'éthyle

2,20 g (4,46 mmoles) de l'isomère Z obtenu à l'exemple 27(b), en solution dans 40 ml de THF, sont traités par 4,91 mmoles de fluorure de tétrabutylammonium dans les conditions décrites à l'exemple 27(c), pour conduire après cristallisation dans l'hexane à 1,25 g (74%) du dérivé attendu, fondant à 123,5°C.

### EXEMPLE 31

### 4-[(Z)-2-(3-(1-méthylcyclohéxyl)-4-méthoxyphényl)propényl]benzoate d'éthyle

1,23 g (3,25 mmoles) du dérivé obtenu à l'exemple 30, en solution dans 15 ml de DMF, sont traités par 533 mg de carbonate de potassium et 0,75 ml d'iodure de méthyle. Le milieu réactionnel est agité à température ambiante, pendant 48 h., puis chauffé à 50°C pendant 24 h. Après le même traitement qu'à l'exemple 2(a), suivi d'une chromatographie sur silice dans l'éluant hexane/éther (95:5), on isole 709 mg (56%) du dérivé attendu sous forme d'huile jaunâtre.
RMN (1H) : dppm (CDCl3):
1,1(2H,m);1,19(3H,s);1,25(4H,m);1,31(3H,t);1,34(2H,m);1,99(2H,m);2,22(3H,s);3,81 (3H,s);4,31(2H,q);6,44(1H,s);6,80(1H,d);6,98(4H,m);7,76(2H,d).

### EXEMPLE 32

### Acide 4-[(Z)-2-(3-(1-méthylcyclohéxyl)-4-méthoxyphényl)propényl]benzoïque

0,69 g (1,76 mmole) de l'ester obtenu à l'exemple 31, dans 15 ml de méthanol, sont traités par 1,58 g d'hydroxyde de sodium. Le mélange réactionnel est agité à 40°C pendant 2 h. On isole, après le même traitement qu'à l'exemple 8, 462 mg (72%) du dérivé attendu, fondant à 175-176°C.

### EXEMPLE 33

### 4-[(Z)-2-(3-tert-butyl-4-méthoxyphényl)propényl]benzoate d'éthyle

### a) 3-tert-butyl-4-méthoxyphényléthanone

6,23 g (30 mmoles) d'acide 3-tert-butyl-4-méthoxyphényl benzoïque sont traités par 48 ml de méthyl lithium (1,6 M dans l'éther), dans les conditions décrites à l'exemple 25(a) pour conduire à 6,01 g (97%) du dérivé attendu, sous forme d'huile jaunâtre.

RMN (1H) : d ppm (CDCl3): 1,39(9H,s);2,56(3H,s);3,91(3H,s);6,89(1H,d);7,82(1H,d); 7,94(1H,d).

### b) 4-[(Z)-2-(3-tert-butyl-4-méthoxyphényl)propényl]benzoate d'éthyle

6,01 g (29 mmoles) de la cétone obtenue à l'exemple 33(a) sont mis à réagir sur 10,5 g (35 mmoles) de 4- éthoxycarbonylbenzyl phosphonate de diéthyle dans les conditions décrites à l'exemple 27(b), pour conduire à 3,86 g (38%) du dérivé attendu, sous forme d'huile jaunâtre et à 5,42 g (53%) de l'isomère 4-[(E)-2-(3-tert-butyl-4- méthoxyphényl)propényl]benzoate d'éthyle fondant à : 71,5°C

RMN (1H) : dppm (CDCl3):
1,26(9H,s);1,32(3H,t);2,22(3H,s);3,85(3H,s);4,30(2H,q);6,44 (1H,s);6,80(1H,d);7,03(4H,m);7,78(2H,d).

### EXEMPLE 34

### Acide 4-[(Z)-2-(3-tert-butyl-4-méthoxyphényl)propényl]benzoïque

1,21 g (3,43 mmoles) de l'isomère Z obtenu à l'exemple 33(b) dans 20 ml de méthanol, sont traités par 1,60 g d'hydroxyde de sodium et chauffé à reflux 1 h. 30. On isole, après le même traitement qu'à l'exemple 8, suivi d'une recristallisation dans le mélange éthanoVeau, 0,96 g (87%) du dérivé attendu fondant à : 182-184°C.

### EXEMPLE 35

### Acide 4-[(E)-2-(3-tert-butyl-4-hydroxyphényl)propényl]benzoïque

A 2 g (5,67 mmoles) de l'ester E, obtenu à l'exemple 33(b) dans 40 ml de DMF, on ajoute 1,84 g de méthanethiolate de lithium. Le mélange réactionnel est agité sous azote à 120°C, pendant 4 h. Après le même traitement qu'à l'exemple 8, suivi d'une chromatographie dans l'éluant éther/hexane (80:20), on isole 1,69 g d'un mélange d'isomères d'acide 4-[-2-(3-tert-butyl-4-hydroxyphényl)propényl]benzoïque E et Z dans les proportions respectives (83:17). Une recristallisation dans le mélange hexane/éther conduit à 225 mg du dérivé attendu, fondant à : 209°C.

### EXEMPLE 36

### Acide 4-[(E)-2-(3-(1-méthylcyclohéxyl)-4-hydroxyphényl)éthényl]benzoïque

### a) 4-tert-butyldiméthylsilyloxy-3-(1-méthylcyclohéxyl)benzaldéhyde

Dans un tricol de 500 ml, on place 1,54 g de magnesium, puis additionne par une ampoule à brome, une solution contenant 22,09 g (57,7 mmoles) de 4-tert-butyldiméthylsilyloxy-3(1-méthylcyclohéxyl)bromobenzène. La réaction est activée par quelques gouttes de dibromoéthane. On ajoute ensuite 4,45 ml (57 mmoles) de DMF et laisse agiter 30 min à température ambiante. Le milieu réactionnel est versé dans l'eau puis extrait par de l'acétate d'éthyle. Après rinçage de la phase organique à l'eau, séchage sur sulfate de magnésium, évaporation et chromatographie sur silice dans l'éluant dichlorométhane/hexane (50:50), on isole 16 g (84%) du dérivé attendu.

RMN (1H) : d ppm (CDCl3): 0,34(6H,s);1,02(9H,s);1,29(3H,s);1,41-1,73(8H,m);2,08(2H,m); 6,88(1H,d);7,60(1H,d);7,83(1H,d).

### b) 4-[(E)-2-(3-(1-méthylcyclohéxyl)-4-tert-butyldiméthylsilyloxyphényl)éthényl]benzoate d'éthyle

5 g de l'aldéhyde obtenu à l'exemple 36(a) et 4,52 g (15,06 mmoles) d'éthoxycarbonylbenzylphosphonate de diéthyle en solution dans 45 ml de THF, sont traités par 0,45 g d'hydrure de sodium (80% dans l'huile), et 0,05 ml de 15-crown 5, dans les conditions décrites à l'exemple 27(b). On isole, après le même traitement et chromatographie sur silice dans l'éluant dichlorométhane/hexane (20:80), 4 g (56%) du dérivé attendu fondant à : 88,7°C.

### c) 4-[(E)-2-(3-(1-méthylcyclohéxyl)-4-hydroxyphényl)éthényl]benzoate d'éthyle

3,59 g (7,5 mmoles) du dérivé obtenu à l'exemple 36(b), en solution dans 35 ml de THF, sont traités par 7,5 ml de fluorure de tétrabutylammonium, dans les conditions décrites à l'exemple 27(c), pour conduire après le même traitement, suivi d'un traitement dans l'hexane, à 2,1 g (77%) du dérivé attendu, fondant à 148,6°C.

### d) Acide 4-[(E)-2-(3-(1-méthylcyclohéxyl)-4-hydroxyphényl)éthényl]benzoïque

508 mg (1,39 mmole) de l'ester obtenu à l'exemple 36(c) dans 10 ml de méthanol, sont traités par 1,05 g d'hydroxyde de sodium. Le mélange réactionnel est chauffé à reflux pendant 45 min puis est traité comme indiqué à l'exemple 8. On isole, après recristallisation dans le mélange diisopropyléther/hexane 265 mg (57%) du dérivé attendu, fondant à 229 °C.

### EXEMPLE 37

### 4-[(E)-2-(3-(1-méthylcyclohéxyl)-4-(6-tert-butoxycarbonylpentyloxy)phényl)éthényl]benzoate d'éthyle

1,57 g (4,34 mmoles) du phénol obtenu à l'exemple 36(c), dans 30 ml de DMF, sont traités par 1,3 g (5,19 mmoles) de 6- bromohexanoate de tert-butyle et 0,717 g de carbonate de potassium. Le mélange réactionnel est agité à 60°C, pendant 50 h. Après le même traitement qu'à l'exemple 1(a), le résidu est chromatographié dans l'éluant éther/hexane (15:85), puis recristallisé dans l'hexane pour conduire à 1,17 g (51%) du dérivé attendu, fondant à 90-91°C.

### EXEMPLE 38

### 4-[(E)-2-(3-(1-méthylcyclohéxyl)-4-(6-carboxypentyloxy)phényl)éthényl]benzoate d'éthyle

0,73 g (1,37 mmole) du diester obtenu à l'exemple 37, en solution dans 30 ml de tétrachlorure de carbone, sont traités par 0,23 ml (1,64 mmole) d'iodure de triméthylsilyle. Le mélange réactionnel est laissé sous agitation et sous azote pendant 18 h à température ambiante. Après le même traitement qu'à l'exemple 1, suivi d'une chromatographie sur silice dans le gradient d'éluant allant du dichlorométhane au mélange dichlorométhane/éther (95:5), on obtient 0,47 g (72%) du produit attendu fondant à 112-114°C.

### EXEMPLE 39

### Acide 4-[(E)-2-(3-(1-adamantyl)-4-hydroxyphényl)éthényl]benzoïque

### a) 4-tert-butyldiméthylsilyloxy-3-(1-adamantyl)benzaldéhyde

A 33 g (78 mmoles) de 3-(1-adamantyl)-4-tert-butyldiméthylsilyloxy-bromo benzène dans 300 ml de THF, on ajoute 2,6 g de magnésium et 0,05 ml de dibromoéthane, puis chauffe à reflux pendant 3 h. 30 sous azote. On ajoute ensuite, après avoir refroidi le mélange à 0°C, 5,8 ml de DMF anhydre, et laisse agiter pendant 1 h à température ambiante. Après évaporation on reprend le résidu dans 100 ml d'eau, acidifie jusqu'à pH 5, extrait à l'éther, lave la phase organique à l'eau et sèche sur sulfate de magnésium. On isole 22,3 g (77%) du dérivé attendu, fondant à 121-122°C.

### b) Acide 4-[(E)-2-(3-(1-adamantyl)-4-tert-butyldiméthylsilyloxyphényl)éthényl]benzoate d'éthyle

A une suspension de 290 mg d'hydrure de sodium (80% dans l'huile), dans 50 ml de THF, on ajoute 3,7 g (10 mmoles) de l'aldéhyde obtenu à l'exemple 39(a), 3 g de 4-éthoxycarbonylbenzylphosphonate de diéthyle et 44 mg de 15- crown 5. La réaction est agitée sous azote à température ambiante pendant une nuit. Après le même traitement qu'à l'exemple 27(b) suivi d'une chromatographie sur silice dans l'éluant éther/hexane (20:80), on isole 2,4 g (46%) du dérivé attendu, fondant à 112-113°C.

### c) Acide 4-[(E)-2-(3-(1-adamantyl)-4-hydroxyphényl)éthényl]benzoïque

2,4 g (4,6 mmoles) de l'acide obtenu à l'exemple 39(b), dans 25 ml de méthanol, sont traités par 1,84g d'hydroxyde de sodium. Le mélange est chauffé à reflux pendant 6 h pour conduire, après le même traitement qu'à l'exemple 3, suivi d'une chromatographie sur silice dans l'éluant éther/hexane (60:40) à 1,26 g (73%) du dérivé attendu, fondant à 279-280°C.

### EXEMPLE 40

### 6-[3-(1-adamantyl)-4-(1,2-dihydroxyéthyl)phényl]-2-naphtoate de benzyle

### a) 6-[3-(1-adamantyl)-4-éthénylphényl]-2-naphtoate de benzyle

13,8 g (22 mmoles) du dérivé obtenu à l'exemple 18(a) dans 280 ml de DMF, sont traités par 9,4 ml (31 mmoles) d'alkyltributylétain, 2,83 g de chlorure de lithium et 515 mg (0,7 mmole) de PdCl2 (P(C6H5)3)2. Le mélange réactionnel est chauffé sous azote à 80°C pendant la nuit, puis on rajoute 154 mg de catalyseur et 3,9 ml de vinyltributylétain et chauffe à nouveau à 80°C durant 48 h. Après le même traitement qu'à l'exemple 20(a), suivi d'une chromatographie sur silice dans l'éluant dichlorométhane/hexane (30:70), on isole 4,54 g (41%) du dérivé attendu, fondant à 158-160°C.

### b) 6-[3-(1-adamantyl)-4-(1,2-dihydroxyéthyl)phényl]-2-naphtoate de benzyle

2,5 g (5 mmoles) du dérivé obtenu à l'exemple 40(a) dans 5 ml d'eau, 15 ml de tert-butanol et 0,4 ml de pyridine sont traités par 760 mg (6,8 mmoles) de N-oxyde de triméthylamine et 25 mg de tetroxyde d'osmium. Le mélange réactionnel est chauffé à reflux 6 h, puis est laissé sous agitation la nuit à température ambiante. On ajoute au milieu réactionnel 4 ml d'hydrogénosulfite de sodium (2M) et 20 ml d'eau, puis extrait par de l'acétate d'éthyle. La phase organique est rincée à l'eau, séchée sur sulfate de magnésium et évaporee. un isole après chromatographie sur silice, dans l'éluant dichlorométhane/éther (90:10) 1,92 g (72%) du dérivé attendu, fondant à 193°C.

### EXEMPLE 41

### Acide 6-[3-(1-adamantyl)-4-(1,2-dihydroxyéthyl)phényl]-2-naphtoïque

A 1,90 g (3,57 mmoles) de l'ester benzylique obtenu à l'exemple 40(b), dans 50 ml de dioxanne, on ajoute 0,6 g de palladium sur charbon (10%). Le mélange est agité sous une pression d'hydrogène de 7 bars à 50°C pendant 4 h. Après le même traitement qu'à l'exemple 5, suivi d'une recristallisation dans le mélange éthanol/eau, on isole 1,20 g (76%) du dérivé attendu, fondant à 239 °C.

### EXEMPLE 42

### 6-[3-(1-adamantyl)-4-(3-hydroxypropyl)phényl] -2-naphtoate de benzyle

5,13 g (10 mmoles) du dérivé obtenu à l'exemple 20(a), en solution dans 20 ml de THF, sont traités à 0°C par addition, goutte à goutte, de 15 mmoles de 9-borabicyclo[3.3.1]nonane (0,5 M/THF). Le mélange réactionnel est agité sous azote 1 h à 0°C et 1 h à température ambiante. On ajoute ensuite à 0°C 25 ml d'hydroxyde de sodium (1M) et 20 ml d'eau oxygénée (30%) et laisse agiter à 0°C pendant 1 h puis 2 h à température ambiante. Après évaporation du THF et extraction par du dichlorométhane (3x100 ml), la phase organique est lavée, séchée sur sulfate de magnésium, puis évaporée. Après chromatographie sur silice dans l'éluant dichlorométhane/hexane (80:20), on isole 4,67 g (88%) du dérivé attendu, fondant à 176°C.

### EXEMPLE 43

### Acide 6-[3-(1-adamantyl)-4-(3-hydroxypropyl)phényl]-2-naphtoïque

2,67 g (5 mmoles) de l'ester obtenu à l'exemple 42, en solution dans 50 ml de dioxanne sont agités sous une pression d 'hydrogène de 7 bars pendant 7 h à température ambiante, puis 1 h à 50°C, en présence de 800 mg de palladium sur charbon (10%). Après le même traitement qu'à l'exemple 5 suivi d'une recristallisation dans le mélange éthanol/eau, on isole 1,78 g (81%) du dérivé attendu, fondant à 262,4°C.

### EXEMPLE 44

### 6-[3-(1-adamantyl)-4-(3-acétoxypropyl)phényl]-2-naphtoate de benzyle

2 g (3,77 mmoles) du dérivé obtenu à l'exemple 42 en solution dans 40 ml de THF, sont traités par 0,8 ml de triéthylamine et 0,4 ml de chlorure d'acétyle. Le mélange réactionnel est laissé sous agitation une nuit à température ambiante puis est versé dans l'eau, extrait par de l'éther, lavé avec une solution aqueuse saturée de bicarbonate de sodium, puis séché sur sulfate de magnésium. Après chromatographie sur silice dans l'éluant dichlorométhane/hexane (60:40) on obtient 1,78 g (82%) du dérivé attendu, fondant à 132°C.

### EXEMPLE 45

### Acide 6-[3-(1-adamantyl)-4-(3-acétoxypropyl)phényl]-2-naphtoïque

1,77 g (3,1 mmoles) du diester obtenu à l'exemple 44, en solution dans 50 ml de dioxanne sont hydrogénés en présence de 530 mg de palladium sur charbon (10%) à 50°C, sous une pression d'hydrogène de 7 bars. On isole après le même traitement qu'à l'exemple 5, suivi d'une recristallisation dans l'acétate d'éthyle, 1,13 g (83%) du produit attendu, fondant à 260°C.

### EXEMPLE 46

### 6-[3-(1-adamantyl)-4-(2,3-dihydroxypropyl)phényl]-2-naphtoate de benzyle

5,13 g (10 mmoles) du dérivé obtenu à l'exemple 20(a) dans 9 ml d'eau, 30 ml de tert-butanol et 0,8 ml de pyridine sont traités par, 1,51 g (136 mmoles) de N-oxyde de triméthylamine et 20 mg de tétroxyde d'osmium. Le mélange réactionnel est chauffé à reflux pendant 24 h. Après le même traitement qu'à l'exemple 40(b), suivi d'une chromatographie sur silice, dans un gradient d'éluant allant du mélange dichlorométhane/éther (9:1) à (7:3), on isole 4 g (74%) du dérivé attendu, fondant à 199,5°C.

### EXEMPLE 47

### Acide 6-[3-(1-adamantyl)-4-(2,3-dihydroxypropyl)phényl]-2-naphtoïque

4 g (7,32 mmoles) de l'ester obtenu à l'exemple 46, en solution dans 100 ml de dioxanne, sont hydrogénés en présence de 1,20 g de palladium sur charbon (10%) sous une pression d'hydrogène de 7 bars, durant 5 h à 50°C. Après le même traitement qu'à l'exemple 5, suivi d'une recristallisation dans le mélange éthanol/eau, on isole 3 g (90%) du dérivé attendu, fondant à 258°C.

### EXEMPLE 48

### N-méthoxycarbonylméthyl-4-(6-benzyloxycarbonylnaphtyl)-2-(1-adamantyl)phényl carboxamide

3,14 g (5 mmoles) du triflate obtenu à l'exemple 18(a) dans 60 ml de DMF, sont traités par 2,80 ml (20 mmoles) de triéthylamine, 57 mg d'acétate de palladium, 280 mg de DPPF et 1,27 g (10 mmoles) d'ester méthylique de glycine sous sa forme chlorhydrate. Le milieu réactionnel est chauffé à 80°C sous une pression de monoxyde de carbone de 3 bars pendant 16 h. Après le même traitement qu'à l'exemple 20(a), suivi d'une chromatographie sur silice dans le dichlorométhane, on isole 1,25 g (43%) du dérivé attendu, fondant à 116-117°C.

### EXEMPLE 49

### N-méthoxycarbonylméthyl4-(6-carboxynaphtyl)-2-(1-adamantyl)phénylcarboxamide

1,25 g (2,1 mmoles) de l'ester benzylique obtenu à l'exemple 48, dans 50 ml de dioxanne, sont hydrogénés à 70°C en présence de 190 mg de palladium sur charbon (10%), sous une pression d'hydrogène de 7 bars, pendant 12 h. Après le même traitement qu'à l'exemple 5, suivi d'une recristallisation dans le mélange acétate d'éthyl/hexane, on isole 742 mg (71%) du dérivé attendu, fondant à 272 °C.

### EXEMPLE 50

### Acide 6-[3-(1-adamantyl)-4-(N,N-diméthylcarbamoyl)phényl]-2-naphtoïque

3,40 g (7,4 mmoles) du chlorure de l'acide obtenu à l'exemple 10, préparés comme indiqué à l'exemple 26(a) sont placés dans 10 ml d'hexaméthylphosphoramide, sont traités par 56 mg de (C6H5)CH2Pd (P(C6H5)3)2Cl et agités sous azote à 65°C durant 3 jours. Après le même traitement qu'à l'exemple 20(a), suivi d'une chromatographie sur silice, dans le gradient d'éluant dichlorométhane/hexane allant de (60:40) à (90:10), on isole 1 g (29%) du dérivé 6-[3-(1-adamantyl)-4-(N,N- diméthylcarbamoyl)phényl]-2-naphtoate de benzyle.

990 mg (2,11 mmoles) de cet ester dans 25 ml de méthanol sont traités par 2 g d'hydroxyde de sodium. Le mélange réactionnel est chauffé à reflux pendant 1 h puis est traité comme à l'exemple 3. On isole, après recristallisation dans l'éthanol absolu, 630 mg (66%) du produit attendu, fondant à 318-320°C.

### EXEMPLE 51

### 6-[3-(1-adamantyl)-4-(2(R),3-dihydroxypropyloxy)phényl]-2-naphtoate de benzyle

### a) 6-[3-(1-adamantyl)-4-(2,2-diméthyl-1,3-dioxolanne-4(R)-méthyloxy)phényl]-2-naphtoate de benzyle

9,76 g (20 mmoles) du phénol obtenu à l'exemple 4(a), dans 60 ml de DMF sont traités successivement par 604 mg (20 mmoles) d'hydrure de sodium (80% dans l'huile), puis par 6,86 g (24 mmoles) de (2R)-3-tosyloxy-1,2-propanediol acétonide. Le melange réactionnel est agité sous azote à température ambiante pendant 24 h. Après le même traitement qu'à l'exemple 1(a), suivi d'une chromatographie sur silice dans le gradient d'éluant hexane/acétone/dichlorométhane allant de (75:5:20) à (80:10:10), on isole 8,35 g (69%) du dérivé attendu, fondant à 170-172°C.

### b) 6-[3-(1-adamantyl)-4-(2(R),3-dihydroxypropyloxy)phényl]-2-naphtoate de benzyle

4,80 g (7,96 mmoles) de l'acétonide obtenut à l'exemple 51(a), sont placés en suspension dans 100 ml d'une solution aqueuse d'acide formique (40%). Le mélange réactionnel est chauffé à 100°C pendant 5 h, puis est agité un week-end à température ambiante. Après le même traitement qu'à l'exemple 18(b), suivi d'une chromatographie sur silice dans le gradient d'éluant dichlorométhane/acétate d'éthyle allant de (90:10) à (80:20) et d'une recristallisation dans le mélange éther/hexane, on isole 3,46 g (77%) du dérivé attendu, fondant à 204°C.

### EXEMPLE 52

### Acide 6-[3-(1-adamantyl)-4-(2(R),3-dihydroxypropyloxy)phényl]-2-naphtoïque

3,45 g (6 mmoles) de l'ester benzylique obtenu à l'exemple 51, dans 50 ml de méthanol sont traités par 4 g d'hydroxyde de sodium. Le milieu réactionnel est chauffé à reflux pendant 2 h, puis est traité comme indiqué à l'exemple 6. Après une recristallisation dans le mélange éthanol/eau, on isole 2,28 g (87%) du dérivé attendu, fondant à 271-272°C, aD= +1,5°(C=1,DMF).

### EXEMPLE 53

### Acide 6-[3-(1-adamantyl)-4,5-méthylènedioxyphényl]-2-naphtoïque

### a) 3-(1-adamantyl)-4,5-méthylènedioxybromobenzène

A 5 g (24,9 mmoles) de 3,4-méthylènedioxybromobenzène dans 35 ml de cyclo-hexane et 35 ml d'heptane, on ajoute 4,82 g (24,9 mmoles) d'acétoxyadamantane et 0,75 ml (139 mmoles) d'acide sulfurique concentré. Le milieu réactionnel est agité à température ambiante pendant une nuit. Ce milieu est alors hydrolysé avec 250 ml d'eau, neutralisé avec du bicarbonate de sodium, extrait par 200 ml de dichlorométhane, séché sur sulfate de magnésium et évaporé, pour conduire à 2,89 g (34,8%) du dérivé attendu fondant à 134°C.

### b) 6-[3-(1-adamantyl)-4,5-méthylènedioxyphényl]-2-naphtoate de méthyle

2,75 g (8,21 mmoles) du dérivé obtenu à l'exemple 53(a) dans 10 ml de THF, sont traités à -76°C par 3,61 ml de n- butyllithium (2,5 M/hexane), laisse revenir la température à 0°C puis ajoute 0,44 g (3,28 mmoles) de chlorure de zinc et laisse agiter sous azote pendant 15 min, puis ajoute 0,53 g (1,99 mmoles) de 6-bromo-2-naphtoate de méthyle. La réaction est laissée sous agitation 2 h à température ambiante, puis est traitée comme à l'exemple 2(b). On isole, après chromatographie sur silice, dans l'éluant hexane/acétate d'éthyle (85:5) 1,25 g (38%)du dérivé attendu, fondant à 217- 220°C.

### c) Acide 6-[3-(1-adamantyl)-4,5-méthylènedioxyphényl]-2-naphtoïque

150 mg (0,341 mmole) de l'ester obtenu à l'exemple 53(b) dans 3 ml de n-butanol, sont traités par 45 mg d'hydroxyde de potassium et agités à 80°C pendant 1 h. Après le même traitement qu'à l'exemple 3, le résidu est trituré dans l'hexane. Le précipité est alors séché pour conduire à 123,5 mg (85%) du dérivé attendu, fondant à 300-305°C.

### EXEMPLE 54

### N-éthyl 6-[3-(1-adamantyl)-4-méthoxycarbonylphényl]-2-naphtylcarboxamide

A 10 ml d'une solution aqueuse d'éthylamine à 70%, on ajoute 0,74 g (1,61 mmole) du chlorure d'acide préparé à l'exemple 26(a) et agite à température ambiante pendant une nuit. Après le même traitement qu'à l'exemple 26(b), suivi d'une chromatographie sur silice dans le dichlorométhane, on isole 440 mg (58%) du dérivé attendu, fondant à 201,9°C.

### EXEMPLE 55

### N,N-morpholyl 6-[3-(1-adamantyl)-4-méthoxycarbonylphényl]-2-naphtylcarboxamide

A une solution de 0,28 ml (3,22 mmoles) de morpholine, dans 10 ml de THF contenant 0,22 ml de triéthylamine, on ajoute 0,74 g (1,61 mmole) du chlorure d'acide 26(a) et agite à température ambiante pendant une nuit. Après le même traitement qu'à l'exemple 26(b), suivi d'une chromatographie sur silice dans le mélange dichlorométhane/éther (90:10), on isole 377 mg (46%) du dérivé attendu, fondant à 152°C.

### EXEMPLE 56

### 4-[(E)-2-(3-tert-butyl-4-méthoxyphényl)propényl]phénylcarbinol

1,41 g (4,35 mmoles) de l'acide obtenu à l'exemple 29 sont traités par 330 mg d'hydrure de lithium aluminium. Le mélange réactionnel est chauffé à reflux pendant 2 h. 30, puis est traité par une solution saturée de chlorure d'ammonium, puis est extrait par 300 ml d'éther. Après séchage et évaporation de la phase organique, le résidu est recristallisé dans l'hexane pour conduire à 1,21 g (90%) du dérivé attendu, fondant à 88-90°C.

### EXEMPLE 57

### 4-[(E)-2-(3-tert-butyl-4-méthoxyphényl)propényl]benzylacétate

800 mg (2,58 mmoles) de l'alcool obtenu à l'exemple 56, en solution dans 10 ml de pyridine, sont traités par 0,28 ml de chlorure d'acétyle et sont laissés à réagir 1 h à température ambiante. Après le même traitement qu'à l'exemple 44, suivi d'une chromatographie sur silice dans le mélange dichlorométhane/hexane (50:50), on isole 880 mg (97%) du dérivé attendu sous forme d'huile incolore.

RMN (1H) : dppm (CDCl3): 1,41(9H,s);2,11(3H,s);2,26(3H,s);3,86(3H,s);5,11(2H,s);6,75(1H, s);6,87(1H,d);7,35(5H,m);7,46(1H,d).

### EXEMPLES DE FORMULATIONS

### A. VOIE ORALE

### (a) Comprimé de 0,2 g

| | |
|---|---|
| Composé de l'exemple 8 | 0,001 g |
| Amidon | 0,114 g |
| Phosphate bicalcique | 0,020 g |
| Silice | 0,020 g |
| Lactose | 0,030 g |
| Talc | 0,010 g |
| Stéarate de magnésium | 0,005 g |

Dans cet exemple, le composé de l'exemple 8 peut être remplacé par la même quantité du composé de l'exemple 13.

### (b) Suspension buvable en ampoules de 5 ml

| | |
|---|---|
| Composé de l'exemple 3 | 0,500 g |
| Glycérine | 0,500 g |
| Sorbitol à 70 % | 0,500 g |
| Saccharinate de sodium | 0,010 g |
| Parahydroxybenzoate de méthyle | 0,040 g |
| Arôme qs | |
| Eau purifiée qsp | 5 ml |

Dans cet exemple, le composé de l'exemple 3 peut être remplacé par la même quantité du composé de l'exemple 19.

### (c) Comprimé de 0,8 g

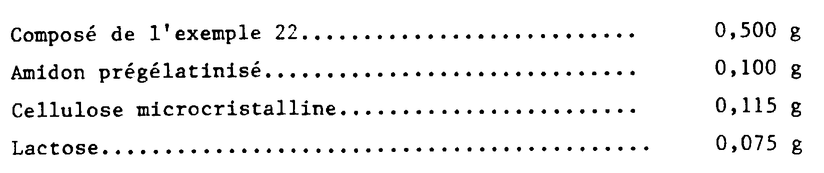

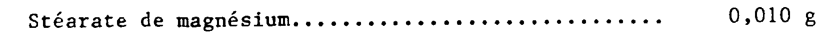

Dans cet exemple, le composé de l'exemple 22 peut être remplacé par la même quantité du composé de l'exemple 15.

### (d) Suspension buvable en ampoules de 10 ml

| | |
|---|---|
| Composé de l'exemple 5 | 0,200 g |
| Glycérine | 1,000 g |
| Sorbitol à 70 % | 1,000 g |
| Saccharinate de sodium | 0,010 g |
| Parahydroxybenzoate de méthyle | 0,080 g |
| Arôme qsp | |
| Eau purifiée qsp | 10 ml |

### B. VOIE TOPIQUE

### (a) Onguent

| | |
|---|---|
| Composé de l'exemple 8 | 0,020 g |
| Myristate d'isopropyle | 81,700 g |
| Huile de vaseline fluide | 9,100 g |
| Silice vendue par la Société DEGUSSA sous la dénomination "Aérosil 200" | 9,180 g |

Dans cet exemple, le composé de l'exemple 8 peut être remplacé par la même quantité du composé de l'exemple 13.

### (b) Onguent

| | |
|---|---|
| Composé de l'exemple 3 | 0,300 g |
| Vaseline blanche codex qsp | 100 g |

Dans cet exemple, le composé de l'exemple 3 peut être remplacé par la même quantité du composé de l'exemple 19.

### (c) Crème Eau-dans-l'Huile non ionique

| | |
|---|---|
| Composé de l'exemple 22 | 0,100 g |
| Mélange d'alcools de lanoline émulsifs, de cires et d'huiles raffinés, vendu par la Société BDF sous la dénomination "Eucerine anhydre" | 39,900 g |
| Parahydroxybenzoate de méthyle | 0,075 g |
| Parahydroxybenzoate de propyle | 0,075 g |
| Eau déminéralisée stérile qsp | 100 g |

Dans cet exemple, le composé de l'exemple 22 peut être remplacé par la même quantité du composé de l'exemple 15.

### (d) Lotion

| | |
|---|---|
| Composé de l'exemple 5 | 0,100 g |
| Polyéthylène glycol (PEG 400) | 69,900 g |
| Ethanol 95 % | 30,000 g |

### (e) Onguent hydrophobe

| | |
|---|---|
| Composé de l'exemple 6 | 0,300 g |
| Myristate d'isopropyle | 36,400 g |
| Huile de silicone vendue par la Société Rhône Poulenc sous la dénomination "Rhodorsil 47 V 300" | 36,400 g |
| Cire d'abeille | 13,600 g |
| Huile de silicone vendue par la Société Goldschmidt sous la dénomination "Abil 300.000 cst" qsp | 100 g |

### (f) Crème Huile-dans-l'Eau non ionique

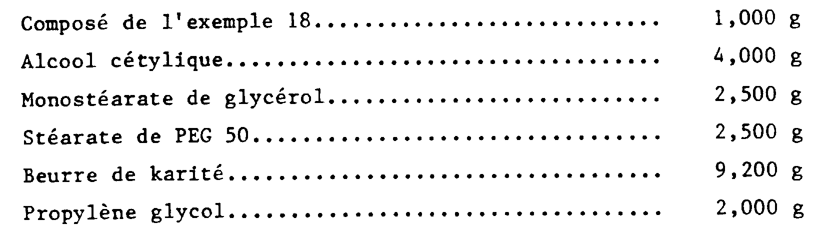

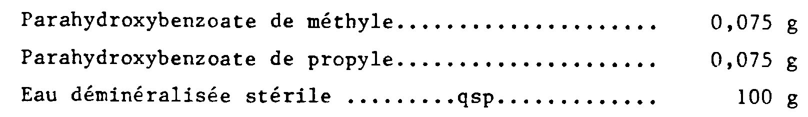

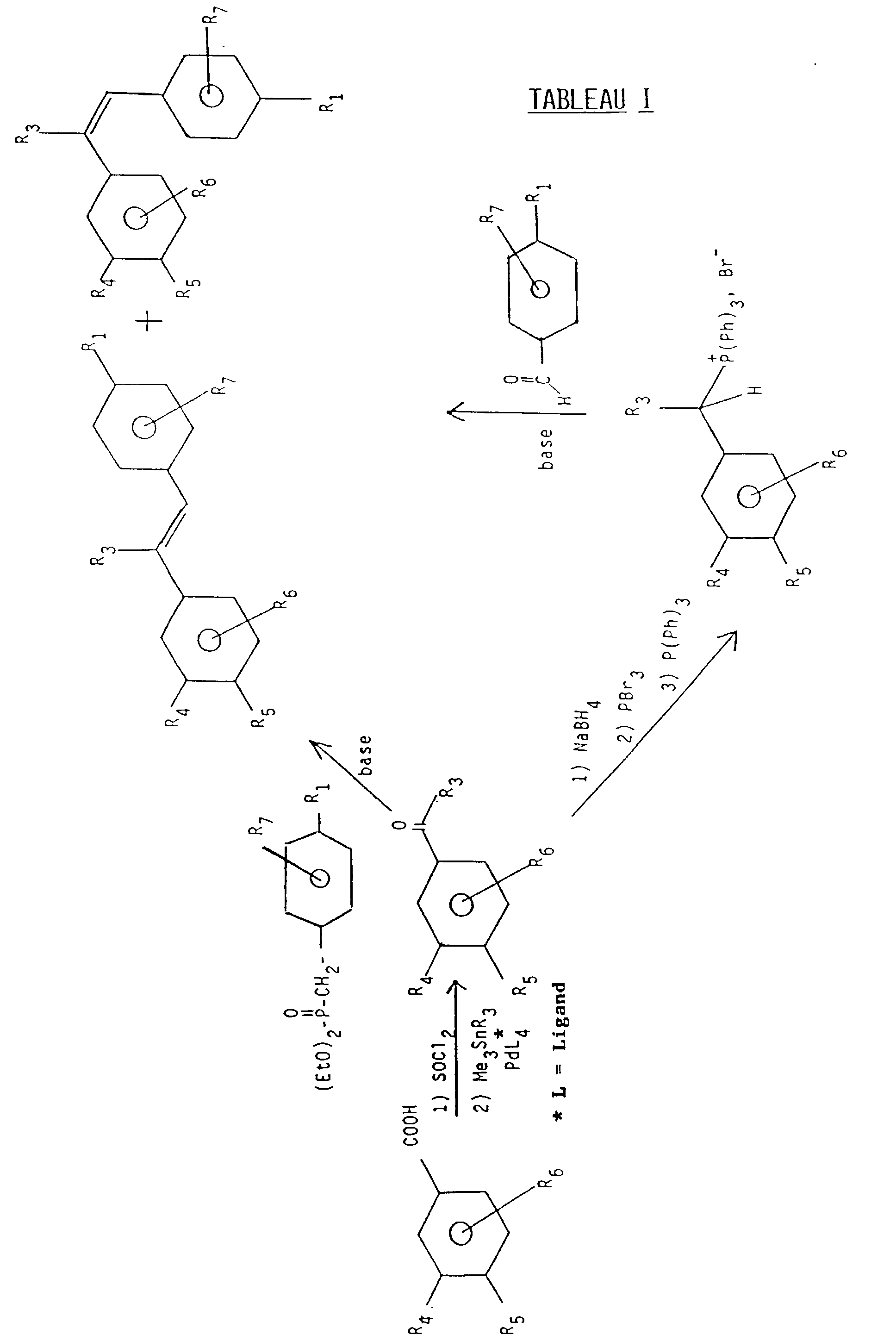

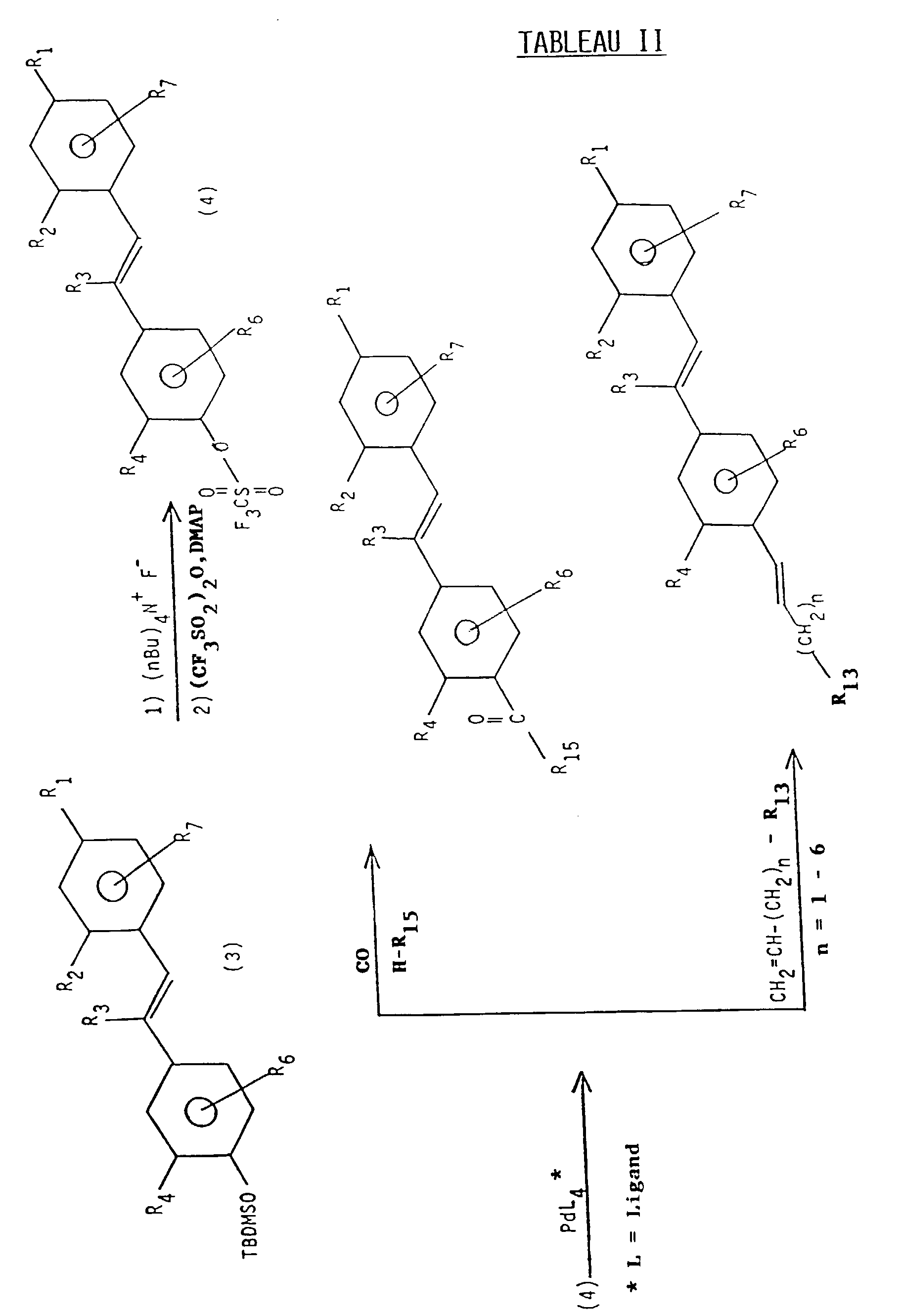

## Revendications (Revendications pour l'(les) Etat(s) contractant(s) suivant(s): AT, BE, CH, DE, DK, FR, GB, IT, LI, NL, SE)

1. Composés bicycliques aromatiques, caractérisés par le fait qu'ils répondent à la formule générale suivante : 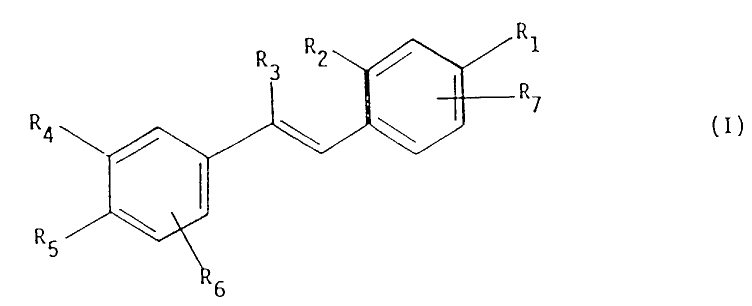 dans laquelle :
R₁ représente
(i) un atome d'hydrogène,
(ii) le radical -CH₃,
(iii) le radical -CH₂-O-R₈,
R₈ représentant un atome d'hydrogène ou un radical alkyle inférieur,
(iv) un radical OR₈,
(v) un radical 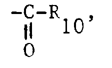 R₁₀ représentant :
(a) un atome d'hydrogène,
(b) un radical 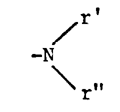
r' et r" représentant un atome
d'hydrogène, un radical alkyle inférieur, un radical mono ou polyhydroxyalkyle, un radical aryle éventuellement substitué ou un reste d'amino acide ou de sucre
ou encore pris ensemble forment un hétérocycle,
(c) un radical -OR₁₁
R₁₁ représentant un atome d'hydrogène, un radical alkyle linéaire ou ramifié ayant de 1 à 20 atomes de carbone, un radical mono ou polyhydroxyalkyle, un radical aryle ou aralkyle éventuellement substitué(s) ou un reste de sucre ou un reste d'aminoacide, et
(vi) un radical -S(O)ₜR₈,
t étant 0, 1 ou 2 et R₈ étant tel que défini ci-dessus,
R₂ représente un atome d'hydrogène,
R₃ représente un atome d'hydrogène, un radical aryle, un radical aralkyle ou un radical alkyle inférieur éventuellement substitué par un hydroxyle, par un alcoxy inférieur ou par un radical 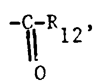
R₁₂ représentant un atome d'hydrogène, un radical alkyle inférieur, un radical hydroxyle, un radical alcoxy inférieur ou un radical 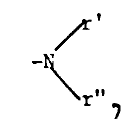
ou R₂ et R₃ pris ensemble forment avec le noyau benzénique un cycle naphtalénique,
R₄ représente un radical alkyle linéaire ou ramifié ou non ayant de 1 à 15 atomes de carbone ou un radical cycloaliphatique,
R₅ représente le radical -(CH₂)ₙ-R₁₃, le radical -CH=CH-(CH₂)ₙ-R₁₃, ou le radical -O(CH₂)ₘR₁₄
n étant 0 ou 1 à 6,
m étant 1 à 6
R₁₃ représentant le radical 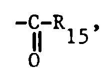
un radical monohydroxyalkyle, un radical polyhydroxyalkyle dont les hydroxyles sont éventuellement protégés sous forme de méthoxy ou d'acétoxy, un radical alkyle inférieur epoxydé ou le radical 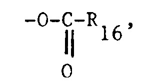
R₁₅ représentant le radical OR₁₆ ou le radical 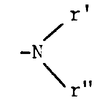
R₁₆ représentant un atome d'hydrogène, un radical alkyle inférieur, un radical aryle, ou un radical aralkyle,
R₁₄ représentant un radical hydroxyle lorsque m > 2, un radical monohydroxyalkyle, un radical polyhydroxyalkyle, le radical 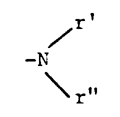
le radical 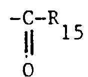 ou un radical alkényle mono ou polyhydroxylé ayant de 2 à 10 atomes de carbone, ou lorsque R₂ et R₃ ne sont pas pris ensemble m peut être 0 et/ou R₁₄ peut représenter un atome d'hydrogène ou un radical alkyle inférieur.
R₆ et R₇ représentent un atome d'hydrogène, un atome d'halogène, un radical alkyle inférieur ou le radical -OR₁₆,
R₅ et R₆ peuvent en outre former un cycle méthylène dioxy lorsque R₆ est en position 3 du noyau benzénique,
et les sels des composés de formule (I) lorsque R₁ représente une fonction acide carboxylique et les analogues chiraux desdits composés de formule (I) ainsi que les isomères géométriques desdits composés lorsque R₂ et R₃ ne sont pas pris ensemble.

2. Composés selon la revendication 1, caractérisés par le fait qu'ils se présentent sous forme de sels d'un métal alcalin ou alcalino-terreux ou encore de zinc ou d'une amine organique.

3. Composés selon la revendication 1, caractérisés par le fait qu'ils se présentent sous forme de sels d'un acide minéral ou organique choisi dans le groupe constitué par l'acide chlorhydrique, l'acide sulfurique, l'acide acétique, l'acide citrique, l'acide fumarique, l'acide hemisuccinique, l'acide maléique et l'acide mandélique.

4. Composés selon la revendication 1, caractérisés par le fait que le radical alkyle inférieur a de 1 à 6 atomes de carbone et est pris de préférence dans le groupe constitué par les radicaux méthyle, éthyle, isopropyle, butyle et tertiobutyle.

5. Composés selon la revendication 1, caractérisés par le fait que le radical alcoxy inférieur est pris dans le groupe constitué par un radical méthoxy, éthoxy, isopropoxy et butoxy.

6. Composés selon la revendication 1, caractérisés par le fait que le radical cycloaliphatique est choisi parmi le radical 1-méthyl cyclohexyle et le radical 1-adamantyle.

7. Composés selon la revendication 1, caractérisés par le fait que le radical monohydroxyalkyle est le radical hydroxyméthyle, 2-hydroxyéthyle, 2-hydroxypropyle, 3-hydroxypropyle, 4-hydroxybutyle, 5-hydroxypentyle ou 6-hydroxyhexyle.

8. Composés selon la revendication 1, caractérisés par le fait que le radical polyhydroxyalkyle comporte de 3 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxypentyle et le reste du pentaérythritol.

9. Composés selon la revendication 1, caractérisés par le fait que le radical aryle est un radical phényle éventuellement substitué par au moins un atome d'halogène, un hydroxyle ou une fonction nitro.

10. Composés selon la revendication 1, caractérisés par le fait que le radical aralkyle est le radical benzyle ou phénéthyle éventuellement substitué par au moins un atome d'halogène, un hydroxyle ou une fonction nitro.

11. Composés selon la revendication 1, caractérisés par le fait que l'hétérocycle est pris dans le groupe constitué par un radical pipéridino, morpholino, pyrrolidino ou pipérazino, éventuellement substitué en position 4 par un radical alkyle en C₁-C₆ ou mono ou polyhydroxyalkyle.

12. Composés selon l'une quelconque des revendications précédentes, caractérisés par le fait qu'ils sont pris dans le groupe constitué par :
Chlorhydrate de l'acide 6-[3-(1-adamantyl)-4-(3-aminopropyloxy) phényl]-2-naphtoïque;
6-[3-(1-adamantyl)-4-(2,3-dihydroxypropyloxy)phény]-2-naphtoate de méthyle ;
Acide 6-[3-(1-adamantyl)-4-(2,3-dihydroxypropyloxy)phényl] -2-naphtoïque ;
6-[3-(1-adamantyl)-4-méthoxycarbonylméthyloxyphényl] -2-naphtoate de benzyle ;
Acide 6-[3-(1-adamantyl)-4-méthoxycarbonylméthyloxyphényl] -2-naphtoïque ;
Acide 6-[3-(1-adamantyl)-4-carboxyméthyloxyphényl] -2-naphtoïque ;
6-[3-(1-adamantyl)-4-(3-hydroxypropyloxy)phényl]-2-naphtoate de méthyle ;
Acide 6-[3-(1-adamantyl)-4-(3-hydroxypropyloxy)phényl] -2-naphtoïque ;
6-[3-(1-adamantyl)-4-benzyloxycarbonylphényl]-2-naphtoate de méthyle ;
6-[3-(1-adamantyl)-4-carboxyphényl]-2-naphtoate de méthyle ;
6-[3-(1-adamantyl)-4-hydroxymethylphényl]-2-naphtoate de méthyle ;
Acide 6-[3-(1-adamantyl)-4-hydroxyméthylphényl]-2-naphtoïque;
Acide 6-[3-(1-adamantyl)-4-acétoxyméthylphényl]-2-naphtoïque;
6-[3-(1-adamantyl)-4-methoxycarbonylphényl]-2-naphtoate de méthyle ;
Acide 6-[3-(1-adamantyl)-4-méthoxycarbonylphényl]-2-naphtoïque ;
Acide 6-[3-(1-adamantyl)-4-carboxyphényl]-2-naphtoïque ;
Acide 6-[3-(1-adamantyl)-4-carboxamidophényl]-2-naphtoïque ;
6- [3-(1-adamantyl)-4-méthoxycarbonyléthénylphényl]-2-naphtoate de benzyle ;
Acide 6-[3-(1-adamantyl)-4-(méthoxycarbonyléthyl)phényl] -2-naphtoïque ;
6-[3(1-adamantyl)-4-(2,3-époxypropyl)phényl]-2-naphtoate de benzyle ;
Acide 6-[3-(1-adamantyl)-4-(2-hydroxypropyl)phényl] -2-naphtoïque ;
Acide 6-[3-(1-adamantyl)-4-(3-méthoxy-2-hydroxypropyl)phényl] -2-naphtoïque.
Acide 4[(E)-2-(3-(1-adamantyl)-4-méthoxy phényl)propenyl] benzoïque ;
4-[(E)-2-(3-(1-adamantyl)-4-méthoxyphényl)propényl]benzoate de benzyle ;
Acide 4-[(E)-2-(3-(1-adamantyl)-4-hydroxyphényl)-1-propényl]benzoïque;
6-[3-(1-adamantyl)4-méthoxycarbonylphényl]-2-naphtylcarboxamide ;
4-[(E)-2-(3-(1-méthylcyclohexyl)-4- hydroxyphényl)propényl]benzoate d'éthyle ;
4-[(E)-2-(3-(1-méthylcyclohexyl)-4- méthoxyphényl)propényl]benzoate d'éthyle ;
Acide 4-[(E)-2-(3-(1-méthylcyclohexyl)-4- méthoxyphényl)propényl]benzoïque;
4-[(Z)-2-(3-(1-méthylcyclohexyl)-4- hydroxyphényl)propényl]benzoate d'éthyle ;
4-[(Z)-2-(3-(1-méthylcyclohexyl)-4- méthoxyphényl)propényl]benzoate d'éthyle ;
Acide 4-[(Z)-2-(3-(1-méthylcyclohexyl)-4- méthoxyphényl)propényl]benzoïque;
4-[(Z)-2-(3-tert-butyl-4-méthoxyphényl)propényl]benzoate d'éthyle ;
Acide 4-[(Z)-2-(3-tert-butyl-4-méthoxyphényl)propényl] benzoïque ;
Acide 4-[(E)-2-(3-tert-butyl-4-hydroxyphényl)propényl]benzoïque;
Acide 4-[(E)-2-(3-(1-méthylcyclohexyl)-4- hydroxyphényl)éthényl]benzoïque;
4-[(E)-2-(3-(1-méthylcyclohexyl)-4-(6-tert- butoxycarbonylpentyloxy)phényl) éthényl]benzoate d'éthyle ;
4-[(E)-2-(3-(1-méthylcyclohexyl)4-(6-carboxypentyloxy)phényl)éthényl]benzoate d'éthyle ;
Acide 4-[(E)-2-(3-(1-adamantyl)-4-hydroxyphényl)éthényl] benzoïque ;
6-[3-(1-adamantyl)-4-(1,2-dihydroxyéthyl)phényl]-2-naphtoate de benzyle ; Acide 6-[3-(1-adamantyl)-4-(1,2-dihydroxyéthyl)phényl]-2- naphtoïque ;
6-[3-(1-adamantyl)-4-(3-hydroxypropyl)phényl]-2-naphtoate de benzyl ;
Acide 6-[3-(1-adamantyl)-4-(3-hydroxypropyl)phényl]-2- naphtoïque ;
6-[3-(1-adamantyl)-4-(3-acétoxypropyl)phényl]-2-naphtoate de benzyle ;
Acide 6-[3-(1-adamantyl)-4-(3-acétoxypropyl)phényl]-2- naphtoïque ;
6-[3-(1-adamantyl)-4-(2,3-dihydroxypropyl)phényl]-2-naphtoate de benzyle;
Acide 6-[3-(1-adamantyl)-4-(2,3-dihydroxypropyl)phényl]-2-naphtoïque ;
N-méthoxycarbonylméthyl-4-(6-benzyloxycarbonylnaphtyl)-2-(1-adamantyl)phényl carboxamide ;
N-méthoxycarbonylméthyl4-(6-Carboxynaphtyl)-2-(1-adamantyl)phénylcarboxamide ;
Acide 6-[3-(1-adamantyl)-4-(N,N-diméthylcarbamoyl)phényl]-2-naphtoïque ;
6-[3-(1-adamantyl)-4-(2(R),3-dihydroxypropyloxy)phényl]-2- naphtoate de benzyle ;
Acide 6-[3-(1-adamantyl)-4-(2(R),3-dihydroxypropyloxy)phényl]-2-naphtoïque;
Acide 6-[3-(1-adamantyl)-4,5-méthylène dioxyphényl]-2-naphtoïque;
N-éthyl 6-[3-(1-adamantyl)-4-méthoxycarbonylphényl]-2-naphtylcarboxamide ;
N,N-morpholyl 6-[3-(1-adamantyl)-4-méthoxycarbonylphényl]-2-naphtylcarboxamide;
4-[(E)-2-(3-tert-butyl-4-méthoxyphényl)propényl]phénylcarbinol; et
4-[(E)-2-(3-tert-butyl-4-méthoxyphényl)propényl]benzylacétate.

13. Composition pharmaceutique, caractérisée par le fait qu'elle contient dans un véhicule approprié, pour une administration par voie entérale, parentérale, topique ou oculaire, au moins un composé de formule (I) selon l'une quelconque des revendications 1 à 12.

14. Composition selon la revendication 13, caractérisée par le fait qu'elle contient de 0,001 à environ 5% en poids d'un composé de formule (I).

15. Utilisation d'un composé selon l'une quelconque des revendications 1 à 12 pour la préparation d'une composition pharmaceutique destinée au traitement des affections dermatologiques, rhumatismales, respiratoires ainsi qu'ophtalmologiques.

16. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 12.

17. Composition cosmétique selon la revendication 16, caractérisée par le fait qu'elle contient le composé de formule (I) à une concentration comprise entre 0,001 et 3%.

## Revendications (Revendications pour l'(les) Etat(s) contractant(s) suivant(s): ES)

1. Composition cosmétique pour l'hygiène corporelle et capillaire, caractérisée par le fait qu'elle contient, dans un véhicule cosmétique approprié, au moins un composé de formule (I) suivante : 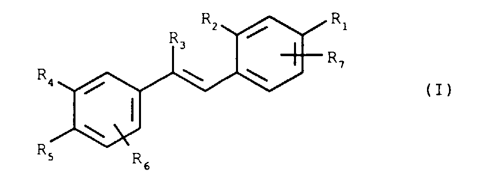 dans laquelle :
R₁ représente
(i) un atome d'hydrogène,
(ii) le radical -CH₃,
(iii) le radical -CH₂-O-R₈,
R₈ représentant un atome d'hydrogène ou un radical alkyle inférieur,
(iv) un radical -OR₈,
(v) un radical 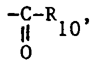 R₁₀ représentant :
(a) un atome d'hydrogène,
(b) un radical 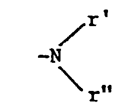
r' et r" représentant un atome
d'hydrogène, un radical alkyle inférieur, un radical mono ou polyhydroxyalkyle, un radical aryle éventuellement substitué ou un reste d'amino acide ou de sucre
ou encore pris ensemble forment un hétérocycle,
(c) un radical -OR₁₁
R₁₁ représentant un atome d'hydrogène, un radical alkyle linéaire ou ramifié ayant de 1 à 20 atomes de carbone, un radical mono ou polyhydroxyalkyle, un radical aryle ou aralkyle éventuellement substitué(s) ou un reste de sucre ou un reste d'aminoacide, et
(vi) un radical S(O)ₜR₈,
t étant 0, 1 ou 2 et R₈ étant tel que défini ci-dessus,
R₂ représente un atome d'hydrogène,
R₃ représente un atome d'hydrogène, un radical aryle, un radical aralkyle ou un radical alkyle inférieur éventuellement substitué par un hydroxyle, par un alcoxy inférieur ou par un radical 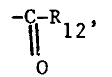
R₁₂ représentant un atome d'hydrogène, un radical alkyle inférieur, un radical hydroxyle, un radical alcoxy inférieur ou un radical 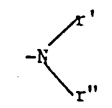
ou R₂ et R₃ pris ensemble forment avec le noyau benzénique un cycle naphtalénique,
R₄ représente un radical alkyle linéaire ou ramifié ou non ayant de 1 à 15 atomes de carbone ou un radical cycloaliphatique,
R₅ représente le radical -(CH₂)ₙ-R₁₃, le radical -CH=CH-(CH₂)ₙ-R₁₃, ou le radical -O(CH₂)ₘR₁₄
n étant 0 ou 1 à 6,
m étant 1 à 6
R₁₃ représentant le radical 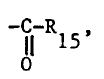
un radical monohydroxyalkyle, un radical polyhydroxyalkyle dont les hydroxyles sont éventuellement protégés sous forme de méthoxy ou d'acétoxy, un radical alkyle inférieur epoxydé ou le radical 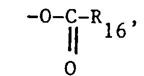
R₁₅ représentant le radical OR₁₆ ou le radical 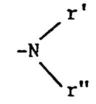
R₁₆ représentant un atome d'hydrogène, un radical alkyle inférieur, un radical aryle, ou un radical aralkyle,
R₁₄ représentant un radical hydroxyle lorsque m > 2, un radical monohydroxyalkyle, un radical polyhydroxyalkyle, le radical 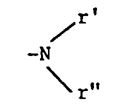
le radical 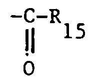 ou un radical alkényle mono ou polyhydroxylé ayant de 2 à 10 atomes de carbone, ou lorsque R₂ et R₃ ne sont pas pris ensemble m peut être O et/ou R₁₄ peut représenter un atome d'hydrogène ou un radical alkyle inférieur.
R₆ et R₇ représentent un atome d'hydrogène, un atome d'halogène, un radical alkyle inférieur ou le radical -OR₁₆,
R₅ et R₆ peuvent en outre former un cycle méthylène dioxy lorsque R₆ est en position 3 du noyau benzénique,
et les sels des composés de formule (I) lorsque R₁ représente une fonction acide carboxylique et les analogues chiraux desdits composés de formule (I) ainsi-que les isomères géométriques desdits composés lorsque R₂ et R₃ ne sont pas pris ensemble.

2. Composition selon la revendication 1, caractérisée par le fait que le composé de formule (I) se présente sous la forme d'un sel d'un métal alcalin ou alcalino-terreux ou encore de zinc ou d'une amine organique.

3. Composition selon la revendication 1, caractérisée par le fait que le composé de formule (I) se présente sous la forme d'un sel d'un acide minéral ou organique choisi dans le groupe constitué par l'acide chlorhydrique, l'acide sulfurique, l'acide acétique, l'acide citrique, l'acide fumarique, l'acide hemisuccinique, l'acide maléique et l'acide mandélique.

4. Composition selon l'une quelconque des revendications précédentes, caractérisée par le fait que le composé de formule (I) est pris dans le groupe constitué par :
Chlorhydrate de l'acide 6-[3-(1-adamantyl)-4-(3-aminopropyloxy) phényl]-2-naphtoïque;
6-[3-(1-adamantyl)-4-(2,3-dihydroxypropyloxy)phényl]-2-naphtoate de méthyle ;
Acide 6-[3-(1-adamantyl)-4-(2,3-dihydroxypropyloxy)phényl] -2-naphtoïque ;
6-[3-(1-adamantyl)-6-méthoxycarbonylméthyloxyphényl] -2-naphtoate de benzyle ;
Acide 6-[3-(1-adamantyl)-4-méthoxycarbonylméthyloxyphényl] -2-naphtoïque ;
Acide 6-[3-(1-adamantyl)-4-carboxyméthyloxyphényl] -2-naphtoïque ;
6- [3-(1-adamantyl)-4-(3-hydroxypropyloxy)phényl]-2-naphtoate de méthyle ;
Acide 6-[3-(1-adamantyl)-4-(3-hydroxypropyloxy)phényl] -2-naphtoïque ;
6-[3-(1-adamantyl)-4-benzyloxycarbonylphényl]-2-naphtoate de méthyle ;
6-[3-(1-adamantyl)-4-carboxyphényl]-2-naphtoate de méthyle ;
6-[3-(1-adamantyl)-4-hydroxymethylphényl]-2-naphtoate de méthyle ;
Acide 6-[3-(1-adamantyl)-4-hydroxyméthylphényl]-2-naphtoïque ;
Acide 6-[3-(1-adamantyl)-4-acétoxyméthylphényl]-2-naphtoïque ;
6-[3-(1-adamantyl)-4-methoxycarbonylphényl]-2-naphtoate de méthyle ;
Acide 6-[3-(1-adamantyl)-4-méthoxycarbonylphényl]-2-naphtoïque;
Acide 6-[3-(1-adamantyl)-4-carboxyphényl]-2-naphtoïque ;
Acide 6-[3-(1-adamantyl)-4-carboxamidophényl]-2-naphtoïque;
6-[3-(1-adamantyl)-4-méthoxycarbonyléthénylphényl]-2-naphtoate de benzyle ;
Acide 6-[3-(1-adamantyl)-4-(méthoxycarbonyléthyl)phényl] -2-naphtoïque ;
6-[3-(1-adamantyl)-4-(2,3-époxypropyl)phényl]-2-naphtoate de benzyle ;
Acide 6-[3-(1-adamantyl)-4-(2-hydroxypropyl)phényl] -2-naphtoïque ;
Acide 6-[3-(1-adamantyl)-4-(3-méthoxy-2-hydroxypropyl)phényl] -2-naphtoïque.
Acide 4[(E)-2-(3-(1-adamantyl)-4-méthoxy phényl)propenyl] benzoïque ;
4-[(E)-2-(3-(1-adamantyl)-4-méthoxyphényl)propényl]benzoate de benzyle;
Acide 4-[(E)-2-(3-(1-adamantyl)-4-hydroxyphényl)-1-propényl]benzoïque ;
6-[3-(1-adamantyl)-4-méthoxycarbonylphényl]-2- naphtylcarboxamide ;
4-[(E)-2-(3-(1-méthylcyclohexyl)-4- hydroxyphényl)propényl]benzoate d'éthyle ;
4- [(E)-2-(3-(1-méthylcyclohexyl)-4-méthoxyphényl)propényl]benzoate d'éthyle;
Acide 4- [(E)-2-(3-(1-méthylcyclohexyl)-4-méthoxyphényl)propényl]benzoïque;
4-[(Z)2-(3-(1-méthylcyclohexyl)-4-hydroxyphényl)propényl]benzoate d'éthyle ;
4-[(Z)-2-(3-(1-méthylcyclohexyl)-4-méthoxyphényl)propényl]benzoate d'éthyle ;
Acide 4-[(Z)-2-(3-(1-méthylcyclohexyl)-4- méthoxyphényl)propényl]benzoïque;
4-[(Z)-2-(3-tert-butyl-4-méthoxyphényl)propényl]benzoate d'éthyle ;
Acide 4- [(Z)-2-(3-tert-butyl-4-méthoxyphényl)propényl]benzoïque ;
Acide 4-[(E)-2-(3-tert-butyl-4-hydroxyphényl)propényl]benzoïque;
Acide 4-[(E)-2-(3-(1-méthylcyclohexyl)-4-hydroxyphényl)éthényl]benzoïque ;
4-[(E)-2-(3-(1-méthylcyclohexyl)-4-(6-tert-butoxycarbonylpentyloxy)phényl) éthényl]benzoate d'éthyle ;
4-[(E)-2-(3-(1-méthylcyclohexyl)-4-(6-carboxypentyloxy)phényl)éthényl]benzoate d'éthyle ;
Acide 4-[(E)2-(3-(1-adamantyl)-4-hydroxyphényl)éthényl]benzoïque ;
6-[3-(1-adamantyl)-4-(1,2-dihydroxyéthyl)phényl]-2-naphtoate de benzyle ;
Acide 6-[3-(1-adamantyl)-4-(1,2-dihydroxyéthyl)phényl]-2-naphtoïque ;
6-[3-(1-adamantyl)-4-(3-hydroxypropyl)phényl]-2-naphtoate de benzyl ;
Acide 6-[3-(1-adamantyl)-4-(3-hydroxypropyl)phenyl]-2-naphtoïque ;
6-[3-(1-adamantyl)-4-(3-acétoxypropyl)phényl]-2-naphtoate de benzyle ;
Acide 6-[3-(1-adamantyl)-4-(3-acétoxypropyl)phényl]-2-naphtoïque ;
6-[3-(1-adamantyl)-4-(2,3-dihydroxypropyl)phényl]-2-naphtoate de benzyle;
Acide 6-[3-(1-admnantyl)-4-(2,3-dihydroxypropyl)phényl]-2-naphtoïque ;
N-méthoxycarbonylméthyl-4-(6-benzyloxycarbonylnaphtyl)-2-(1-adamantyl)phényl carboxamide ;
N-méthoxycarbonylméthyl4-(6-Carboxynaphtyl)-2-(1-adamantyl)phénylcarboxamide ;
Acide 6-[3-(1-adamantyl)-4-(N,N-diméthylcarbamoyl)phényl]-2-naphtoïque ;
6-[3-(1-adamantyl)-4-(2(R),3-dihydroxypropyloxy)phényl]-2-naphtoate de benzyle;
Acide 6-[3-(1-adamantyl)-4-(2(R),3-dihydroxypropyloxy)phényl]-2-naphtoïque;
Acide 6-[3-(1-adamantyl)-4,5-méthylène dioxyphényl]-2-naphtoïque;
N-éthyl 6-[3-(1-adamantyl)-4-méthoxycarbonylphényl]-2-naphtylcarboxamide ;
N,N-morpholyl 6-[3-(1-adamantyl)4-méthoxycarbonylphényl]-2-naphtylcarboxamide ;
4-[(E)-2-(3-tert-butyl-4-méthoxyphényl)propényl]phénylcarbinol; et
4-[(E)2-(3-tert-butyl-4-méthoxyphénylyl]benzylacétate.

5. Composition cosmétique selon l'une quelconque des revendications précédentes, caractérisée par le fait qu'elle contient le composé de formule (I) à une concentration comprise entre 0,0001 et 3 %.

## Revendications (Revendications pour l'(les) Etat(s) contractant(s) suivant(s): GR)

1. Composés bicycliques aromatiques, caractérisés par le fait qu'ils répondent à la formule générale suivante : 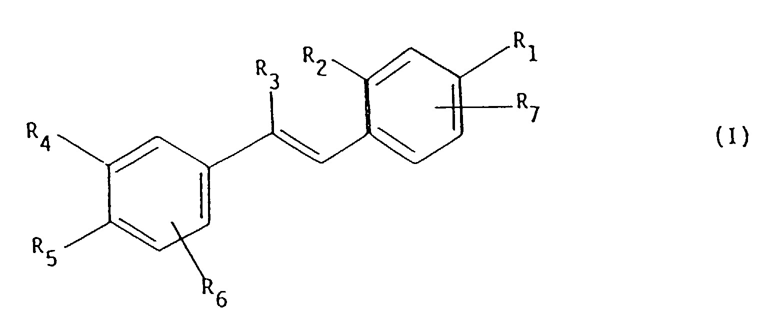 dans laquelle :
R₁ représente
(i) un atome d'hydrogène,
(ii) le radical -CH₃,
(iii) le radical -CH₂-O-R₈,
R₈ représentant un atome d'hydrogène ou un radical alkyle inférieur,
(iv) un radical -OR₈,
(v) un radical 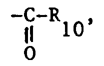 R₁₀ représentant :
(a) un atome d'hydrogène,
(b) un radical 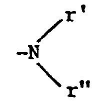
r' et r" représentant un atome
d'hydrogène, un radical alkyle inférieur, un radical mono ou polyhydroxyalkyle, un radical aryle éventuellement substitué ou un reste d'amino acide ou de sucre
ou encore pris ensemble forment un hétérocycle,
(c) un radical -OR₁₁
R₁₁ représentant un atome d'hydrogène, un radical alkyle linéaire ou ramifié ayant de 1 à 20 atomes de carbone, un radical mono ou polyhydroxyalkyle, un radicaux aryle ou aralkyle éventuellement substitué(s) ou un reste de sucre ou un reste d'aminoacide, et
(vi) un radical -S(O)ₜR₈,
t étant 0, 1 ou 2 et R₈ étant tel que défini ci-dessus,
R₂ représente un atome d'hydrogène,
R₃ représente un atome d'hydrogène, un radical aryle, un radical aralkyle ou un radical alkyle inférieur éventuellement substitué par un hydroxyle, par un alcoxy inférieur ou par un radical 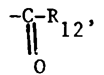
R₁₂ représentant un atome d'hydrogène, un radical alkyle inférieur, un radical hydroxyle, un radical alcoxy inférieur ou un radical 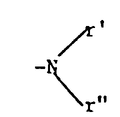
ou R₂ et R₃ pris ensemble forment avec le noyau benzénique un cycle naphtalénique,
R₄ représente un radical alkyle linéaire ou ramifié ou non ayant de 1 à 15 atomes de carbone ou un radical cycloaliphatique,
R₅ représente le radical -(CH₂)ₙ-R₁₃, le radical -CH=CH-(CH₂)ₙ-R₁₃, le radical -O(CH₂)ₘR₁₄
n étant 0 ou 1 à 6,
m étant 1 à 6
R₁₃ représentant le radical 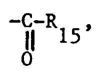
un radical monohydroxyalkyle, un radical polyhydroxyalkyle dont les hydroxyles sont éventuellement protégés sous forme de méthoxy ou d'acétoxy, un radical alkyle inférieur epoxydé ou le radical 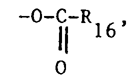
R₁₅ représentant le radical OR₁₆ ou le radical 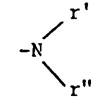
R₁₆ représentant un atome d'hydrogène, un radical alkyle inférieur, un radical aryle, ou un radical aralkyle,
R₁₄ représentant un radical hydroxyle lorsque m > 2, un radical monohydroxyalkyle, un radical polyhydroxyalkyle, le radical 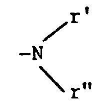
le radical 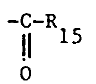 ou un radical alkényle mono ou polyhydroxylé ayant de 2 à 10 atomes de carbone, ou lorsque R₂ et R₃ ne sont pas pris ensemble m peut être 0 et/ou R₁₄ peut représenter un atome d'hydrogène ou un radical alkyle inférieur.
R₆ et R₇ représentent un atome d'hydrogène, un atome d'halogène, un radical alkyle inférieur ou le radical -OR₁₆,
R₅ et R₆ peuvent en outre former un cycle méthylène dioxy lorsque R₆ est en position 3 du noyau benzénique,
et les sels des composés de formule (I) lorsque R₁ représente une fonction acide carboxylique et les analogues chiraux desdits composés de formule (I) ainsi que les isomères géométriques desdits composés lorsque R₂ et R₃ ne sont pas pris ensemble.

2. Composés selon la revendication 1, caractérisés par le fait qu'ils se présentent sous forme de sels d'un métal alcalin ou alcalino-terreux ou encore de zinc ou d'une amine organique.

3. Composés selon la revendication 1, caractérisés par le fait qu'ils se présentent sous forme de sels d'un acide minéral ou organique choisi dans le groupe constitué par l'acide chlorhydrique, l'acide sulfurique, l'acide acétique, l'acide citrique, l'acide fumarique, l'acide hemisuccinique, l'acide maléique et l'acide mandélique.

4. Composés selon la revendication 1, caractérisés par le fait que le radical alkyle inférieur a de 1 à 6 atomes de carbone et est pris dans le groupe constitué par les radicaux méthyle, éthyle, isopropyle, butyle et tertiobutyle.

5. Composés selon la revendication 1, caractérisés par le fait que le radical alcoxy inférieur est pris dans le groupe constitué par un radical méthoxy, éthoxy, isopropoxy et butoxy.

6. Composés selon la revendication 1, caractérisés par le fait que le radical cycloaliphatique est choisi parmi le radical 1-méthyl cyclohexyle et le radical 1-adamantyle.

7. Composés selon la revendication 1, caractérisés par le fait que le radical monohydroxyalkyle est le radical hydroxyméthyle, 2-hydroxyéthyle, 2-hydroxypropyle, 3-hydroxypropyle, 4-hydroxybutyle, 5-hydroxypentyle ou 6-hydroxyhexyle.

8. Composés selon la revendication 1, caractérisés par le fait que le radical polyhydroxyalkyle comporte de 3 à 6 atomes de carbone et de 2 à 5 groupes hydroxyles et est pris dans le groupe constitué par le radical 2,3-dihydroxypropyle, 2,3,4-trihydroxybutyle, 2,3,4,5-tétrahydroxypentyle et le reste du pentaérythritol.

9. Composés selon la revendication 1, caractérisés par le fait que le radical aryle est un radical phényle éventuellement substitué par au moins un atome d'halogène, un hydroxyle ou une fonction nitro.

10. Composés selon la revendication 1, caractérisés par le fait que le radical aralkyle est le radical benzyle ou phénéthyle éventuellement substitué par au moins un atome d'halogène, un hydroxyle ou une fonction nitro.

11. Composés selon la revendication 1, caractérisés par le fait que l'hétérocycle est pris dans le groupe constitué par un radical pipéridino, morpholino, pyrrolidino ou pipérazino, éventuellement substitué en position 4 par un radical alkyle en C₁-C₆ ou mono ou polyhydroxyalkyle.

12. Composés selon l'une quelconque des revendications précédentes, caractérisés par le fait qu'ils sont pris dans le groupe constitué par :
Chlorhydrate de l'acide 6-[3-(1-adamantyl)-4-(3-aminopropyloxy) phényl]-2-naphtoïque;
6-[3-(1-adamantyl)-4-(2,3-dihydroxypropyloxy)phényl]-2-naphtoate de méthyle ;
Acide 6-[3-(1-adamantyl)-4-(2,3-dihydroxypropyloxy)phényl] -2-naphtoïque ;
6-[3-(1-adamantyl)-4-méthoxycarbonylméthyloxyphényl] -2-naphtoate de benzyle ;
Acide 6-[3-(1-adamantyl)-4-méthoxycarbonylméthyloxyphényl] -2-naphtoïque ;
Acide 6-[3-(1-adamantyl)-4-carboxyméthyloxyphényl] -2-naphtoïque ;
6-[3-(1-adamantyl)-4-(3-hydroxypropyloxy)phényl]-2-naphtoate de méthyle ;
Acide 6-[3-(1-adamantyl)-4-(3-hydroxypropyloxy)phényl] -2-naphtoïque ;
6-[3-(1-adamantyl)-4-benzylozycarbonylphényl]-2-naphtoate de méthyle ;
6-[3-(1-adamantyl)-4-carboxyphényl]-2-naphtoate de méthyle ;
6-[3-(1-adamantyl)-4-hydroxymethylphényl]-2-naphtoate de méthyle ;
Acide 6-[3-(1-adamantyl)-4-hydroxyméthylphényl]-2-naphtoïque;
Acide 6-[3-(1-adamantyl)-4-acétoxyméthylphény]-2-naphtoïque;
6-[3-(1-adamantyl)-4-methoxycarbonylphényl]-2-naphtoate de méthyle ;
Acide 6-[3-(1-adamantyl)-4-méthoxycarbonylphényl]-2-naphtoïque;
Acide 6-[3-(1-adamantyl)-4-carboxyphényl]-2-naphtoïque;
Acide 6-[3-(1-adamantyl)-4-carboxamidophényl]-2-naphtoïque;
6-[3-(1-adamantyl)-4-méthoxycarbonyléthénylphényl]-2-naphtoate de benzyle ;
Acide 6-[3-(1-adamantyl)-4-(méthoxycarbonyléthyl)phényl] -2-naphtoïque ;
6-[3-(1-adamantyl)-4-(2,3-époxypropyl)phényl]-2-naphtoate de benzyle ;
Acide 6-[3-(1-adamantyl)-4-(2-hydroxypropyl)phényl] -2-naphtoïque ;
Acide 6-[3-(1-adamantyl)-4-(3-méthoxy-2-hydroxypropyl)phényl] -2-naphtoïque.
Acide 4[(E)-2-(3-(1-adamantyl)-4-méthoxy phényl)propenyl] benzoïque ;
4-[(E)-2-(3-(1-adamantyl)-4-méthoxyphényl)propényl]benzoate de benzyle;
Acide 4-[(E)-2-(3-(1-adamantyl)-4-hydroxyphényl)-1-propényl]benzoïque ;
6-[3-(1-adamantyl)-4-méthoxycarbonylphényl]-2-naphtylcarboxamide;
4-[(E)-2-(3-(1-méthylcyclohexyl)-4-hydroxyphényl)propényl]benzoate diéthyle ;
4-[(E)-2-(3-(1-méthylcyclohexyl)-4-méthoxyphényl)propényl]benzoate d'éthyle ;
Acide 4-[(E)-2-(3-(1-méthylcyclohexyl)-4-méthoxyphényl)propényl]benzoïque ;
4-[(Z)-2-(3-(1-méthylcyclohexyl)-4-hydroxyphényl)propényl]benzoate d'éthyle ;
4-[(Z)-2-(3-(1-méthylcyclohexyl)-4-méthoxyphényl)propényl]benzoate d'éthyle ;
Acide 4-[(Z)-2-(3-(1-méthylcyclohexyl)-4-méthoxyphényl)propényl]benzoïque;
4-[(Z)-2-(3-tert-butyl-4-méthoxyphényl)propényl]benzoate d'éthyle ;
Acide 4-[(Z)-2-(3-tert-butyl-4-méthoxyphényl)propényl]benzoïque ;
Acide 4-[(E)-2-(3-tert-butyl-4-hydroxyphényl)propényl]benzoïque;
Acide 4-[(E)-2-(3-(1-méthylcyclohexyl)-4- hydroxyphényl)éthényl]benzoïque ;
4-[(E)-2-(3-(1-méthylcyclohexyl)-4-(6-tert-butoxycarbonylpentyloxy)phényl) éthényl]benzoate d'éthyle ;
4-[(E)-2-(3-(1-méthylcyclohexyl)-4-(6-carboxypentyloxy)phényl)éthényl]benzoate d'éthyle ;
Acide 4-[(E)-2-(3-(1-adamantyl)-4-hydroxyphényl)éthényl] benzoïque;
6-[3-(1-adamantyl)-4-(1,2-dihydroxyéthyl)phényl]-2-naphtoate de benzyle ;
Acide 6-[3-(1-adamantyl)-4-(1,2-dihydroxyéthyl)phényl]-2-naphtoïque ;
6-[3-(1-adamantyl)-4-(3-hydroxypropyl)phényl]-2-naphtoate de benzyl ;
Acide 6-[3-(1-adamantyl)-4-(3-hydroxypropyl)phényl]-2- naphtoïque ;
6-[3-(1-adamantyl)-4-(3-acétoxypropyl)phényl]-2-naphtoate de benzyle;
Acide 6-[3-(1-adamantyl)-4-(3-acétoxypropyl)phényl]-2-naphtoïque ;
6-[3-(1-adamantyl)-4-(2,3-dihydroxypropyl)phényl]-2-naphtoate de benzyle ;
Acide 6-[3-(1-adamantyl)-4-(2,3-dihydroxypropyl)phényl]-2-naphtoïque ;
N-méthoxycarbonylméthyl-4-(6-benzyloxycarbonylnaphtyl)-2-(1-adamantyl)phényl carboxamide ;
N-méthoxycarbonylméthyl4-(6-Carboxynaphtyl)-2-(1-adamantyl)phénylcarboxamide ;
Acide 6[3-(1-adamantyl)-4-(N,N-diméthylcarbamoyl)phényl]-2-naphtoïque ;
6[3-(1-adamantyl)4-(2(R),3-dihydroxypropyloxy)phényl]-2-naphtoate de benzyle ;
Acide 6-[3-(1-adamantyl)-4-(2(R),3-dihydroxypropyloxy)phényl]-2-naphtoïque;
Acide 6-[3-(1-adamantyl)-4,5-méthylène dioxyphényl]-2-naphtoïque;
N-éthyl 6-[3-(1-adamantyl)-4-méthoxycarbonylphényl]-2-naphtylcarboxamide;
N,N-morpholyl 6-[3-(1-adamantyl)-4-méthoxycarbonylphényl]-2-naphtylcarboxamide;
4-[(E)-2-(3-tert-butyl-4-méthoxyphényl)propényl]phénylcarbinol; et
4-[(E)-2-(3-tert-butyl-4-méthoxyphényl)propényl]benzylacétate.

13. Composition cosmétique pour l'hygiène corporelle et capillaire, caractérisée par le fait qu'elle contient, dans un véhicule cosmétique approprié, au moins un composé de formule (I) selon l'une quelconque des revendications 1 à 12.

14. Composition cosmétique selon la revendication 13, caractérisée par le fait qu'elle contient le composé de formule (I) à une concentration comprise entre 0,001 et 3 %.

## Claims (Claims for the following Contracting State(s): AT, BE, CH, DE, DK, FR, GB, IT, LI, NL, SE)

1. Aromatic bicyclic compounds, characterized by the fact that they correspond to the following general formula: 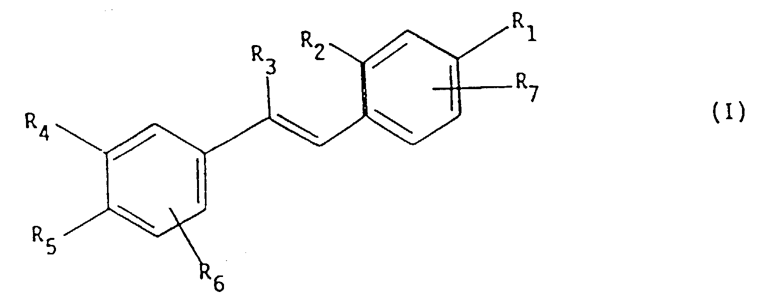 in which:
R₁ represents
(i) a hydrogen atom,
(ii) the radical -CH₃,
(iii) the radical -CH₂-O-R₈,
R₈ representing a hydrogen atom or a lower alkyl radical,
(iv) a radical -OR₈,
(v) a radical 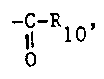 R₁₀ representing:
(a) a hydrogen atom,
(b) a radical 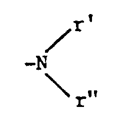 r' and r" representing a hydrogen atom, a lower alkyl radical, a mono- or polyhydroxyalkyl radical, an optionally substituted aryl radical or an amino acid or sugar residue or alternatively, taken together, form a heterocycle,
(c) a radical -OR₁₁
R₁₁ representing a hydrogen atom, a linear or branched alkyl radical having 1 to 20 carbon atoms, a mono- or polyhydroxyalkyl radical, an aryl or aralkyl radical which is (are) optionally substituted or a sugar residue or an amino acid residue, and
(vi) a radical -S(O)ₜR₈,
t being 0, 1 or 2 and R₈ being as defined above,
R₂ represents a hydrogen atom,
R₃ represents a hydrogen atom, an aryl radical, an aralkyl radical or a lower alkyl radical optionally substituted by a hydroxyl, by a lower alkoxy or by a radical 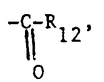
R₁₂ representing a hydrogen atom, a lower alkyl radical, a hydroxyl radical, a lower alkoxy radical or a radical 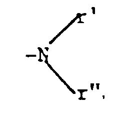
or R₂ and R₃, taken together, form, with the benzene nucleus, a naphthalene ring,
R₄ represents a linear or branched or unbranched alkyl radical having 1 to 15 carbon atoms or a cycloaliphatic radical,
R₅ represents the radical -(CH₂)ₙ-R₁₃, the radical -CH=CH-(CH₂)ₙ-R₁₃, or the radical -O(CH₂)ₘR₁₄
n being 0 or 1 to 6,
m being 1 to 6
R₁₃ representing the radical 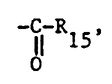
a monohydroxyalkyl radical or a polyhydroxyalkyl radical whose hydroxyls are optionally protected in methoxy or acetoxy form, an epoxidized lower alkyl radical or the radical 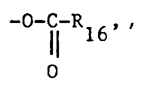
R₁₅ representing the radical OR₁₆ or the radical 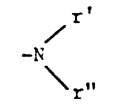
R₁₆ representing a hydrogen atom, a lower alkyl radical, an aryl radical or an aralkyl radical,
R₁₄ representing a hydroxyl radical when m > 2, a monohydroxyalkyl radical, a polyhydroxyalkyl radical, the radical 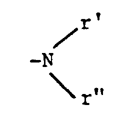 the radical 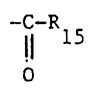 or a mono- or polyhydroxylated alkenyl radical having 2 to 10 carbon atoms, or when R₂ and R₃ are not taken together, m may be 0 and/or R₁₄ may represent a hydrogen atom or a lower alkyl radical,
R₆ and R₇ represent a hydrogen atom, a halogen atom, a lower alkyl radical or the radical -OR₁₆,
R₅ and R₆ may, in addition, form a methylenedioxy ring when R₆ is in the 3-position of the benzene nucleus, and the salts of the compounds of formula (I) when R₁ represents a carboxylic acid functional group and the chiral analogues of the said compounds of formula (I) and the geometrical isomers of the said compounds when R₂ and R₃ are not taken together.

2. Compounds according to Claim 1, characterized by the fact that they exist in the form of salts of an alkali or alkaline-earth metal or alternatively of zinc or of an organic amine.

3. Compounds according to Claim 1, characterized by the fact that they exist in the form of salts of an inorganic or organic acid chosen from the group consisting of hydrochloric acid, sulphuric acid, acetic acid, citric acid, fumaric acid, hemisuccinic acid, maleic acid and mandelic acid.

4. Compounds according to Claim 1, characterized by the fact that the lower alkyl radical has 1 to 6 carbon atoms and is chosen preferably from the group consisting of methyl, ethyl, isopropyl, butyl and tent-butyl radicals.

5. Compounds according to Claim 1, characterized by the fact that the lower alkoxy radical is chosen from the group consisting of a methoxy, ethoxy, isopropoxy and butoxy radical.

6. Compounds according to Claim 1, characterized by the fact that the cycloaliphatic radical is chosen from the 1-methylcyclohexyl radical and 1-adamantyl radical.

7. Compounds according to Claim 1, characterized by the fact that the monohydroxyalkyl radical is the hydroxymethyl, 2-hydroxyethyl, 2-hydroxypropyl, 3-hydroxypropyl, 4-hydroxybutyl, 5-hydroxypentyl or 6-hydroxyhexyl radical.

8. Compounds according to Claim 1, characterized by the fact that the polyhydroxyalkyl radical comprises 3 to 6 carbon atoms and 2 to 5 hydroxyl groups and is chosen from the group consisting of the 2,3-dihydroxypropyl, 2,3,4-trihydroxybutyl and 2,3,4,5-tetrahydroxypentyl radical and the pentaerythritol residue.

9. Compounds according to Claim 1, characterized by the fact that the aryl radical is a phenyl radical optionally substituted by at least one halogen atom, a hydroxyl or a nitro functional group.

10. Compounds according to Claim 1, characterized by the fact that the aralkyl radical is the benzyl or phenethyl radical optionally substituted by at least one halogen atom, a hydroxyl or a nitro functional group.

11. Compounds according to Claim 1, characterized by the fact that the heterocycle is chosen from the group consisting of a piperidino, morpholino, pyrrolidino or piperazino radical, optionally substituted in the 4-position by a C₁-C₆ alkyl radical or a mono- or polyhydroxyalkyl radical.

12. Compounds according to any one of the preceding claims, characterized by the fact that they are chosen from the group consisting of:
6-[3-(1-Adamantyl)-4-(3-aminopropyloxy)phenyl]-2-naphthoic acid hydrochloride;
Methyl 6-[3-(1-adamantyl)-4-(2,3-dihydroxypropyloxy)-phenyl]-2-naphthoate;
6-[3-(1-Adamantyl)-4-(2,3-dihydroxypropyloxy)phenyl]-2-naphthoic acid;
Benzyl 6-[3-(1-adamantyl)-4-methoxycarbonylmethyloxyphenyl]-2-naphthoate;
6-[3-(1-Adamantyl)-4-methoxycarbonylmethyloxyphenyl]-2-naphthoic acid;
6-[3-(1-Adamantyl)-4-carboxymethyloxyphenyl]-2-naphthoic acid;
Methyl 6-[3-(1-adamantyl)-4-(3-hydroxypropyloxy)phenyl]2-naphthoate;
6-[3-(1-Adamantyl)-4-(3-hydroxypropyloxy)phenyl]-2-naphthoic acid;
Methyl 6-[3-(1-adamantyl)-4-benzyloxycarbonylphenyl]-2-naphthoate;
Methyl 6-[3-(1-adamantyl)-4-carboxyphenyl]-2-naphthoate;
Methyl 6-[3-(1-adamantyl)-4-hydroxymethylphenyl]-2-naphthoate;
6-[3-(1-Adamantyl)-4-hydroxymethylphenyl]-2-naphthoic acid;
6-[3-(1-Adamantyl)-4-acetoxymethylphenyl]-2-naphthoic acid;
Methyl 6-[3-(1-adamantyl)-4-methoxycarbonylphenyl]-2-naphthoate;
6-[3-(1-Adamantyl)-4-methoxycarbonylphenyl]-2-naphthoic acid;
6-[3-(1-Adamantyl)-4-carboxyphenyl]-2-naphthoic acid;
6-[3-(1-Adamantyl)-4-carboxamidophenyl]-2-naphthoic acid;
Benzyl 6-[3-(1-adamantyl)-4-methoxycarbonylethenylphenyl]-2-naphthoate;
6-[3-(1-Adamantyl)-4-(methoxycarbonylethyl)phenyl]-2-naphthoic acid;
Benzyl 6-[3-(1-adamantyl)-4-(2,3-epoxypropyl)phenyl]-2-naphthoate;
6-[3-(1-Adamantyl)-4-(2-hydroxypropyl)phenyl]-2-naphthoic acid;
6-[3-(1-Adamantyl)-4-(3-methoxy-2-hydroxypropyl)phenyl]-2-naphthoic acid;
4-[(E)-2-(3-(1-adamantyl)-4-methoxyphenyl)propenyl]-benzoic acid;
Benzyl 4-[(E)-2-(3-(1-adamantyl)-4-methoxyphenyl)propenyl]benzoate;
4-[(E)-2-(3-(1-adamantyl)-4-hydroxyphenyl)-1-propenyl]benzoic acid;
6-[3-(1-Adamantyl)-4-methoxycarbonylphenyl]-2-naphthylcarboxamide;
Ethyl 4- [(E)-2-(3-(1-methylcyclohexyl)-4-hydroxyphenyl)-propenyl]benzoate;
Ethyl 4-[(E)-2-(3-(1-methylcyclohexyl)-4-methoxyphenyl)propenyl]benzoate;
4-[(E)-2-(3-(1-methylcyclohexyl)-4-methoxyphenyl)propenyl]benzoic acid;
Ethyl 4-[(Z)-2-(3-(1-methylcyclohexyl)-4-hydroxyphenyl)-propenyl]benzoate;
Ethyl 4-[(Z)-2-(3-(1-methylcyclohexyl)-4-methoxyphenyl)-propenyl]benzoate;
4-[(Z)-2-(3-(1-methylcyclohexyl)-4-methoxyphenyl)propenyl]benzoic acid;
Ethyl 4-[(Z)-2-(3-tert-butyl-4-methoxyphenyl)propenyl]benzoate;
4-[(Z)-2-(3-tert-butyl-4-methoxyphenyl)propenyl]benzoic acid;
4-[(E)-2-(3-tert-butyl-4-hydroxyphenyl)propenyl]benzoic acid;
4-[(E)-2-(3-(1-methylcyclohexyl)-4-hydroxyphenyl)ethenyl]benzoic acid;
Ethyl 4-[(E)-2-(3-(1-methylcyclohexyl)-4-(6-tert-butoxycarbonylpentyloxy)phenyl)ethenyl]benzoate;
Ethyl 4-[(E)-2-(3-(1-methylcyclohexyl)-4-(6-carboxypentyloxy)phenyl)ethenyl]benzoate;
4-[(E)-2-(3-(1-adamantyl)-4-hydroxyphenyl)ethenyl]benzoic acid;
Benzyl 6-[3-(1-adamantyl)-4-(1,2-dihydroxyethyl)phenyl]2-naphthoate;
6-[3-(1-Adamantyl)-4-(1,2-dihydroxyethyl)phenyl]-2-naphthoic acid;
Benzyl 6-[3-(1-adamantyl)-4-(3-hydroxypropyl)phenyl]-2-naphthoate;
6-[3-(1-Adamantyl)-4-(3-hydroxypropyl)phenyl]-2-naphthoic acid;
Benzyl 6-[3-(1-adamantyl)-4-(3-acetoxypropyl)phenyl]-2-naphthoate;
6-[3-(1-Adamantyl)-4-(3-acetoxypropyl)phenyl]-2-naphthoic acid;
Benzyl 6-[3-(1-adamantyl)-4-(2,3-dihydroxypropyl)phenyl]-2-naphthoate;
6-[3-(1-Adamantyl)-4-(2,3-dihydroxypropyl)phenyl]-2-naphthoic acid;
N-methoxycarbonylmethyl-4-(6-benzyloxycarbonylnaphthyl)2-(1-adamantyl)phenylcarboxamide;
N-methoxycarbonylmethyl-4-(6-carboxynaphthyl)-2-(1-adamantyl)phenylcarboxamide;
6-[3-(1-Adamantyl)-4-(N,N-dimethylcarbamoyl)phenyl]-2-naphthoic acid;
Benzyl 6-[3-(1-adamantyl)-4-(2(R),3-dihydroxypropyloxy)phenyl]-2-naphthoate;
6-[3-(1-Adamantyl)-4-(2(R),3-dihydroxypropyloxy)phenyl]2-naphthoic acid;
6-[3-(1-Adamantyl)-4,5-methylenedioxyphenyl]-2-naphthoic acid;
N-ethyl 6-[3-(1-adamantyl)-4-methoxycarbonylphenyl]-2-naphthylcarboxamide;
N,N-morpholyl 6-[3-(1-adamantyl)-4-methoxycarbonylphenyl]-2-naphthylcarboxamide;
4-[(E)-2-(3-tert-butyl-4-methoxyphenyl)propenyl]phenylcarbinol; and
4-(E)-2-(3-tert-butyl-4-methoxyphenyl)propenyl]benzylacetate.

13. Pharmaceutical composition, characterized by the fact that it contains, in a vehicle suitable for an enteral, parenteral, topical or ocular administration, at least one compound of formula (I) according to any one of Claims 1 to 12.

14. Composition according to Claim 13, characterized by the fact that it contains from 0.001 to about 5 % by weight of a compound of formula (I).

15. Use of a compound according to any one of Claims 1 to 12 for the preparation of a pharmaceutical composition intended for the treatment of dermatological, rheumatic, respiratory as well as ophthalmological conditions.

16. Cosmetic composition for body and hair care, characterized by the fact that it contains, in a suitable cosmetic vehicle, at least one compound of formula (I) according to any one of Claims 1 to 12.

17. Cosmetic composition according to Claim 16, characterized by the fact that it contains the compound of formula (I) at a concentration of between 0.001 and 3 %.

## Claims (Claims for the following Contracting State(s): ES)

1. Cosmetic composition for body and hair care, characterized by the fact that it contains, in a suitable cosmetic vehicle, at least one compound of formula (I) below: 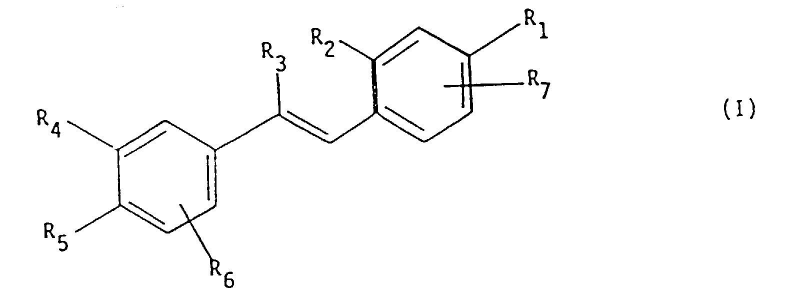 in which:
R₁ represents
(i) a hydrogen atom,
(ii) the radical -CH₃,
(iii) the radical -CH₂-O-R₈,
R₈ representing a hydrogen atom or a lower alkyl radical,
(iv) a radical -OR₈,
(v) a radical 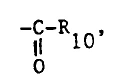 R₁₀ representing:
(a) a hydrogen atom,
(b) a radical 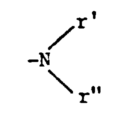 r' and r" representing a hydrogen atom, a lower alkyl radical, a mono- or polyhydroxyalkyl radical, an optionally substituted aryl radical or an amino acid or sugar residue or alternatively, taken together, form a heterocycle,
(c) a radical -OR₁₁
R₁₁ representing a hydrogen atom, a linear or branched alkyl radical having 1 to 20 carbon atoms, a mono- or polyhydroxyalkyl radical, an aryl or aralkyl radical which is (are) optionally substituted or a sugar residue or an amino acid residue, and
(vi) a radical -S(O)ₜR₈,
t being 0, 1 or 2 and R₈ being as defined above,
R₂ represents a hydrogen atom,
R₃ represents a hydrogen atom, an aryl radical, an aralkyl radical or a lower alkyl radical optionally substituted by a hydroxyl, by a lower alkoxy or by a radical 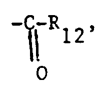
R₁₂ representing a hydrogen atom, a lower alkyl radical, a hydroxyl radical, a low alkoxy radical or a radical 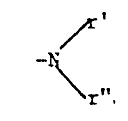
or R₂ and R₃, taken together, form, with the benzene nucleus, a naphthalene ring,
R₄ represents a linear or branched or unbranched alkyl radical having 1 to 15 carbon atoms or a cycloaliphatic radical,
R₅ represents the radical -(CH₂)ₙ-R₁₃, the radical -CH=CH-(CH₂)ₙ-R₁₃, or the radical -O(CH₂)ₘR₁₄
n being 0 or 1 to 6,
m being 1 to 6
R₁₃ representing the radical 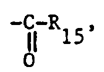
a monohydroxyalkyl radical or a polyhydroxyalkyl radical whose hydroxyls are optionally protected in methoxy or acetoxy form, an epoxidized lower alkyl radical or the radical 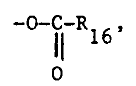
R₁₅ representing the radical OR₁₆ or the radical 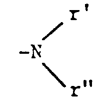
R₁₆ representing a hydrogen atom, a lower alkyl radical, an aryl radical or an aralkyl radical,
R₁₄ representing a hydroxyl radical when m > 2, a monohydroxyalkyl radical, a polyhydroxyalkyl radical, the radical 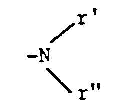 the radical 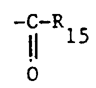 or a mono- or polyhydroxylated alkenyl radical having 2 to 10 carbon atoms, or when R₂ and R₃ are not taken together, m may be 0 and/or R₁₄ may represent a hydrogen atom or a lower alkyl radical,
R₆ and R₇ represent a hydrogen atom, a halogen atom, a lower alkyl radical or the radical -OR₁₆,
R₅ and R₆ may, in addition, form a methylenedioxy ring when R₆ is in the 3-position of the benzene nucleus, and the salts of the compounds of formula (I) when R₁ represents a carboxylic acid functional group and the chiral analogues of the said compounds of formula (I) and the geometrical isomers of the said compounds when R₂ and R₃ are not taken together.

2. Composition according to Claim 1, characterized by the fact that the compound of formula (I) exists in the form of a salt of an alkali or alkaline-earth metal or alternatively of zinc or of an organic amine.

3. Composition according to Claim 1, characterized by the fact that the compound of formula (I) exists in the form of a salt of an inorganic or organic acid chosen from the group consisting of hydrochloric acid, sulphuric acid, acetic acid, citric acid, fumaric acid, hemisuccinic acid, maleic acid and mandelic acid.

4. Composition according to any one of the preceding claims, characterized by the fact that the compound of formula (I) is chosen from the group consisting of:
6-[3-(1-Adamantyl)-4-(3-aminopropyloxy)phenyl]-2-naphthoic acid hydrochloride;
Methyl 6-[3-(1-adamantyl)-4-(2,3-dihydroxypropyloxy)phenyl] -2-naphthoate;
6-[3-(1-Adamantyl)-4-(2,3-dihydroxypropyloxy)phenyl]-2-naphthoic acid;
Benzyl 6-[3-(1-adamantyl)-4-methoxycarbonylmethyloxyphenyl]-2-naphthoate;
6-[3-(1-Adamantyl)-4-methoxycarbonylmethyloxyphenyl]-2-naphthoic acid;
6-[3-(1-Adamantyl)-4-carboxymethyloxyphenyl]-2-naphthoic acid;
Methyl 6-[3-(1-adamantyl)-4-(3-hydroxypropyloxy)phenyl]-2-naphthoate;
6- [3-(1-Adamantyl)-4-(3-hydroxypropyloxy)phenyl]-2-naphthoic acid;
Methyl 6-[3-(1-adamantyl)-4-benzyloxycarbonylphenyl]-2-naphthoate;
Methyl 6-[3-(1-adamantyl)-4-carboxyphenyl]-2-naphthoate;
Methyl 6-[3-(1-adamantyl)-4-hydroxymethylphenyl]-2-naphthoate; 6-[3-(1-Adamantyl)-4-hydroxymethylphenyl]-2-naphthoic acid;
6-[3-(1-Adamantyl)-4-acetoxymethylphenyl]-2-naphthoic acid;
Methyl 6-[3-(1-adamantyl)-4-methoxycarbonylphenyl]-2-naphthoate;
6-[3-(1-Adamantyl)-4-methoxycarbonylphenyl]-2-naphthoic acid;
6-[3-(1-Adamantyl)-4-carboxyphenyl]-2-naphthoic acid;
6-[3-(1-Adamantyl)-4-carboxamidophenyl]-2-naphthoic acid;
Benzyl 6-[3-(1-adamantyl)-4-methoxycarbonylethenylphenyl]-2-naphthoate;
6-[3-(1-Adamantyl)-4-(methoxycarbonylethyl)phenyl]-2-naphthoic acid;
Benzyl 6-[3-(1-adamantyl)-4-(2,3-epoxypropyl)phenyl]-2-naphthoate;
6-[3-(1-Adamantyl)-4-(2-hydroxypropyl)phenyl]-2-naphthoic acid;
6-[3-(1-Adamantyl)-4-(3-methoxy-2-hydroxypropyl)phenyl]-2-naphthoic acid;
4-[(E)-2-(3-(1-adamantyl)-4-methoxyphenyl)propenyl]-benzoic acid;
Benzyl 4-[(E)-2-(3-(1-adamantyl)-4-methoxyphenyl)propenyl]benzoate;
4-[(E)-2-(3-(1-adamantyl)-4-hydroxyphenyl)-1-propenyl]benzoic acid;
6-[3-(1-Adamantyl)-4-methoxycarbonylphenyl]-2-naphthylcarboxamide;
Ethyl 4-[(E)-2-(3-(1-methylcyclohexyl)-4-hydroxyphenyl)-propenyl]benzoate;
Ethyl 4-[(E)-2-(3-(1-methylcyclohexyl)-4-methoxyphenyl)-propenyl]benzoate;
4-[(E)-2-(3-(1-methylcyclohexyl)-4-methoxyphenyl)propenyl]benzoic acid;
Ethyl 4-[(Z)-2-(3-(1-methylcyclohexyl)-4-hydroxyphenyl)propenyl]benzoate;
Ethyl 4-[(Z)-2-(3-(1-methylcyclohexyl)-4-methoxyphenyl)-propenyl]benzoate;
4-[(Z)-2-(3-(1-methylcyclohexyl)-4-methoxyphenyl)propenyl]benzoic acid;
Ethyl 4-[(Z)-2-(3-tert-butyl-4-methoxyphenyl)propenyl]benzoate;
4-[(Z)-2-(3-tert-butyl-4-methoxyphenyl)propenyl]benzoic acid;
4-[(E)-2-(3-tert-butyl-4-hydroxyphenyl)propenyl]benzoic acid;
4-[(E)-2-(3-(1-methylcyclohexyl)-4-hydroxyphenyl)ethenyl]benzoic acid;
Ethyl 4-[(E)-2-(3-(1-methylcyclohexyl)-4-(6-tert-butoxycarbonylpentyloxy)phenyl)ethenyl]benzoate;
Ethyl 4-[(E)-2-(3-(1-methylcyclohexyl)-4-(6-carboxypentyloxy)phenyl)ethenyl]benzoate;
4-[(E)-2-(3-(1-adamantyl)-4-hydroxyphenyl)ethenyl]benzoic acid;
Benzyl 6-[3-(1-adamantyl)-4-(1,2-dihydroxyethyl)phenyl]2-naphthoate;
6-[3-(1-Adamantyl)-4-(1,2-dihydroxyethyl)phenyl]-2-naphthoic acid;
Benzyl 6-[3-(1-adamantyl)-4-(3-hydroxypropyl)phenyl]-2-naphthoate;
6-[3-(1-Adamantyl)-4-(3-hydroxypropyl)phenyl]-2-naphthoic acid;
Benzyl 6-[3-(1-adamantyl)-4-(3-acetoxypropyl)phenyl]-2-naphthoate;
6-[3-(1-Adamantyl)-4-(3-acetoxypropyl)phenyl]-2-naphthoic acid;
Benzyl 6-[3-(1-adamantyl)-4-(2,3-dihydroxypropyl)phenyl]-2-naphthoate;
6-[3-(1-Adamantyl)-4-(2,3-dihydroxypropyl)phenyl]-2-naphthoic acid;
N-methoxycarbonylmethyl-4-(6-benzyloxycarbonylnaphthyl)-2-(1-adamantyl)phenylcarboxamide;
N-methoxycarbonylmethyl-4-(6-carboxynaphthyl)-2-(1-adamantyl)phenylcarboxamide;
6-[3-(1-Adamantyl)-4-(N,N-dimethylcarbamoyl)phenyl]-2-naphthoic acid;
Benzyl 6-[3-(1-adamantyl)-4-(2(R),3-dihydroxypropyloxy)phenyl]-2-naphthoate;
6-[3-(1-Adamantyl)-4-(2(R),3-dihydroxypropyloxy)phenyl]-2-naphthoic acid;
6-[3-(1-Adamantyl)-4,5-methylenedioxyphenyl]-2-naphthoic acid;
N-ethyl 6-[3-(1-adamantyl)-4-methoxycarbonylphenyl]-2-naphthylcarboxamide;
N,N-morpholyl 6-[3-(1-adamantyl)-4-methoxycarbonylphenyl]-2-naphthylcarboxamide;
4-[(E)-2-(3-tert-butyl-4-methoxyphenyl)propenyl]phenylcarbinol; and
4-(E)-2-(3-tert-butyl-4-methoxyphenyl)propenyl]benzylacetate.

5. Cosmetic composition according to any one of the preceding claims, characterized by the fact that it contains the compound of formula (I) at a concentration of between 0.0001 and 3 %.

## Claims (Claims for the following Contracting State(s): GR)

1. Aromatic bicyclic compounds, characterized by the fact that they correspond to the following general formula: 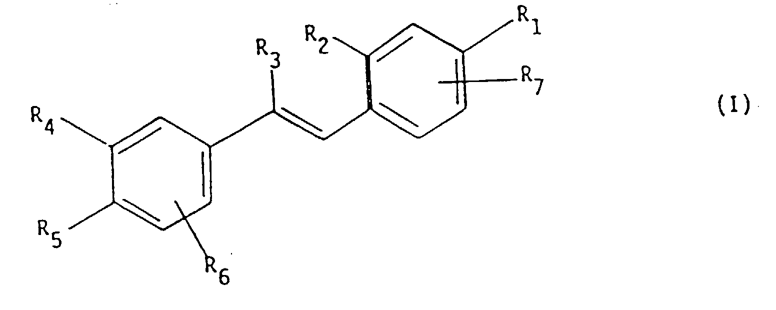 in which:
R₁ represents
(i) a hydrogen atom,
(ii) the radical -CH₃,
(iii) the radical -CH₂-O-R₈,
R₈ representing a hydrogen atom or a lower alkyl radical,
(iv) a radical -OR₈,
(v) a radical 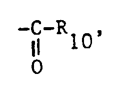 R₁₀ representing:
(a) a hydrogen atom,
(b) a radical 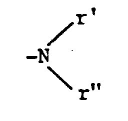 r' and r'' representing a hydrogen atom, a lower alkyl radical, a mono- or polyhydroxyalkyl radical, an optionally substituted aryl radical or an amino acid or sugar residue or alternatively, taken together, form a heterocycle,
(c) a radical -OR₁₁
R₁₁ representing a hydrogen atom, a linear or branched alkyl radical having 1 to 20 carbon atoms, a mono- or polyhydroxyalkyl radical, an aryl or aralkyl radical which is (are) optionally substituted or a sugar residue or an amino acid residue, and
(vi) a radical -S(O)ₜR₈,
t being 0, 1 or 2 and R₈ being as defined above,
R₂ represents a hydrogen atom,
R₃ represents a hydrogen atom, an aryl radical, an aralkyl radical or a lower alkyl radical optionally substituted by a hydroxyl, by a lower alkoxy or by a radical 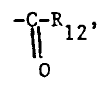
R₁₂ representing a hydrogen atom, a lower alkyl radical, a hydroxyl radical, a low alkoxy radical or a radical 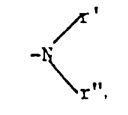
or R₂ and R₃, taken together, form, with the benzene nucleus, a naphthalene ring,
R₄ represents a linear or branched or unbranched alkyl radical having 1 to 15 carbon atoms or a cycloaliphatic radical,
R₅ represents the radical -(CH₂)ₙ-R₁₃, the radical -CH=CH-(CH₂)ₙ-R₁₃, or the radical -O(CH₂)ₘR₁₄
n being 0 or 1 to 6,
m being 1 to 6
R₁₃ representing the radical 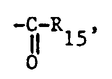
a monohydroxyalkyl radical or a polyhydroxyalkyl radical whose hydroxyls are optionally protected in methoxy or acetoxy form, an epoxidized lower alkyl radical or the radical 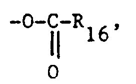
R₁₅ representing the radical OR₁₆ or the radical 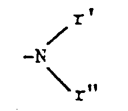
R₁₆ representing a hydrogen atom, a lower alkyl radical, an aryl radical or an aralkyl radical,
R₁₄ representing a hydroxyl radical when m > 2, a monohydroxyalkyl radical, a polyhydroxyalkyl radical, the radical 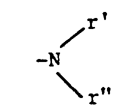 the radical 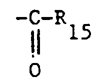 or a mono- or polyhydroxylated alkenyl radical having 2 to 10 carbon atoms, or when R₂ and R₃ are not taken together, m may be 0 and/or R₁₄ may represent a hydrogen atom or a lower alkyl radical,
R₆ and R₇ represent a hydrogen atom, a halogen atom, a lower alkyl radical or the radical -OR₁₆,
R₅ and R₆ may, in addition, form a methylenedioxy ring when R₆ is in the 3-position of the benzene nucleus, and the salts of the compounds of formula (I) when R₁ represents a carboxylic acid functional group and the chiral analogues of the said compounds of formula (I) and the geometrical isomers of the said compounds when R₂ and R₃ are not taken together.

2. Compounds according to Claim 1, characterized by the fact that they exist in the form of salts of an alkali or alkaline-earth metal or alternatively of zinc or of an organic amine.

3. Compounds according to Claim 1, characterized by the fact that they exist in the form of salts of an inorganic or organic acid chosen from the group consisting of hydrochloric acid, sulphuric acid, acetic acid, citric acid, fumaric acid, hemisuccinic acid, maleic acid and mandelic acid.

4. Compounds according to Claim 1, characterized by the fact that the lower alkyl radical has 1 to 6 carbon atoms and is chosen from the group consisting of methyl, ethyl, isopropyl, butyl and tent-butyl radicals.

5. Compounds according to Claim 1, characterized by the fact that the lower alkoxy radical is chosen from the group consisting of a methoxy, ethoxy, isopropoxy and butoxy radical.

6. Compounds according to Claim 1, characterized by the fact that the cycloaliphatic radical is chosen from the 1-methylcyclohexyl radical and 1-adamantyl radical.

7. Compounds according to Claim 1, characterized by the fact that the monohydroxyalkyl radical is the hydroxymethyl, 2-hydroxyethyl, 2-hydroxypropyl, 3-hydroxypropyl, 4-hydroxybutyl, 5-hydroxypentyl or 6-hydroxyhexyl radical.

8. Compounds according to Claim 1, characterized by the fact that the polyhydroxyalkyl radical comprises 3 to 6 carbon atoms and 2 to 5 hydroxyl groups and is chosen from the group consisting of the 2,3-dihydroxypropyl, 2,3,4-trihydroxybutyl and 2,3,4,5-tetrahydroxypentyl radical and the pentaerythritol residue.

9. Compounds according to Claim 1, characterized by the fact that the aryl radical is a phenyl radical optionally substituted by at least one halogen atom, a hydroxyl or a nitro functional group.

10. Compounds according to Claim 1, characterized by the fact that the aralkyl radical is the benzyl or phenethyl radical optionally substituted by at least one halogen atom, a hydroxyl or a nitro functional group.

11. Compounds according to Claim 1, characterized by the fact that the heterocycle is chosen from the group consisting of a piperidino, morpholino, pyrrolidino or piperazino radical, optionally substituted in the 4-position by a C₁-C₆ alkyl radical or a mono- or polyhydroxyalkyl radical.

12. Compounds according to any one of the preceding claims, characterized by the fact that they are chosen from the group consisting of:
6-[3-(1-Adamantyl)-4-(3-aminopropyloxy)phenyl]-2-naphthoic acid hydrochloride;
Methyl 6-[3-(1-adamantyl)-4-(2,3-dihydroxypropyloxy)phenyl]-2-naphthoate;
6-[3-(1-Adamantyl)-4-(2,3-dihydroxypropyloxy)phenyl]-2-naphthoic acid;
Benzyl 6-[3-(1-adamantyl)-4-methoxycarbonylmethyloxyphenyl]-2-naphthoate;
6-[3-(1-Adamantyl)-4-methoxycarbonylmethyloxyphenyl]-2-naphthoic acid;
6-[3-(1-Adamantyl)-4-carboxymethyloxyphenyl]-2-naphthoic acid;
Methyl 6-[3-(1-adamantyl)-4-(3-hydroxypropyloxy)phenyl]2-naphthoate;
6-[3-(1-Adamantyl)-4-(3-hydroxypropyloxy)phenyl]-2-naphthoic acid;
Methyl 6-[3-(1-adamantyl)-4-benzyloxycarbonylphenyl]-2-naphthoate;
Methyl 6-[3-(1-adamantyl)-4-carboxyphenyl]-2-naphthoate;
Methyl 6-[3-(1-adamantyl)-4-hydroxymethylphenyl]-2-naphthoate;
6-[3-(1-Adamantyl)-4-hydroxymethylphenyl]-2-naphthoic acid;
6-[3-(1-Adamantyl)-4-acetoxymethylphenyl]-2-naphthoic acid;
Methyl 6-[3-(1-adamantyl)-4-methoxycarbonylphenyl]-2-naphthoate;
6-[3-(1-Adamantyl)-4-methoxycarbonylphenyl]-2-naphthoic acid;
6-[3-(1-Adamantyl)-4-carboxyphenyl]-2-naphthoic acid;
6-[3-(1-Adamantyl)-4-carboxamidophenyl]-2-naphthoic acid;
Benzyl 6-[3-(1-adamantyl)-4-methoxycarbonylethenylphenyl]-2-naphthoate;
6-[3-(1-Adamantyl)-4-(methoxycarbonylethyl)phenyl]-2-naphthoic acid;
Benzyl 6-[3-(1-adamantyl)-4-(2,3-epoxypropyl)phenyl]-2-naphthoate;
6-[3-(1-Adamantyl)-4-(2-hydroxypropyl)phenyl]-2-naphthoic acid;
6-[3-(1-Adamantyl)-4-(3-methoxy-2-hydroxypropyl)phenyl]-2-naphthoic acid;
4-[(E)-2-(3-(1-adamantyl)-4-methoxyphenyl)propenyl]-benzoic acid;
Benzyl 4-[(E)-2-(3-(1-adamantyl)-4-methoxyphenyl)propenyl]benzoate;
4-[(E)-2-(3-(1-adamantyl)-4-hydroxyphenyl)-1-propenyl]benzoic acid;
6-[3-(1-Adamantyl)-4-methoxycarbonylphenyl]-2-naphthylcarboxamide;
Ethyl 4-[(E)-2-(3-(1-methylcyclohexyl)-4-hydroxyphenyl)-propenyl]benzoate;
Ethyl 4-[(E)-2-(3-(1-methylcyclohexyl)-4-methoxyphenyl)propenyl]benzoate;
4-[(E)-2-(3-(1-methylcyclohexyl)-4-methoxyphenyl)propenyl]benzoic acid;
Ethyl 4-[(Z)-2-(3-(1-methylcyclohexyl)-4-hydroxyphenyl)propenyl]benzoate;
Ethyl 4-[(Z)-2-(3-(1-methylcyclohexyl)-4-methoxyphenyl)propenyl]benzoate;
4-[(Z)-2-(3-(1-methylcyclohexyl)-4-methoxyphenyl)propenyl]benzoic acid;
Ethyl 4-[(Z)-2-(3-tert-butyl-4-methoxyphenyl)propenyl]benzoate;
4-[(Z)-2-(3-tert-butyl-4-methoxyphenyl)propenyl]benzoic acid;
4-[(E)-2-(3-tert-butyl-4-hydroxyphenyl)propenyl]benzoic acid;
4-[(E)-2-(3-(1-methylcyclohexyl)-4-hydroxyphenyl)ethenyl]benzoic acid;
Ethyl 4-[(E)-2-(3-(1-methylcyclohexyl)-4-(6-tert-butoxycarbonylpentyloxy)phenyl)ethenyl]benzoate;
Ethyl 4-[(E)-2-(3-(1-methylcyclohexyl)-4-(6-carboxypentyloxy)phenyl)ethenyl]benzoate;
4-[(E)-2-(3-(1-adamantyl)-4-hydroxyphenyl)ethenyl]benzoic acid;
Benzyl 6-[3-(1-adamantyl)-4-(1,2-dihydroxyethyl)phenyl]2-naphthoate;
6-[3-(1-Adamantyl)-4-(1,2-dihydroxyethyl)phenyl]-2-naphthoic acid;
Benzyl 6-[3-(1-adamantyl)-4-(3-hydroxypropyl)phenyl]-2-naphthoate;
6-[3-(1-Adamantyl)-4-(3-hydroxypropyl)phenyl]-2-naphthoic acid;
Benzyl 6-[3-(1-adamantyl)-4-(3-acetoxypropyl)phenyl]-2-naphthoate;
6-[3-(1-Adamantyl)-4-(3-acetoxypropyl)phenyl]-2-naphthoic acid;
Benzyl 6-[3-(1-adamantyl)-4-(2,3-dihydroxypropyl)phenyl]-2-naphthoate;
6-[3-(1-Adamantyl)-4-(2,3-dihydroxypropyl)phenyl]-2-naphthoic acid;
N-methoxycarbonylmethyl-4-(6-benzyloxycarbonylnaphthyl)2-(1-adamantyl)phenylcarboxamide;
N-methoxycarbonylmethyl-4-(6-carboxynaphthyl)-2-(1-adamantyl)phenylcarboxamide;
6-[3-(1-Adamantyl)-4-(N,N-dimethylcarbamoyl)phenyl]-2-naphthoic acid;
Benzyl 6-[3-(1-adamantyl)-4-(2(R),3-dihydroxypropyloxy)phenyl]-2-naphthoate;
6-[3-(1-Adamantyl)-4-(2(R),3-dihydroxypropyloxy)phenyl]2-naphthoic acid;
6-[3-(1-Adamantyl)-4,5-methylenedioxyphenyl]-2-naphthoic acid;
N-ethyl 6-[3-(1-adamantyl)-4-methoxycarbonylphenyl]-2-naphthylcarboxamide;
N,N-morpholyl 6-[3-(1-adamantyl)-4-methoxycarbonylphenyl]-2-naphthylcarboxamide;
4-[(E)-2-(3-tert-butyl-4-methoxyphenyl)propenyl]phenylcarbinol; and
4-(E)-2-(3-tert-butyl-4-methoxyphenyl)propenyl]benzylacetate.

13. Cosmetic composition for body and hair care, characterized by the fact that it contains, in a suitable cosmetic vehicle, at least one compound of formula (I) according to any one of Claims 1 to 12.

14. Cosmetic composition according to Claim 13, characterized by the fact that it contains the compound of formula (I) at a concentration of between 0.001 and 3 %.

## Patentansprüche (Patentansprüche für folgende(n) Vertragsstaat(en): AT, BE, CH, DE, DK, FR, GB, IT, LI, NL, SE)

1. Verbindungen mit zwei aromatischen Ringen, dadurch gekennzeichnet, daß sie der folgenden allgemeinen Formel entsprechen: 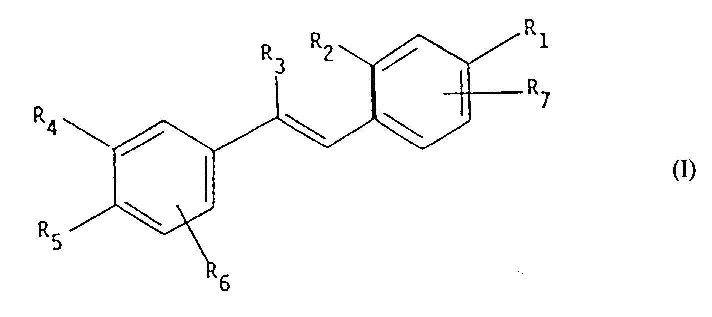 worin,
**R**_{**1**}
(i) ein Wasserstoffatom,
(ii) die Gruppe CH₃,
(iii) den Rest CH₂-O-R₈ bedeutet, wobei
**R**_{**8**} ein Wasserstoffatom oder einen niedrigen Alkylrest darstellt,
(iv) den Rest -OR₈ und
(v) den Rest 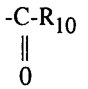 darstellt, wobei
**R**_{**10**}
(a) ein Wasserstoffatom, oder
(b) den Rest 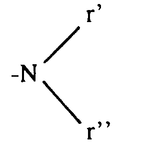 darstellt, wobei r' und r" ein Wasserstoffatom, einen niedrigen Alkylrest, einen Mono- oder Polyhydroxyalkylrest, einen gegebenenfalls substituierten Arylrest oder einen Aminosäure- oder Zuckerrest oder zusammengenommen einen Heterocyclus bedeuten,
(c) einen Rest -OR₁₁ bedeutet, wobei
**R**_{**11**} ein Wasserstoffatom, einen gerad- oder verzweigtkettigen Alkylrest mit 1 bis 20 Kohlenstoffatomen, einen Mono- oder Polyhydroxyalkylrest, einen oder mehrere ggf. substituierte Aryl- oder Aralkylreste oder einen Zucker- oder Aminosäurerest darstellt, und
(vi) einen Rest -S(O)ₜR₈ bedeutet, wobei
t die Zahl 0, 1 oder 2 bedeutet, und R₈ die obenstehende Bedeutung aufweist,
**R**_{**2**} ein Wasserstoffatom und
**R**_{**3**} ein Wasserstoffatom, einen Alkylrest, einen ggf. durch eine Hydroxylgruppe, ein niedriges Alkoxy oder durch einen Rest 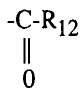 ggf. substituierten Aralkylrest oder einen niedrigen Alkylrest bedeuten, wobei
**R**_{**12**} ein Wasserstoffatom, einen niedrigen Alkylrest, eine Hydroxylgruppe, einen niedrigen Alkoxyrest oder einen Rest 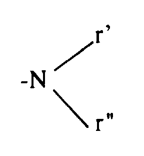 bedeutet oder R₂ und R₃ zusammengenommen mit dem Benzolring einen Naphthalinring bilden,
**R**_{**4**} einen gerad- oder ggf. verzweigtkettigen Alkylrest mit 1-5 Kohlenstoffatomen oder einen cycloaliphatischen Rest bedeutet,
**R**_{**5**} den Rest (CH₂)ₙ-R₁₃, den Rest CH=CH-(CH₂)ₙ-R₁₃ oder den Rest -O(CH₂)ₘR₁₄ darstellt, wobei
n die Zahl 0 oder 1 bis 6 und
m die Zahl 1 bis 6 bedeuten und
**R**_{**13**} den Rest 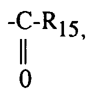 einen Monohydroxyalkylrest oder einen Polyhydroxyalkylrest bedeutet, worin die Hydroxylgruppen gegebenenfalls in Form von Methoxy oder Acetoxy geschützt sind oder einen niedrigen epoxidierten Alkylrest oder den Rest 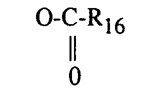 bedeutet, wobei
**R**_{**15**} den Rest OR₁₆ oder den Rest 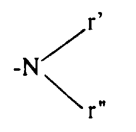 darstellt, wobei
**R**_{**16**} ein Wasserstoffatom, einen niedrigen Alkylrest, einen Arylrest oder einen Aralkylrest darstellt,
**R**_{**14**} eine Hydroxylgruppe für den Fall, daß m>2 ist, einen Monohydroxyalkylrest, den Rest 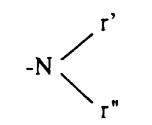 den Rest 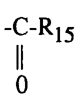 oder
einen mono- oder polyhydroxylierten Alkenylrest mit 2 bis 10 Kohlenstoffatomen bedeutet, oder dann, wenn R₂ und R₃ nicht zusammengenommen sind, m 0 sein kann und/oder R₁₄ ein Wasserstoffatom oder einen niedrigen Alkylrest darstellen kann,
**R**_{**6**} und **R**_{**7**} ein Wasserstoffatom, ein Halogenatom, einen niedrigen Alkylrest oder den Rest -OR₁₆ bedeuten,
**R**_{**5**} und **R**_{**6**} darüber hinaus einen Methylendioxyring bilden können, sofern sich R₆ in der Stellung 3 des Benzolkerns befindet, sowie
die Salze der Verbindungen gemäß der Formel (1), sofern R₁ eine Carbonsäurefunktion bedeutet, sowie die chiralen Analoga der Verbindungen gemäß der Formel (I) und die geometrischen Isomeren dieser Verbindungen, sofern R₂ und R₃ nicht zusammengenommen sind.

2. Verbindungen nach Anspruch 1, dadurch gekennzeichnet, daß sie in Form von Salzen eines Alkali- oder Erdalkalimetalls oder auch in Form von Zinksalzen oder in Form von Salzen eines organischen Amins vorliegen.

3. Verbindungen nach Anspruch 1, dadurch gekennzeichnet, daß sie in Form von Salzen einer Mineralsäure oder organischen Säure vorliegen, die aus der aus Salzsäure, Schwefelsäure, Essigsäure, Zitronensäure, Fumarsäure, Semibernsteinsäure, Maleinsäure und Mandelsäure bestehenden Gruppe ausgewählt sind.

4. Verbindungen nach Anspruch 1, dadurch gekennzeichnet, daß der niedrige Alkylrest 1 bis 6 Kohlenstoffatome aufweist und vorzugsweise aus der aus dem Methyl-, Ethyl-, Isopropyl-, Butyl- und tert-Butylradikal bestehenden Gruppe ausgewählt ist.

5. Verbindungen nach Anspruch 1. dadurch gekennzeichnet, daß der niedere Alkoxyrest aus der aus einem Methoxy-, Ethoxy-, Isopropoxy- und Butoxyradikal bestehenden Gruppe ausgewählt ist.

6. Verbindungen nach Anspruch 1, dadurch gekennzeichnet, daß der cycloaliphatische Rest aus der 1-Methylcyclohexylgruppe und der 1-Adamantylgruppe ausgewählt ist.

7. Verbindungen nach Anspruch 1, dadurch gekennzeichnet, daß der Monohydroxyalkylrest die Hydroxymethylgruppe, 2-Hydroxyethylgruppe, 2-Hydroxypropylgruppe, 3-Hydroxypropylgruppe, 4-Hydroxybutylgruppe, 5-Hydroxypentyl- oder 6-Hydroxyhexylgruppe darstellt.

8. Verbindungen nach Anspruch 1, dadurch gekennzeichnet, daß der Polyhydroxyalkylrest 3 bis 6 Kohlenstoffatome und 2 bis 5 Hydroxylgruppen trägt sowie aus der aus dem 2,3-Dihydroxypropylradikal, 2,3,4-Trihydroxybutylradikal, 2,3,4,5-Tetrahydroxypentylradikal und dem Pentaerythritradikal bestehenden Gruppe ausgewählt ist.

9. Verbindungen nach Anspruch 1, dadurch gekennzeichnet, daß der Arylrest eine ggf. durch mindestens ein Halogenatom, eine Hydroxylguppe oder eine Nitrofunktion substituierte Phenylgruppe darstellt.

10. Verbindungen nach Anspruch 1, dadurch gekennzeichnet, daß der Aralkylrest die Benzylgruppe oder die ggf. durch mindestens ein Halogenatom, eine Hydroxylgruppe oder eine Nitrofunktion substituierte Phenethylgruppe darstellt.

11. Verbindungen nach Anspruch 1, dadurch gekennzeichnet, daß der Heterocyclus aus der aus einem Piperidinradikal, Morpholinradikal, Pyrrolidinradikal oder Piperazinradikal bestehenden Gruppe ausgewählt ist, wobei ggf. in der Stellung 4 eine Substitution durch einen C₁-C₆-Alkylrest oder einen Mono- oder Polyhydroxyalkylrest vorgenommen ist.

12. Verbindungen nach einem der vorstehenden Ansprüche. dadurch gekennzeichnet, daß sie aus der aus den folgenden Stoffen bestehenden Gruppe ausgewählt sind:
6-[3-(1-Adamantyl)-4-(3-aminopropyloxy)-phenyl]-2-naphthoesäure•Hydrochlorid,
Methyl-6-[3-(1-adamantyl)-4-(2,3-dihydroxypropyloxy)-phenyl]-2-naphthoat,
6-[3-(1-Adamantyl)-4-(2,3-dihydroxypropyloxy)-phenyl]-2-naphthoesäure,
Benzyl-6-[3-(1-adamantyl)-4-methoxycarbonylmethylphenyl]-2-naphthoat,
6-[3-(1-Adamantyl)-4-methoxycarbonylmethyloxyphenyl]-2-naphthoesäure,
6-[3-(1-Adamantyl)-4-carboxymethyloxyphenyl]-2-naphthoesäure,
Methyl-6-[3-(1-adamantyl)-4-(3-hydroxypropyloxy)-phenyl]-2-naphthoat,
6-[3-(1-Adamantyl)-4-(3-hydroxypropyloxy)-phenyl]-2-naphthoesäure,
Methyl-6-[3-(1-adamantyl)-4-benzyloxycarbonylphenyl]-2-naphthoat,
Methyl-6-[3-(1-adamantyl)-4-carboxyphenyl]-2-naphthoat,
Methyl-6-[3-(1-adamantyl)-4-hydroxymethylphenyl]-2-naphthoat,
6-[3-(1-Adamantyl)-4-hydroxymethylphenyl]-2-naphthoesäure,
6-[3-(1-Adamantyl)-4-acetoxymethylphenyl]-2-naphthoesäure,
Methyl-6-[-3-(1-adamantyl)-4-methoxycarbonylphenyl]-2-naphthoat,
6-[3-(1-Adamantyl)-4-methoxycarbonylphenyl]-2-naphthoesäure,
6-[-3-(1-Adamantyl)-4-carboxyphenyl]-2-naphthoesäure,
6-[-3-(1-Adamantyl)-4-carboxamidophenylj-2-naphthoesäure,
Benzyl-6-[3-(1-adamantyl)-4-methoxycarbonylethenylphenyl]-2-naphthoat
6-[3-(1-Adamantyl)-4-(methoxycarbonylethyl)-phenyl]-2-naphthoesäure,
Benzyl-6-[3-(1-adamantyl)-4-(2,3-epoxypropyl)-phenyl]-2-naphthoat,
6-[3-(1-Adamantyl)-4-(hydroxypropyl)-phenyl]-naphthoesäure,
6-[3-(1-Adamantyl)-4-(3-methoxy-2-hydroxypropyl)-phenyl]-2-naphthoesäure,
4-{(E)-2-[3-(1-Adamantyl)-4-methoxyphenyl]-propenyl}-benzoesäure,
Benzyl-4-{(E)-2-[3-(1-adamantyl)-4-methoxyphenyl]-propenyl}-benzoat,
4-{(E)-[3-(1-Adamantyl)-4-hydroxyphenyl]-1-propenyl}-benzolsäure,
6-[3-(1-Adamantyl)-4-methoxycarbonylphenyl]-2-naphtylcarboxamid,
Ethyl-4-{(E)-2-[3-(1-methylcyclohexyl)-4-hydroxyphenyl]-propenyl}-benzoat,
Ethyl-4-{(E)-2-[3-(1-methylcyclohexyl)-4-methoxyphenyl]-propenyl}-benzoat,
4-{(E)-2-[3-(1-Methylcyclohexyl)4-methoxyphenyl]-propenyl)}-benzoesäure,
Ethyl-4{(Z)-2-[3-(1-methylcyclohexyl)-4-hydroxyphenyl]-propenyl}-benzoat,
Ethyl-4-{(Z)-2-[3-(1-methylcyclohexyl)-4-methoxyphenyl]-propenyl}-benzoat,
4-{(Z)-2-[3-(-Methylcyclohexyl)-4-methoxyphenyl]-propenyl}-benzoesäure,
Ethyl-4-[(Z)-2-(3-tert-butyl-4-methoxyphenyl)-propenyl]-benzoat,
4-[(Z)-2-(3-tert-Butyl-4-methoxyphenyl)-propenyl]-benzoesäure,
4-[(E)-2-(3-tert-Butyl-4-hydroxyphenyl)-propenyl]-benzoesäure,
4-{(E)-2-[3-(1-Methylcyclohexyl)-4-hydroxyphenyl]-ethenyl-benzoesäure,
Ethyl-4-{(E)-2-[3-(1-methylcyclohexyl)-4-(6-tert-butoxycarbonylpentyloxy)-phenyl]ethenyl}-benzoat,
Ethyl-4-{(E)-2-[3-(1-methylcyclohexyl)-4-(6-carboxypentyloxy)-phenyl]-ethenyl}-benzoat,
4-{(E)-2-[3-(1-Adamantyl)-4-hydroxyphenyl]-ethenyl}-benzoesäure,
Benzyl-6-[3-(1-adamantyl)-4-(1,2-dihydroxyethyl)-phenyl]-2-naphthoat,
6-[3-(1-Adamantyl)-4-(1,2-dihydroxyethyl)-phenyl]-2-naphthoesäure,
Benzyl-6-[3-(1-adamantyl)-4-(3-hydroxypropyl)-phenyl]-2-naphthoat,
6-[3-(1-Adamantyl)-4-(3-hydroxypropyl)-phenyl]-2-naphthoesäure,
Benzyl-6-[3-(1-adamantyl)-4-(3-acetoxypropyl)-phenyl]-2-naphthoat,
6-[3-(1-Adamantyl)-4-(3-acetoxypropyl)-phenyl]-2-naphthoesäure,
Benzyl-6-[3-(1-adamantyl)-4-(2,3-dihydroxypropyl)-phenyl]-2-naphthoat,
6-[3-(1-Adamantyl)-4-(2,3-dihydroxypropyl)-phenyl]-2-naphthoesäure,
N-Methoxycarbonylmethyl-4-(6-benzyloxycarbonylnaphthyl)-2-(1-adamantyl)-phenylcarboxamid,
N-Methoxycarbonylmethyl-4-(6-carboxynaphthyl)-2-(1-adamantyl)-phenylcarboxamid,
6-[3-1-Adamantyl)-4-(N,N-dimethylcarbamoyl)-phenyl]-2-naphthoesäure,
Benzyl-6-{3-(1-adamantyl)-4-[2-(R),3-dihydroxypropyloxy]-phenyl}-2-naphthoat,
6-{3-(1-Adamantyl)-4-[2(R),3-dihydroxypropyloxy]-phenyl}-2-naphthoesäure,
6-[3-(1-Adamantyl)-4,5-methylendioxyphenyl]-2-naphthoesäure,
N-ethyl-6-[3-(1-adamantyl)-4-methoxycarbonylphenyl]-2-naphthylcarboxamid,
N,N-morpholyl-6-[3-(1-adamantyl)-4-methoxycarbonylphenyl]-2-naphthylcarboxamid,
4-[(E)-2-(3-tert-Butyl-4-methoxyphenyl)-propenyl]-phenylcarbinol, sowie
4-[(E)-2-(3-tert-Butyl-4-methoxyphenyl)-propenyl]-benzylacetat.

13. Arzneimittel, dadurch gekennzeichnet, daß es in einem geeigneten Träger zur enteralen, parenteralen, topischen oder ophthalmologischen Verabreichung mindestens eine Verbindung gemäß der Formel (I) nach einem der Ansprüche 1 bis 12 enthält.

14. Arzneimittel nach Anspruch 13, dadurch gekennzeichnet, daß es 0,001 bis etwa 5 Gew.-% einer Verbindung gemäß der Formel (I) enthält.

15. Verwendung einer Verbindung nach einem der Ansprüche 1 bis 12 zur Herstellung eines Arzneimittels, das zur Behandlung von dermatologischen, rheumatischen, respiratorischen wie auch ophthalmologischen Affektionen bestimmt ist.

16. Kosmetische Zubereitung für die Körper- und Haarpflege, dadurch gekennzeichnet, daß sie in einem geeigneten kosmetischen Träger mindestens eine Verbindung gemäß der Formel (I) nach einem der Ansprüche 1 bis 12 enthält.

17. Kosmetische Zubereitung nach Anspruch 16, dadurch gekennzeichnet, daß sie die Verbindung gemäß der Formel (I) in einer Konzentration im Bereich zwischen 0,001 und 3 % enthält.

## Patentansprüche (Patentansprüche für folgende(n) Vertragsstaat(en): ES)

1. Kosmetische Zubereitung für die Körper- und Haarpflege, dadurch gekennzeichnet, daß sie in einem geeigneten kosmetischen Träger mindestens eine Verbindung gemäß der folgenden Formel (I) enthält: 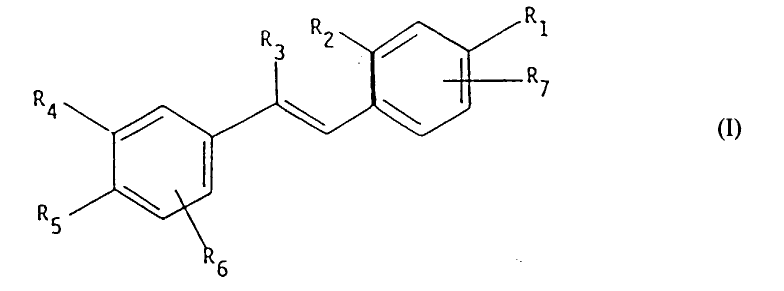 worin,
**R**_{**1**}
(i) ein Wasserstoffatom,
(ii) die Gruppe CH₃,
(iii) den Rest CH₂-O-R₈ bedeutet, wobei
**R**_{**8**} ein Wasserstoffatom oder einen niedrigen Alkylrest darstellt,
(iv) den Rest -OR₈ und
(v) den Rest 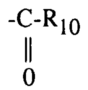 darstellt, wobei
**R**_{**10**}
(a) ein Wasserstoffatom,
(b) den Rest 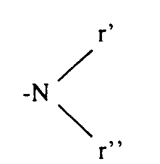 darstellt, wobei r' und r" ein Wasserstoffatom, einen niedrigen Alkylrest, einen Mono- oder Polyhydroxyalkylrest, einen ggf. substituierten Arylrest oder einen Aminosäure- oder Zuckerrest oder zusammengenommen einen Heterocyclus bedeuten,
(c) einen Rest -OR₁₁ bedeutet, wobei
**R**_{**11**} ein Wasserstoffatom, einen gerad- oder verzweigtkettigen Alkylrest mit 1-20 Kohlenstoffatomen, einen Mono- oder Polyhydroxyalkylrest, einen oder mehrere ggf. substituierte Aryl- oder Aralkylreste oder einen Zucker- oder Aminosäurerest darstellt und
(vi) einen Rest -S(O)ₜR₈ bedeutet, wobei
t die Zahl 0, 1 oder 2 bedeutet, und R₈ die obenstehende Bedeutung aufweist,
**R**_{**2**} ein Wasserstoffatom und
**R**_{**3**} ein Wasserstoffatom, einen Alkylrest, einen ggf. durch eine Hydroxylgruppe, ein niedriges Alkoxy oder durch einen Rest 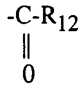 ggf. substituierten Aralkylrest- oder einen niedrigen Alkylrest bedeuten, wobei
**R**_{**12**} ein Wasserstoffatom, einen niedrigen Alkylrest, eine Hydroxylgruppe, einen niedrigen Alkoxyrest oder einen Rest 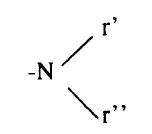 bedeutet oder R₂ und R₃ zusammengenommen mit dem Benzolring einen Naphthalinring bilden,
**R**_{**4**} einen gerad- oder ggf. verzweigtkettigen Alkylrest mit 1-5 Kohlenstoffatomen oder einen cycloaliphatischen Rest bedeutet,
**R**_{**5**} den Rest (CH₂)ₙ-R₁₃, den Rest CH=CH-(CH₂)ₙ-R₁₃ oder
den Rest -O(CH₂)ₘR₁₄ darstellt, wobei
n die Zahl 0 oder 1 bis 6 und
m die Zahl 1 bis 6 bedeuten und
**R**_{**13**} den Rest 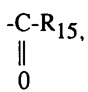 einen Monohydroxyalkylrest oder einen Polyhydroxyalkylrest bedeutet, worin die Hydroxylgruppen ggf. in Form von Methoxy oder Acetoxy geschützt sind oder einen niedrigen epoxidierten Alkylrest oder den Rest 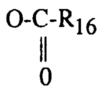 bedeutet, wobei
**R**_{**15**} den Rest OR₁₆ oder den Rest 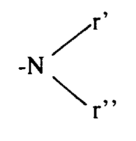 darstellt, wobei
**R**_{**16**} ein Wasserstoffatom, einen niedrigen Alkylrest, einen Arylrest oder einen Aralkylrest darstellt,
**R**_{**14**} eine Hydroxylgruppe für den Fall, daß m>2 ist, einen Monohydroxyalkylrest, den Rest 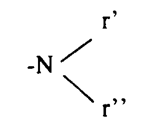 den Rest 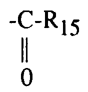 oder
einen mono- oder polyhydroxylierten Alkenylrest mit 2 bis 10 Kohlenstoffatomen bedeutet, oder dann, wenn R₂ und R₃ nicht zusammengenommen sind, m 0 sein kann und/oder R₁₄ ein Wasserstoffatom oder einen niedrigen Alkylrest darstellen kann,
**R**_{**6**} und **R**_{**7**} ein Wasserstoffatom, ein Halogenatom, einen niedrigen Alkylrest oder den Rest -OR₁₆ bedeuten,
**R**_{**5**} und **R**_{**6**} darüber hinaus einen Methylendioxyring bilden können, sofern sich R₆ in der Stellung 3 des Benzolkerns befindet, sowie
die Salze der Verbindungen gemäß der Formel (I), sofern R₁ eine Carbonsäurefunktion bedeutet, sowie die chiralen Analoga der Verbindungen gemäß der Formel (I) und die geometrischen Isomeren dieser Verbindungen, sofern R₂ und R₃ nicht zusammengenommen sind.

2. Zubereitung nach Anspruch 1, dadurch gekennzeichnet, daß die Verbindung gemäß Formel (I) in Form eines Salzes eines Alkali- oder Erdalkalimetalls oder auch in Form eines Zinksalzes oder in Form eines Salzes eines organischen Amins vorliegt.

3. Zubereitung nach Anspruch 1, dadurch gekennzeichnet, daß die Verbindung gemäß Formel (I) in Form eines Salzes einer Mineralsäure oder organischen Säure vorliegt, die aus der aus Salzsäure, Schwefelsäure, Essigsäure, Zitronensäure, Fumarsäure, Semibernsteinsäure, Maleinsäure und Mandelsäure bestehenden Gruppe ausgewählt ist.

4. Zubereitung nach einem der vorstehenden Ansprüche, dadurch gekennzeichnet, daß die Verbindung gemäß der Formel (I) aus der aus den folgenden Stoffen bestehenden Gruppe ausgewählt ist:
6-[3-(1-Adamantyl)-4-(3-aminopropyloxy)-phenyl]-2-naphthoesäure•Hydrochlorid,
Methyl-6-[3-(1-adamantyl)-4-(2,3-dihydroxypropyloxy)-phenyl]-2-naphthoat,
6-[3-(1-Adamantyl)-4-(2,3-dihydroxypropyloxy)-phenyl]-2-naphthoesäure,
Benzyl-6-[3-(1-adamantyl)-4-methoxycarbonylmethylphenyl]-2-naphthoat,
6-[3-(1-Adamantyl)-4-methoxycarbonylmethyloxyphenyl]-2-naphthoesäure,
6-[3-(1-Adamantyl)-4-carboxymethyloxyphenyl]-2-naphthoesäure,
Methyl-6-[3-(1-adamantyl)-4-(3-hydroxypropyloxy)-phenyl]-2-naphthoat,
6-[3-(1-Adamantyl)-4-(3-hydroxypropyloxy)-phenyl]-2-naphthoesäure,
Methyl-6-[3-(1-adamantyl)-4-benzyloxycarbonylphenyl]-2-naphthoat
Methyl-6-[3-(1-adamantyl)-4-carboxyphenyl]-2-naphthoat,
Methyl-6-[3-(1-adamantyl)-4-hydroxymethylphenyl]-2-naphthoat,
6-[3-(1-Adamantyl)-4-hydroxymethylphenyl]-2-naphthoesäure,
6-[3-(1-Adamantyl)-4-acetoxymethylphenyl]-2-naphthoesäure,
Methyl-6-[-3-(1-adamantyl)-4-methoxycarbonylphenyl]-2-naphthoat,
6-[3-(1-Adamantyl)-4-methoxycarbonylphenyl]-2-naphthoesäure,
6-[-3-(1-Adamantyl)-4-carboxyphenyl]-2-naphthoesäure,
6-[-3-(1-Adamantyl)-4-carboxamidophenyl]-2-naphthoesäure,
Benzyl-6-[3-(1-adamantyl)-4-methoxycarbonylethenylphenyl]-2-naphthoat,
6-[3-(1-Adamantyl)-4-(methoxycarbonylethyl)-phenyl]-2-naphthoesäure,
Benzyl-6-[3-(1-adamantyl)-4-(2,3-epoxypropyl)-phenyl]-2-naphthoat,
6-[3-(1-Adamantyl)-4-(hydroxypropyl)-phenyl]-naphthoesäure,
6-[3-(1-Adamantyl)-4-(3-methoxy-2-hydroxypropyl)-phenyl]-2-naphthoesäure,
4-{(E)-2-[3-(1-Adamantyl)-4-methoxyphenyl]-propenyl)-benzoesäure,
Benzyl-4-{(E)-2-[3-(1-adamantyl)-4-methoxyphenyl]-propenyl}-benzoat,
4-{(E)-[3-(1-Adamantyl)-4-hydroxyphenyl]-1-propenyl}-benzoesäure,
6-[3-(1-Adamantyl)-4-methoxycarbonylphenyl]-2-naphtylcarboxamid,
Ethyl-4-{(E)-2-[3-(1-methylcyclohexyl)-4-hydroxyphenyl]-propenyl}-benzoat,
Ethyl-4-{(E)-2-[3-(1-methylcyclohexyl)-4-methoxyphenyl]-propenyl}-benzoat,
4-{(E)-2-[3-(1-Methylcyclohexyl)-4-methoxyphenyl]-propenyl}-benzoesäure,
Ethyl-4{(Z)-2-[3-(1-methylcyclohexyl)-4-hydroxyphenyl]-propenyl}-benzoat,
Ethyl-4-{(Z)-2-[3-(1-methylcyclohexyl)-4-methoxyphenyl]-propenyl}-benzoat,
4-{(Z)-2-[3-(1-Methylcyclohexyl)-4-methoxyphenyl]-propenyl}-benzoesäure,
Ethyl-4-[(Z)-2-(3-tert-butyl-4-methoxyphenyl)-propenyl]-benzoat,
4-[(Z)-2-(3-tert-Butyl-4-methoxyphenyl)-propenyl]-benzoesäure,
4-[(E)-2-(3-tert-Butyl-4-hydroxyphenyl)-propenyl]-benzoesäure,
4-{(E)-2-[3-(1-Methylcyclohexyl)-4-hydroxyphenyl]-ethenyl}-benzoesäure,
Ethyl-4-{(E)-2-[3-(1-methylcyclohexyl)-4-(6-tert-butoxycarbonylpentyloxy)-phenyl]ethenyl}-benzoat,
Ethyl-4-{(E)-2-[3-(1-methylcyclohexyl)-4-(6-carboxypentyloxy)-phenyl]-ethenyl}-benzoat,
4-{(E)-2-[3-(1-Adamantyl)-4-hydroxyphenyl]-ethenyl}-benzoesäure,
Benzyl-6-[3-(1-adamantyl)-4-(1,2-dihydroxyethyl)-phenyl]-2-naphthoat,
6-[3-(1-Adamantyl)-4-(1,2-dihydroxyethyl)-phenyl]-2-naphthoesäure,
Benzyl-6-[3-(1-adamantyl)-4-(3-hydroxypropyl)-phenyl]
6-[3-(1-Adamantyl)-4-(3-hydroxypropyl)-phenyl]-2-naphthoesäure,
Benzyl-6-[3-(1-adamantyl)-4-(3-acetoxypropyl)-phenyl]-2-naphthoat,
6-[3-(1-Adamantyl)-4-(3-acetoxypropyl)-phenyl]-2-naphthoesäure,
Benzyl-6-[3-(1-adamantyl)-4-(2,3-dihydroxypropyl)-phenyl]-2-naphthoat,
6-[3-(1-Adamantyl)-4-(2,3-dihydroxypropyl)-phenyl]-2-naphthoesäure,
N-Methoxycarbonylmethyl-4-(6-benzyloxycarbonylnaphthyl)-2-(1-adamantyl)-phenylcarboxamid,
N-Methoxycarbonylmethyl-4-(6-carboxynaphthyl)-2-(1-adamantyl)-phenylcarboxamid,
6-[3-1-Adamantyl)-4-(N,N-dimethylcarbamoyl)-phenyl]-2-naphthoesäure,
Benzyl-6-{3-(1-adamantyl)-4-[2-(R),3-dihydroxypropyloxy]-phenyl}-2-naphthoat,
6-{3-(1-Adamantyl)-4-[2(R),3-dihydroxypropyloxy]-phenyl}-2-naphthoesäure,
6-[3-(1-Adamantyl)-4,5-methylendioxyphenyl]-2-naphthoesäure,
N-ethyl-6-[3-(1-adamantyl)-4-methoxycarbonylphenyl]-2-naphthylcarboxamid,
N, N-morpholyl-6-[3-(1-adamantyl)-4-methoxycarbonylphenyl]-2-naphthylcarboxamid,
4-[(E)-2-(3-tert-Butyl-4-methoxyphenyl)-propenyl]-phenylcarbinol, sowie
4-[(E)-2-(3-tert-Butyl-4-methoxyphenyl)-propenyl]-benzylacetat.

5. Kosmetische Zubereitung nach einem der vorstehenden Ansprüche, dadurch gekennzeichnet, daß sie die Verbindung gemäß der Formel (I) in einer Konzentration im Bereich zwischen 0,0001 (0,001) und 3% enthält.

## Patentansprüche (Patentansprüche für folgende(n) Vertragsstaat(en): GR)

1. Verbindungen mit zwei aromatischen Ringen, dadurch gekennzeichnet, daß sie der folgenden allgemeinen Formel (I) entsprechen: 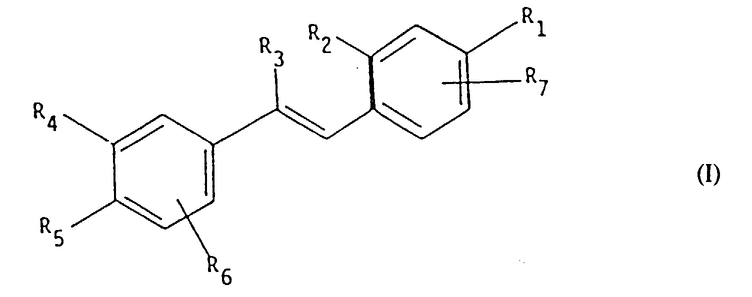 worin,
**R**_{**1**}
(i) ein Wasserstoffatom,
(ii) die Gruppe CH₃,
(iii) den Rest CH₂-O-R₈ bedeutet, wobei
**R**_{**8**} ein Wasserstoffatom oder einen niedrigen Alkylrest darstellt,
(iv) den Rest -OR₈ und
(v) den Rest 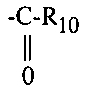 darstellt, wobei
**R**_{**10**}
(a) ein Wasserstoffatom,
(b) den Rest 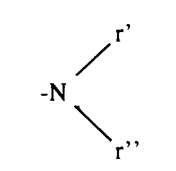 darstellt, wobei r' und r" ein Wasserstoffatom, einen niedrigen Alkylrest, einen Mono- oder Polyhydroxyalkylrest, einen ggf. substituierten Arylrest oder einen Aminosäure- oder Zuckerrest oder zusammengenommen einen Heterocyclus bedeuten,
(c) einen Rest -OR₁₁ bedeutet, wobei
**R**_{**11**} ein Wasserstoffatom, einen gerad- oder verzweigtkettigen Alkylrest mit 1-20 Kohlenstoffatomen, einen Mono- oder Polyhydroxyalkylrest, einen oder mehrere ggf. substituierte Aryl- oder Aralkylreste oder einen Zucker- oder Aminosäurerest darstellt und
(vi) einen Rest -S(O)ₜR₈ bedeutet, wobei
t die Zahl 0, 1 oder 2 bedeutet, und R₈ die obenstehende Bedeutung aufweist,
**R**_{**2**} ein Wasserstoffatom und
**R**_{**3**} ein Wasserstoffatom, einen Alkylrest, einen ggf. durch eine Hydroxylgruppe, ein niedriges Alkoxy oder durch einen Rest 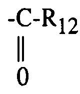 ggf. substituierten Aralkylrest- oder einen niedrigen Alkylrest bedeuten, wobei
**R**_{**12**} ein Wasserstoffatom, einen niedrigen Alkylrest, eine Hydroxylgruppe, einen niedrigen Alkoxyrest oder einen Rest 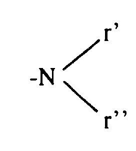 bedeutet oder R₂ und R₃ zusammengenommen mit dem Benzolring einen Naphthalinring bilden,
**R**_{**4**} einen gerad- oder ggf. verzweigtkettigen Alkylrest mit 1-5 Kohlenstoffatomen oder einen cycloaliphatischen Rest bedeutet,
**R**_{**5**} den Rest (CH₂)ₙ-R₁₃, den Rest CH=CH-(CH₂)ₙ-R₁₃ oder
den Rest -O(CH₂)ₘR₁₄ darstellt, wobei
n die Zahl 0 oder 1 bis 6 und
m die Zahl 1 bis 6 bedeuten und
**R**_{**13**} den Rest 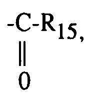 einen Monohydroxyalkylrest oder einen Polyhydroxyalkylrest bedeutet, worin die Hydroxylgruppen ggf. in Form von Methoxy oder Acetoxy geschützt sind oder einen niedrigen epoxidierten Alkylrest oder den Rest 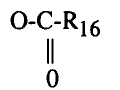 bedeutet, wobei
**R**_{**15**} den Rest OR₁₆ oder den Rest 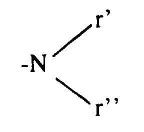 darstellt, wobei
**R**_{**16**} ein Wasserstoffatom, einen niedrigen Alkylrest, einen Arylrest oder einen Aralkylrest darstellt,
**R**_{**14**} eine Hydroxylgruppe für den Fall, daß m>2 ist, einen Monohydroxyalkylrest, den Rest 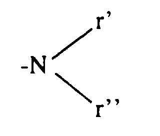 den Rest 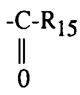 oder
einen mono- oder polyhydroxylierten Alkenylrest mit 2 bis 10 Kohlenstoffatomen bedeutet, oder dann, wenn R₂ und R₃ nicht zusammengenommen sind, m 0 sein kann und/oder R₁₄ ein Wasserstoffatom oder einen niedrigen Alkylrest darstellen kann,
**R**_{**6**} und **R**_{**7**} ein Wasserstoffatom, ein Halogenatom, einen niedrigen Alkylrest oder den Rest -OR₁₆ bedeuten,
**R**_{**5**} und **R**_{**6**} darüber hinaus einen Methylendioxyring bilden können, sofern sich R₆ in der Stellung 3 des Benzolkerns befindet, sowie
die Salze der Verbindungen gemäß der Formel (I), sofern R₁ eine Carbonsäurefunktion bedeutet, sowie die chiralen Analoga der Verbindungen gemäß der Formel (I) und die geometrischen Isomeren dieser Verbindungen, sofern R₂ und R₃ nicht zusammengenommen sind.

2. Verbindungen nach Anspruch 1, dadurch gekennzeichnet, daß sie in Form von Salzen eines Alkali- oder Erdalkalimetalls oder auch in Form von Zinksalzen oder in Form von Salzen eines organischen Amins vorliegen.

3. Verbindungen nach Anspruch 1, dadurch gekennzeichnet, daß sie in Form von Salzen einer Mineralsäure oder organischen Säure vorliegen, die aus der aus Salzsäure, Schwefelsäure, Essigsäure, Zitronensäure, Fumarsäure, Semibernsteinsäure, Maleinsäure und Mandelsäure bestehenden Gruppe ausgewählt sind.

4. Verbindungen nach Anspruch 1, dadurch gekennzeichnet, daß der Niederalkylrest 1-6 Kohlenstoffatome aufweist und aus der aus dem Methyl-, Ethyl-, Isopropyl-, Butyl- und tert-Butylradikal bestehenden Gruppe ausgewählt ist.

5. Verbindungen nach Anspruch 1, dadurch gekennzeichnet, daß der Niederalkoxyrest aus der aus dem Methoxy-, Ethoxy-, Isopropoxy- und Butoxyradikal bestehenden Gruppe ausgewählt ist.

6. Verbindungen nach Anspruch 1, dadurch gekennzeichnet, daß der cycloaliphatische Rest aus der 1-Methylcyclohexylgruppe und der 1-Adamantylgruppe ausgewählt ist.

7. Verbindungen nach Anspruch 1, dadurch gekennzeichnet, daß der Monohydroxyalkylrest die Hydroxymethylgruppe, 2-Hydroxyethylgruppe, 2-Hydroxypropylgruppe, 3-Hydroxypropylgruppe, 4-Hydroxybutylgruppe, 5-Hydroxypentyl- oder 6-Hydroxyhexylgruppe darstellt.

8. Verbindungen nach Anspruch 1, dadurch gekennzeichnet, daß der Polyhydroxyalkylrest 3 bis 6 Kohlenstoffe und 2 bis 5 Hydroxylgruppen trägt sowie aus der aus dem-2,3-Dihydroxypropylradikal, 2,3,4-Trihydroxybutylradikal, 2,3,4,5-Tetrahydroxypentylradikal und dem Pentaerythritradikal bestehenden Gruppe ausgewählt ist.

9. Verbindungen nach Anspruch 1, dadurch gekennzeichnet, daß der Arylrest eine ggf. durch mindestens ein Halogenatom, eine Hydroxylgruppe oder eine Nitrofunktion substituierte Phenylgruppe darstellt.

10. Verbindungen nach Anspruch 1, dadurch gekennzeichnet, daß der Aralkylrest die Benzylgruppe oder die ggf. durch mindestens ein Halogenatom, eine Hydroxylgruppe oder eine Nitrofunktion substituierte Phenethylgruppe darstellt.

11. Verbindungen nach Anspruch 1, dadurch gekennzeichnet, daß der Heterocyclus aus der aus einem Piperidinradikal, Morpholinradikal, Pyrrolidinradikal oder Piperazinradikal bestehenden Gruppe ausgewählt ist, wobei ggf. in der Stellung 4 eine Substitution durch einen C₁-C₆-Alkylrest oder einen Mono- oder Polyhydroxyalkylrest vorgenommen ist.

12. Verbindungen nach einem der vorstehenden Ansprüche, dadurch gekennzeichnet, daß sie aus der aus den folgenden Stoffen bestehenden Gruppe ausgewählt sind:
6-[3-(1-Adamantyl)-4-(3-aminopropyloxy)-phenyl]-2-naphthoesäure•Hydrochlorid
Methyl-6-[3-(1-adamantyl)-4-(2,3-dihydroxypropyloxy)-phenyl]-2-naphthoat,
6-[3-(1-Adamantyl)-4-(2,3-dihydroxypropyloxy)-phenyl]-2-naphthoesäure,
Benzyl-6-[3-(1-adamantyl)-4-methoxycarbonylmethylphenyl]-2-naphthoat,
6-[3-(1-Adamantyl)-4-methoxycarbonylmethyloxyphenyl]-2-naphthoesäure,
6-[3-(1-Adamantyl)-4-carboxymethyloxyphenyl]-2-naphthoesäure,
Methyl-6-[3-(1-adamantyl)-4-(3-hydroxypropyloxy)-phenyl]-2-naphthoat,
6-[3-(1-Adamantyl)-4-(3-hydroxypropyloxy)-phenyl]-2-naphthoesäure,
Methyl-6-[3-(1-adamantyl)-4-benzyloxycarbonylphenyl]-2-naphthoat,
Methyl-6-[3-(1-adamantyl)-4-carboxyphenyl]-2-naphthoat,
Methyl-6-[3-(1-adamantyl)-4-hydroxymethylphenyl]-2-naphthoat,
6-[3-(1-Adamantyl)-4-hydroxymethylphenyl]-2-naphthoesäure,
6-[3-(1-Adamantyl)-4-acetoxymethylphenyl]-2-naphthoesäure,
Methyl-6-[-3-(1-adamantyl)-4-methoxycarbonylphenyl]-2-naphthoat,
6-[3-(1-Adamantyl)-4-methoxycarbonylphenyl]-2-naphthoesäure,
6-[-3-(1-Adamantyl)-4-carboxyphenyl]-2-naphthoesäure,
6-[-3-(1-Adamantyl)-4-carboxamidophenyl]-2-naphthoesäure,
Benzyl-6-[3-(1-adamantyl)-4-methoxycarbonylethenylphenyl]-2-naphthoat,
6-[3-(1-Adamantyl)-4-(methoxycarbonylethyl)-phenyl]-2-naphthoesäure,
Benzyl-6-[3-(1-adamantyl)-4-(2,3-epoxypropyl)-phenyl]-2-naphthoat,
6-[3-(1-Adamantyl)-4-(hydroxypropyl)-phenyl]-naphthoesäure,
6-[3-(1-Adamantyl)-4-(3-methoxy-2-hydroxypropyl)-phenyl]-2-naphthoesäure,
4-{(E)-2-[3-(1-Adamantyl)-4-methoxyphenyl]-propenyl}-benzoesäure,
Benzyl-4-{(E)-2-[3-(1-adamantyl)-4-methoxyphenyl]-propenyl}-benzoat,
4-{(E)-[3-(1-Adamantyl)-4-hydroxyphenyl]-1-propenyl}-benzoesäure,
6-[3-(1-Adamantyl)-4-methoxycarbonylphenyl]-2-naphtylcarboxamid,
Ethyl-4-{(E)-2-[3-(1-methylcyclohexyl)-4-hydroxyphenyl]-propenyl}-benzoat,
Ethyl-4-{(E)-2-[3-(1-methylcyclohexyl)-4-methoxyphenyl]-propenyl}-benzoat,
4-{(E)-2-[3-(1-Methylcyclohexyl)-4-methoxyphenyl]-propenyl}-benzoesäure,
Ethyl-4{(Z)-2-[3-(1-methylcyclohexyl)-4-hydroxyphenyl]-propenyl}-benzoat,
Ethyl-4-{(Z)-2-[3-(1-methylcyclohexyl)-4-methoxyphenyl]-propenyl}-benzoat,
4-{(Z)-2-[3-(1-Methylcyclohexyl)-4-methoxyphenyl]-propenyl}-benzoesäure,
Ethyl-4-[(Z)-2-(3-tert-butyl-4-methoxyphenyl)-propenyl]-benzoat,
4-[(Z)-2-(3-tert-Butyl-4-methoxyphenyl)-propenyl]-benzoesäure,
4-[(E)-2-(3-tert-Butyl-4-hydroxyphenyl)-propenyl]-benzoesäure,
4-{(E)-2-[3-(1-Methylcyclohexyl)-4-hydroxyphenyl]-ethenyl}-benzoesäure,
Ethyl-4-{(E)-2-[3-(1-methylcyclohexyl)-4-(6-tert-butoxycarbonylpentyloxy)-phenyl]ethenyl}-benzoat,
Ethyl-4-{(E)-2-[3-(1-methylcyclohexyl)-4-(6-carboxypentyloxy)-phenyl]-ethenyl}-benzoat,
4-{(E)-2-[3-(1-Adamantyl)-4-hydroxyphenyl]-ethenyl}-benzoesäure,
Benzyl-6-[3-(1-adamantyl)-4-(1,2-dihydroxyethyl)-phenyl]-2-naphthoat,
6-[3-(1-Adamantyl)-4-(1,2-dihydroxyethyl)-phenyl]-2-naphthoesäure,
Benzyl-6-[3-(1-adamantyl)-4-(3-hydroxypropyl)-phenyl]-2-naphthoat,
6-[3-(1-Adamantyl)-4-(3-hydroxypropyl)-phenyl]
Benzyl-6-[3-(1-adamantyl)-4-(3-acetoxypropyl)-phenyl]-2-naphthoat,
6-[3-(1-Adamantyl)-4-(3-acetoxypropyl)-phenyl]-2-naphthoesäure,
Benzyl-6-[3-(1-adamantyl)-4-(2,3-dihydroxypropyl)-phenyl]-2-naphthoat,
6-[3-(1-Adamantyl)-4-(2,3-dihydroxypropyl)-phenyl]-2-naphthoesäure,
N-Methoxycarbonylmethyl-4-(6-benzyloxycarbonylnaphthyl)-2-(1-adamantyl)-phenylcarboxamid,
N-Methoxycarbonylmethyl-4-(6-carboxynaphthyl)-2-(1-adamantyl)-phenylcarboxamid,
6-[3-1-Adamantyl)-4-(N,N-dimethylcarbamoyl)-phenyl]-2-naphthoesäure,
Benzyl-6-{3-(1-adamantyl)-4-[2-(R),3-dihydroxypropyloxy]-phenyl}-2-naphthoat,
6-{3-(1-Adamantyl)-4-[2(R),3-dihydroxyprnpyloxy]-phenyl}-2-naphthoesäure,
6-[3-(1-Adamantyl)-4,5-methylendioxyphenyl]-2-naphthoesäure,
N-ethyl-6-[3-(1-adamantyl)-4-methoxycarbonylphenyl]-2-naphthylcarboxamid,
N, N-morpholyl-6-[3-(1-adamantyl)-4-methoxycarbonylphenyl]-2-naphthylcarboxamid,
4-[(E)-2-(3-tert-Butyl-4-methoxyphenyl)-propenyl]-phenylcarbinol, sowie
4-[(E)-2-(3-tert-Butyl-4-methoxyphenyl)-propenyl]-benzylacetat.

13. Kosmetische Zubereitung für die Körper- und Haarpflege, dadurch gekennzeichnet, daß sie in einem geeigneten kosmetischen Träger mindestens eine Verbindung gemäß der Formel (I) nach einem der Ansprüche 1 bis 12 enthält.

14. Kosmetische Zubereitung nach Anspruch 13, dadurch gekennzeichnet, daß sie die Verbindung gemäß der Formel (I) in einer Konzentration im Bereich zwischen 0,0001 (0,001) und 3% enthält.
